# EUROPEAN PATENT SPECIFICATION

(11) **EP 4 345 104 B1**
(45) Date of publication and mention of the grant of the patent: **28.08.2024**
(21) Application number: 22199013.8
(22) Date of filing: 30.09.2022
(51) Int. Cl.: C07K 14/605

(54) **METHOD FOR PREPARING LIRAGLUTIDE**
VERFAHREN ZUR HERSTELLUNG VON LIRAGLUTID
PROCÉDÉ DE PRÉPARATION DE LIRAGLUTIDE

(43) Date of publication of application: 03.04.2024
(73) Proprietor: Bachem Holding AG, 4416 Bubendorf (CH)
(72) Inventor: Eisenhuth, Ralf, 4416 Bubendorf (CH); Bury, Timm, 4416 Bubendorf (CH); Weiss, Juliane, 4416 Bubendorf (CH)
(74) Representative: Jacobi, Markus Alexander

(56) References cited:
- EP-A1- 3 517 543
- WO-A1-2017/162653
- CN-A- 105 111 303
- CN-A- 113 461 800
- CARBAJO DANIEL ET AL: "Optimized Stepwise Synthesis of the API Liraglutide Using BAL Resin and Pseudoprolines", ACS OMEGA, vol. 4, no. 5, 17 May 2019 (2019-05-17), US, pages 8674 - 8680, XP093026215, ISSN: 2470-1343, Retrieved from the Internet <URL:http://pubs.acs.org/doi/pdf/10.1021/acsomega.9b00974> DOI: 10.1021/acsomega.9b00974

## Description

The present invention relates to a method for manufacturing a precipitated liraglutide mixture by cleaving a solid phase conjugated protected liraglutide precursor with trifluoroacetic acid and precipitation with an antisolvent. It further relates to a precipitated mixture, which contains liraglutide or a salt thereof and N-terminally trifluoroacetylated liraglutide or a salt thereof. It also relates to a method for analyzing a precipitated liraglutide mixture, which includes a dissolution of a precipitated liraglutide mixture in a buffered solution for dissolving or diluting, a reverse phase high performance liquid chromatography and a determination of a content of N-terminally trifluoroacetylated liraglutide.

Liraglutide (CAS-No. 204656-20-2, (N-epsilon-(gamma-Glu(N-alpha-hexadecanoyl)))-Lys²⁶Arg³⁴-GLP-1(7-37), theoretical molecular mass of 3751.20 atomic mass units) is a drug substance that has been approved for the treatment of type 2 diabetes and for the treatment of obesity in adults with related comorbidity. It is a long acting derivative of an analogue of the naturally occurring human glucagon-like peptide-1 (GLP-1(7-37)) with 97% homology, i.e. liraglutide's amino acid sequence of its backbone is identical to the human GLP-1(7-37) except for a K34R mutation. Liraglutide is acylated with a hexadecanoyl-glutamyl side chain on lysine at position 26. This lipophilic substituent causes a prolongation of half-life in humans following intravenous administration of 8 hours or following subcutaneous administration of 13 hours versus 1 hour for native GLP-1.

Liraglutide can be produced by a process including recombinant DNA technology. A liraglutide backbone peptide is produced by recombinant DNA technology via yeast fermentation (Saccharomyces cerevisiae), followed by recovery and purification of the liraglutide backbone peptide. Acylation of the liraglutide backbone peptide with the fatty acid chain and further purification leads to the drug substance liraglutide. Liraglutide can also be produced by a process including solid phase peptide synthesis. In view of 31 amino acids in liraglutide's backbone and the gamma-Glu(N-alpha-hexadecanoyl)-substitution at its lysine of the backbone, there are several synthetic strategies for a solid phase synthesis. There is the synthetic approach that all amino acid derivatives of the peptide backbone are condensed step-by-step at the same resin, for example starting with the C-terminal Gly³⁷ covalently bonded to a resin via a linking group of the resin. There is the synthetic approach that smaller or larger peptide fragments are separately synthesized at different resins or in liquid and afterwards these smaller or larger peptide fragments are condensed to a peptide fragment already covalently bonded to a resin.

During solid phase peptide synthesis, side chains of amino acids are protected, if they carry a chemical functionality, which would otherwise detrimentally interfere with a peptide bond formation. These side chain protecting groups are typically acid labile in case of a protection of alpha-amino groups of the amino acids or peptides with base labile 9-fluorenylmethyloxy-carbonyl. For obtaining the desired unprotected liraglutide, the acid labile side chain protecting groups are finally removed by treatment with trifluoroacetic acid. If a liraglutide precursor is covalently bonded to a resin via an acid labile linking group, the cleavage of the liraglutide precursor from the resin occurs also at a treatment with trifluoroacetic acid. The acid sensitivity of the side chain protecting group varies with trityl groups being more acid sensitive protecting groups than tert-butyl groups. Often, the linking group of the resin is comparably sensitive to acid like a trityl group. In such a case, an exposure time to trifluoroacetic acid or a concentration of trifluoroacetic acid is chosen higher than what would be necessary for a cleavage from the linking group of the resin, but is chosen to assure a deprotection of the side chains from the tert-butyl groups. On the other side, an exposure of liraglutide for a long time to trifluoroacetic acid or to an acid with a higher acid strength will drive a decomposition of liraglutide. Beneath a reduction of yield, generated decomposition products add to an impurity load, which has to be removed in a purification.

A liraglutide material has to fulfill high requirements to qualify as an active pharmaceutical ingredient. It happens meanwhile that a regulatory authority asks for an identifying and reporting of a peptide-related impurity which has a content of 0.1 % or more in the liraglutide material. Furthermore, the liraglutide material is formulated to a liraglutide drug product. In the liraglutide drug product, a peptide-related impurity, which is present in a higher content than in the originally registered liraglutide drug product or is newly contained above 0.1% in comparison to the originally registered liraglutide drug product, can be considered as a concern for a risk of immunogenicity. The efforts to be invested in a purification from a certain crude liraglutide material into a liraglutide material suitable as an active pharmaceutical ingredient are considerable. For a liraglutide material, which is produced by a process including solid phase peptide synthesis, there are often at least two column chromatographic steps involved. For an occurring individual peptide-related impurity, it is of relevance to minimize its generation as such. For each individual peptide-related impurity, its low content prior to purification contributes to a generally beneficial reduction of the efforts for a chosen purification by lowering the overall impurity content or impurity load of a crude liraglutide material. An individual peptide-related impurity can be of a special relevance, if it is difficult to separate in a chosen purification procedure. For such a special individual peptide-related impurity, its low content provides a specially beneficial reduction of the efforts for a chosen purification by lowering the special impurity content or impurity load of a crude liraglutide material.

Liraglutide is reported to be prone to formation of different aggregates. Some aggregates are reported as strongly self-associated oligomers, for example heptamers, octamers or dodecamers. A pH-induced oligomer transformation between the octamer above pH 6.9 and the dodecamer below pH 6.9 is described in aqueous solutions. Some aggregates are reported to be made by covalent cross-binding between two or more liraglutide or partly degraded liraglutide or by incorporating tracing impurities into liraglutide. These aggregates are a highly diverse group of aggregate compounds and named sometimes high molecular weight proteins. It is reported that a continuing aggregation of liraglutide leads on a macroscopic level to fibril nuclei, fibril seeds and fibrils. The latter are observable with the naked eyes. One reported factor, which promotes aggregation, is a pH value below 8.2, for example 6.8 or 6.4. These pH values are still well above a reported iso-electric point of liraglutide of 4.0.

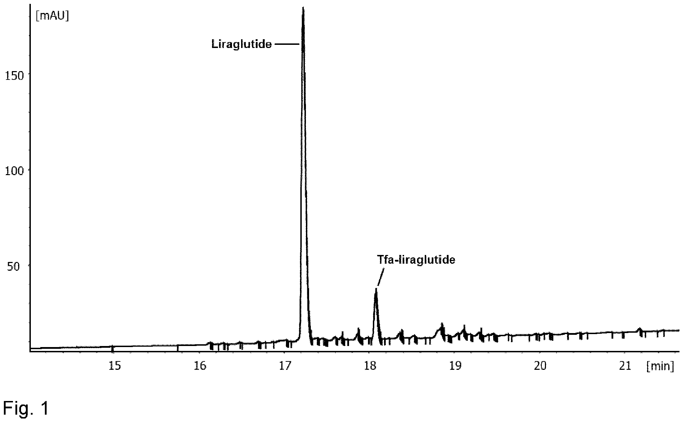

Peptide Research, Vol. 5, No. 5 (1992), p. 287-292 relates to N-alpha-trifluoroacetylation of N-terminal hydroxyamino acids as a new side reaction in peptide synthesis. In the synthesis of the double-chain bis-cystinyl fragment 225-232/225'-232' of the human IgG1 hinge region, the final acidolytic deprotection step, i.e. cleavage of the two O-tert-butyl-threonine ether functional groups, with 99% aqueous TFA is accompanied with formation of N-alpha-mono-trifluoroacetylated product. At Figure 1, the HPLC of the product distribution between the hinge peptide, its N-alpha-mono-trifluoroacetylated derivative and its N-alpha-bis-trifluoroacetylated derivative upon treatment of the hinge peptide with (A) 99% TFA for 17 h, (B) with 99% TFA and 2 equiv. trifluoroacetic anhydride for 2 h and (C) with 2 equiv. N-trifluoro-imidazole in CH₂Cl₂ and trimethylamine for 12 h and subsequent exposure to 90% TFA for 1.5 h is shown. Hydrolysis with 1 M NaOH of the product mixture led to the fully deprotected hinge peptide with concomitant formation of minor amounts of decomposition products. The test samples for HPLC are obtained by precipitation of an aliquot of the reaction mixture with diisopropylether and drying over KOH pellets. The HPLC method employs a C18 column and a mobile phase of 0.1 M sodium phosphate (pH 3.5) in ACN. It is stated that the model studies clearly reveal a significant sequence dependency for the rate of N-alpha-trifluoroacetylation. It is concluded that TFA is not sufficient for quantitative protonation of the amino functions. A mixture of 99% TFA : 6 M HCI : dimethyl sulfide (9 : 1 : 1) used instead of 99% TFA for strongly enhancing the degree of protonation leads to almost quantitative isolation of the hinge peptide by precipitation with diisopropyl ether. A preparation of the sample for injection at the liquid chromatography is not disclosed.

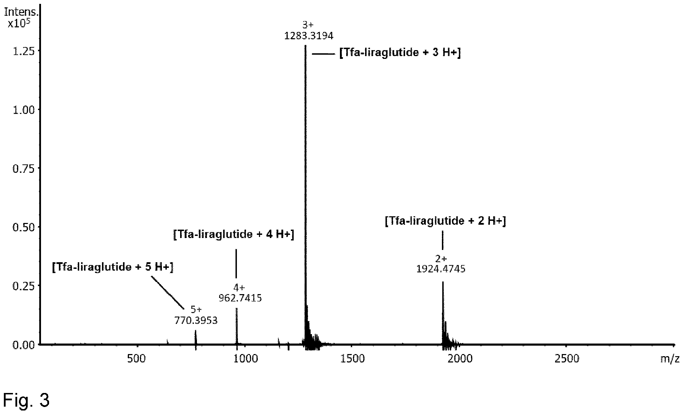

J. Chem. Soc. Perkin Trans. 1, 22 (1993), p. 2843-2849 relates to a synthesis of azapeptides. At a desired decapeptide, i.e. Ser-Glu-Val-Lys-azaMet-Asp-Ala-Glu-Phe-Arg, the final peptide-resin is cleaved with TFA-EDT (95:5) for 16 h to give a crude peptide. An analytical HPLC (C8 column, mobile phase A 0.1% aq. TFA, mobile phase B 90% ACN / 10% H₂O) of this material is conducted and its chromatogram at Fig. 3 a) reveals the presence of the targeted decapeptide and a by-product, which is assigned to be a N-terminally trifluoroacetylated by-product. A semi-preparative HPLC (C8 column, mobile phase A 0.1 % aq. TFA, mobile phase B 90% ACN / 10% H₂O) resulted in 4.2 µmol of targeted decapeptide and 3.0 µmol trifluoroacetylated by-product. A preparation of the sample for injection at the liquid chromatography is not disclosed.

Rapid Communications In Mass Spectroscopy, Vol. 8, 1994, p. 77-81 relates to a matrix-assisted laser desorption ionization for rapid determination of biologically active peptides isolated from support-bound combinatorial peptide libraries. Its figure 3 depicts a MALDI mass spectrum of peptide isolated from a single resin bead. An ion corresponding to H₂N-HHPQFXXXRB is accompanied inter alia by an ion assigned as the N-trifluoroacetylated derivative. It is said that a partial trifluoroacetylation has occurred during a side chain deprotection step. A general deprotection step is described as exposing 100 mg peptide resin, which resin is acid resistant, to 1 mL of a solution from 10 mL TFA, 0.75 g phenol, 0.5 mL mercaptoacetic acid, 0.5 mL anisole and 0.5 mL water for 2 h. This is followed by washing with 90% TFA, 10% diisopropylethylamine in DMF and DMF. After an antibody-based screening of beads in an aqueous environment, a resulting stained bead is exposed to cyanogen bromide in 0.1 N HCI for resin-cleavage by methionine reaction. The cleaved peptide is redissolved in 0.1% aqueous TFA and an aliquot dried together with an added saturated alpha-cyano-4-hydroxy-cinnamic acid solution in aqueous 30% acetonitrile containing 0.1% TFA.

WO 98-08871 relates to derivatives of GLP-1 and analogues thereof having a lipophilic substituent, which have interesting pharmacological properties, in particular they have a more protracted profile of action than GLP-1 (7-37). In its example 37, Arg³⁴Lys²⁶(N^{epsilon}-(gamma-glutamyl(N^{alpha}-hexadecanoyl)))-GLP-1(7-37)-OH is synthesized by reaction of Arg³⁴-GLP-1 (7-37)-OH with N^{alpha}-hexadecanoyl-Glu(ONSu)-OBu^{t} and a TFA treatment during work-up.

Tetrahedron Letters, 53 (2012), p. 4763-4763 relates to unwanted trifluoroacetylation, which occurred at the N-terminus of prolinyl peptides during detachment from the solid phase. Decapeptides are synthesized on a Rink amide resin. For TFA-mediated peptide cleavage, a cleavage composition of 95% TFA, 2.5% triisopropylsilane and 2.5% H₂O (in case of a methionine-containing peptide: 94% TFA, 2.5% ethanedithiol, 2.5% H₂O, 1% triisopropylsilane) is added to the dried resin in an amount of 3 times the volume of the dried resin. After 3 h at room temperature, the mixture is filtrated and washed with TFA. Cold Et₂O is added to the filtrate in 5 -10 times of the volume of the filtrate and the precipitation is centrifuged and washed with cold Et₂O. The obtained pellet is dissolved in H₂O-ACN and lyophilized until a dry powder is obtained. In an unspecified liquid chromatography with mass trace and UV trace, a trifluoroacetylated by-product is observed in some cases. It is stated that although the peptides synthesized had very similar sequences, the amount of trifluoroacetylated by-product varied considerably. A preparation of the sample for injection at the liquid chromatography is not disclosed.

WO 2015-100876 relates to a method for synthesizing liraglutide. In its example 12, fully protected liraglutide CTC resin is treated 2 hours at room temperature with a TFA-containing lysis solution. Crude liraglutide is obtained by precipitation from a TFA-containing lysis solution with anhydrous ether for 1 hour, washings with anhydrous ether and drying. Its figure 3 depicts a HPLC chromatogram of the crude liraglutide. The area percentage of the main peak liraglutide is stated with 83.03%. The method for the analytical HPLC describes a octadecylsilane-bonded silica gel, 0.1% TFA solution as mobile phase A, acetonitrile as a mobile phase for gradient eluation, a flow rate of 1.0 mL per minute, a detection wavelength of 220 nm, a column temperature of 30°C and 20 µL of injection volume of the test solution. A preparation of the test solution respectively the sample for injection at the liquid chromatography is not disclosed.

CN 105111303 A relates to a solid-liquid combined chemical preparation method for liraglutide. Its example 4 discloses a solid phase synthesis of a N-terminally trifluoroacetylated, fully protected liraglutide precursor without a palmitoyl substitution conjugated to a solid phase resin, i.e. Tfa-His(Trt)-Ala-Glu(OtBu)-Gly-Thr(tBu)-Phe-Thr(tBu)-Ser(tBu)-Asp(OtBu)-Val-Ser(tBu)-Ser(tBu)-Tyr(tBu)-Leu-Glu(OtBu)-Gly-Gln(Trt)-Ala-Ala-Lys(N-epsilon-(gamma-Glu(N-alpha-Boc)-OtBu)-Glu(OtBu)-Phe-Ile-Ala-Trp(Boc)-Leu-Val-Arg(Pbf)-Gly-Arg(Pbf)-Gly-[Wang resin]. Inter alia, trifluoroacetic anhydride is employed. In example 5, a crude N-terminally trifluoroacetylated liraglutide precursor without a palmitoyl substitution is obtained from the material of example 4 by an acidolysis with TFA : TIS : H₂O = 95 : 2.5 : 2.5 (volume ratio). In example 8, the crude N-terminally trifluoroacetylated liraglutide precursor without a palmitoyl substitution is purified by a C18 preparative column with a 0.1% TFA / water - 0.1% TFA / ACN system followed by freeze-drying. In example 10, the purified N-terminally trifluoroacetylated liraglutide precursor without a palmitoyl substitution, i.e. Tfa-His-Ala-Glu-Gly-Thr-Phe-Thr-Ser-Asp-Val-Ser-Ser-Tyr-Leu-Glu-Gly-Gln-Ala-Ala-Lys(gamma-Glu-OH)-Glu-Phe-Ile-Ala-Trp-Leu-Val-Arg-Gly-Arg-Gly-OH (SEQ ID NO: 2), is dissolved in an aqueous 10% ACN solution (56 g resp. 15 mmol in 1000 mL), the pH value is adjusted to 11 with aqueous Na₂CO₃ solution, a solution of palmitoyl O-succinate in THF (7.1 g resp. 30 mmol in 150 mL) is added and after 3 h, glycine (2.25 g resp. 30 mmol) is added and stirred for 30 minutes with monitoring the end of the reaction. Then, 150 mL piperidine are added and stirred at room temperature until the end of an alkaline hydrolysis monitored by HPLC. The reaction mixture is filtered and the filtrated desalted by ultrafiltration with the volume being kept constant with pure water. After a purification by a C8 preparative column, liraglutide is obtained. After a similar procedure in examples 6, 7 and 9, another batch of purified and freeze-dried N-terminally trifluoroacetylated liraglutide precursor without a palmitoyl substitution is obtained. In example 11, the N-terminally trifluoroacetylated liraglutide precursor without a palmitoyl substitution is dissolved in an aqueous 10% ACN solution (24.3 g resp. 6.5 mmol in 500 mL), a 10% EDPA / NMP solution is added to a pH value of 11, a solution of palmitoyl O-succinate in THF (4.6 g resp. 13 mmol in 80 mL) is added and after 3 h, glycine (0.98 g resp. 13 mmol) is added and stirred for 30 minutes with monitoring the end of the reaction. Then, 61 mL piperidine are added and stirred at room temperature until the end of an alkaline hydrolysis monitored by HPLC. The reaction mixture is filtered and the filtrated desalted by ultrafiltration with the volume being kept constant with pure water. After a purification by a C8 preparative column, liraglutide is obtained.

CN 105732798 A relates to a synthetic method for liraglutide, which includes the steps of condensing various liraglutide intermediates, deprotecting and cracking full-protected liraglutide, purifying the liraglutide and freeze-drying. In its example 11, a full-protected liraglutide, which is not linked to a resin, is added to a deprotection reagent of TFA : benzyl sulfide : water: EDT = 92 : 4: 2: 2 and kept for 1.5 hours at room temperature. Afterwards, the mixture is poured into pre-cooled 0 °C anhydrous ether and stirred for 3 hours at room temperature. This is followed by centrifugation, washings with water and ether and drying in vacuum to obtain a white solid.

CN 106699871 A relates to a preparation method for liraglutide, which includes the steps of carrying out solid-phase synthesis to form four peptide segments of liraglutide, coupling stepwise various peptide segments to obtain an all-protected liraglutide peptide resin and carrying out cracking, purification and freeze-drying to obtain the liraglutide. In its example 1, a fully protected liraglutide peptide resin is stirred in TFA : EDT : thioanisole : TIS : H₂O = 85 : 5 : 4 : 2 : 4 at room temperature for 2.5 hours. After filtration and washing with TFA, the combined filtrate is added to pre-cooled anhydrous ether and allowed to settle for 2 hours. After a filtration and washing three times with anhydrous ether, drying in vacuum results to obtain a crude liraglutide. In its comparative example 2, the same lysis of a fully protected liraglutide peptide is applied to obtain a crude liraglutide.

WO 2017-162650 relates to a method for preparing glucagon-like peptide, comprising precipitation of the peptide or of a precursor peptide by means of mixing with an antisolvent comprising diisopropyl ether and acetonitrile. In its example 2, protected liraglutide peptide resins are treated with cleavage compositions for 3 hours at room temperature. Addition of different antisolvents initiates a precipitation and the resulting slurry is stirred for 2 hours at room temperature prior to a filtration. After washing with the respective ether and filtration, the obtained crude liraglutide is dried under vacuum at room temperature and analyzed for purity and TFA content by analytical HPLC. A preparation of the sample for injection at the liquid chromatography is not disclosed.

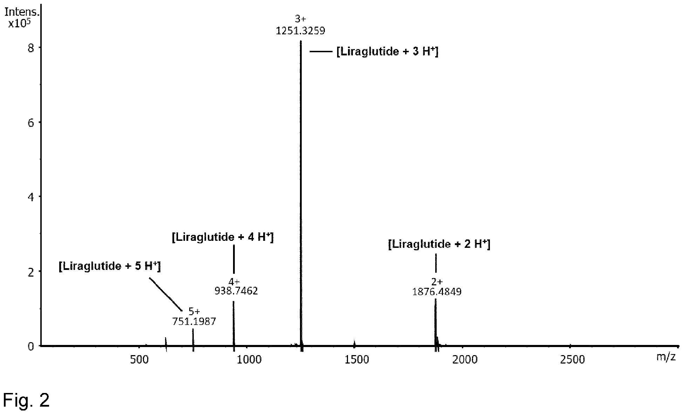

WO 2017-162653 relates to a method of purifying a glucagon-like peptide 1 analog by a two dimensional reversed phase high performance liquid chromatograph protocol. Its example 1 discloses a dissolving of crude liraglutide peptide produced by Fmoc SPPS (purity >= 60%) in a buffer A consisting of 3 % (v/v) acetonitrile in an aqueous solution of a buffer. A general buffer concentration between 20 and 400 mM in the aqueous solutions depending on the nature of the mobile phase buffer is stated. In its table 2, the buffers are NH₄HCO₃, (NH₄)₃PO₄, NH4OAc, TEAP, AcOH, H₃PO₄ and TFA. The obtained solutions are loaded onto a C8 or C18 column for a preparative purification and obtained fractions are individually analyzed by reversed phase UHPLC. In its example 2, crude liraglutide produced by Fmoc SPPS (purity > 60%) is used and the sample loading buffer is aqueous ammonium phosphate at pH 7.5. In its Fig. 2, a chromatogram from the analytical RP-UHPLC of the crude liraglutide preparation used as starting material is depicted. A preparation of the sample for injection at the analytical RP-UHPLC liquid chromatography is not disclosed. In its example 3, crude liraglutide produced by Fmoc SPPS is used as a starting material. A sample loading buffer of aqueous ammonium phosphate pH 7.7 is stated. The general description prefers to dissolve dried crude liraglutide peptide in a phosphate buffer AB0 at a pH selected from the range of 6.6-7.9 (p. 14 / I. 20) or to dissolve a dried crude liraglutide preparation in aqueous buffers of a pH of 6.6-7.9 (p. 15 / I. 33).

CN 107290438 A relates to a method for analyzing polypeptide-related substances by high-performance liquid chromatography and addresses the assurance of liraglutide products' quality in view of the safety of patients' medication. In its experimental part, liraglutide with batch number CS6G809 from Novo Nordisk is dissolved in a solution of 1.42 g NaH₂PO₄ (-> resulting in around 12 mM / L), 14 g of propylene glycol and 5.5 g phenol in 1 L of water and adjusted to pH 8.15 by 1 M NaOH solution. 10 µL of the solution with a concentration of 0.5 mg liraglutide per 1 mL are injected in a series of analytical HPLC methods. In a comparative example, 20 µL of the solution are injected.

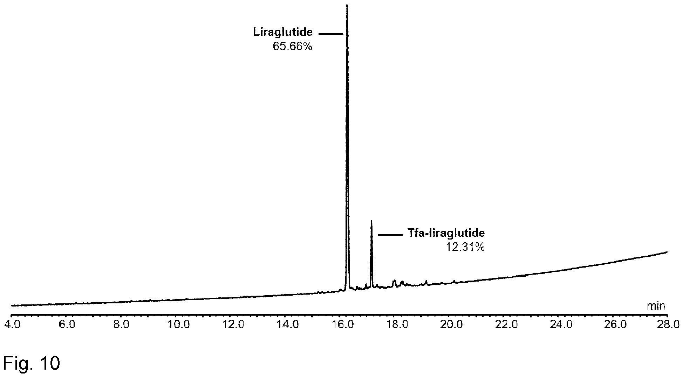

Pharmaceutical Research, 37 (2020), 120 relates to an investigation of the impact of production methods on the stability and impurities of liraglutide drug substances and products. In its figure 10, a chromatogram of a liquid chromatography with mass spectroscopy results for liraglutide for inter alia a liraglutide drug substance of a supplier I is depicted. Amongst around 18 impurity peaks, a peak named "TFA(N-term)" is depicted. In the description of materials, the liraglutide drug substance of supplier I is stated as produced synthetically. Furthermore, it its stated that a dissolving of the liraglutide drug substance of supplier I in water leads to a pH value of 4.3. An analytical method for a RP-HPLC-MS is described to comprise a C18 column, water containing 0.1 % formic acid as an eluent, acetonitrile containing 0.1 % formic acid as an eluent, a gradient eluation, a flow rate of 0.1 mL / min and a column temperature of 59 °C. One liraglutide drug product is described as containing phenol, glycerol and phosphate at pH 8.2 and two liraglutide drug products are described as containing phenol, glycerol and phosphate at pH 7.3. A preparation of a sample for injection at the analytical chromatography methods is not disclosed.

Organic Process Research and Development, 2021, 25, p. 1598-1611 relates to copper(II) lysinate and pseudoproline assistance in the convergent synthesis of liraglutide. Its Figure 3 shows a partial HPLC chromatogram of a crude liragutide obtained in a stepwise SPPS and a partial HPLC chromatogram of a crude liraglutide obtained in a convergent approach. The applied analytical method for the HPLC is described in the experimental part by a C18 column, an eluent A TFA-H₂O 0.1% v/v, an eluent B TFA-ACN 0.01% v/v, a detailed gradient scheme and a detection at 224 nm. In the experimental part, the remark "early eluting peak of the solvent DMSO is not integrated" hints that DMSO is used as a solvent for the injected solution. More details for a preparation of the sample for injection at the analytical HPLC is not disclosed. In the supporting information to the article, Figure S16 shows the complete HPLC chromatogram of a crude liragutide obtained in a stepwise SPPS and the complete HPLC chromatogram of a crude liraglutide obtained in a convergent approach. Both chromatograms include a main peak at around a retention time of one minute, which is the sole relevant "early eluting peak" in the respective chromatogram. In Table S1 of the supporting information to the article, the area percentages of the impurities observed in crude liraglutide obtained in a stepwise SPPS are listed. The area percentage of the liraglutide peak is only 35.97% and accompanied by a series of peaks with area percentages around 2%. In Table S2 of the supporting information to the article, the area percentages of the impurities observed in crude liraglutide obtained in a convergent approach are listed. The area percentage of the liraglutide peak is 64.10%. The two main impurities with 7.53% and 5.11% are assigned as acetylated fragments Ac-9-31 and Ac-17-31 in Figure 3 of the article.

CN 113461800 A relates to a synthesis method of liraglutide, which comprises synthesizing several fragments through a solid phase or a liquid phase, sequentially coupling each fragment and other raw materials in a solution system to obtain an N-Tfa protected liraglutide intermediate, and purifying to obtain a pure intermediate product. After acylation with palmitic acid derivative, the Tfa protecting group is removed under an alkaline condition to obtain liraglutide. In its experimental part, 6 g of the N-Tfa protected liraglutide intermediate is dissolved in 100 mL of a solution of water with 10% acetonitrile, 20 mL THF is added at the acylation step with the palmitic acid derivative, 0.15 g of glycine is added for quenching and finally, 20 mL piperidine is added and followed by monitoring by HPLC the end point of alkali hydrolysis.

It has now surprisingly been found that an observation of a peptide-related impurity in a precipitated liraglutide mixture, which is obtained from a solid phase conjugated protected liraglutide precursor by cleavage with trifluoroacetic acid, an analytical HPLC depends on the way how the analytical HPLC sample is prepared. The peptide-related impurity itself has been identified by liquid chromatography mass spectroscopy as N-terminally trifluoroacetylated liraglutide (Tfa-liraglutide). The peptide-related impurity has been observed when a test sample taken from the precipitated liraglutide mixture has been dissolved in a solution for dissolving or diluting, which contains water and at least 15 vol.% of acetonitrile and a buffer salt solution adjusted to pH 7.8. Without a certain amount of acetonitrile in the solution for dissolving or diluting, Tfa-liraglutide was observed by the analytical HPLC method only at a significantly lower level. Without the buffer salt solution in the solution for dissolving or diluting, Tfa-liraglutide was only observed by the analytical HPLC method at a significantly lower level. A measurement of the pH value of the analytical HPLC sample has revealed that the pH value of the analytical HPLC sample is around 4, when the precipitated liraglutide mixture is dissolved at an amount of 1.0 mg / mL. A quantitative measurement of the trifluoroacetic acid content of the precipitated liraglutide mixture has revealed a relatively high residual content of trifluoroacetic acid, which makes the observed acidic pH value plausible.

It has now surprisingly further been found that the content of Tfa-liraglutide in the precipitated liraglutide mixture, which is obtained from a solid phase conjugated protected liraglutide precursor by cleavage with trifluoroacetic acid, can be reduced. A prolonged stirring of a solid phase conjugated protected liraglutide precursor in a cleavage solution which contains a high amount of trifluoroacetic acid but also some water leads even to an increase of the content of Tfa-liraglutide in the afterwards isolated precipitated liraglutide mixture. In contrast, a prolonged stirring after addition of an antisolvent leads to a reduction of the content of Tfa-liraglutide in the afterwards isolated precipitated liraglutide mixture. Thus, this precipitated liraglutide mixture contains already a reduced amount of the peptide-related impurity Tfa-liraglutide. While it seems that dissolving a precipitated liraglutide mixture in acidic aqueous solution with a pH value around 4 leads also to a decrease of Tfa-liraglutide, this treatment in an acidic aqueous solution is one additional acidic treatment. This matters as an acidic treatment around the isoelectric point of liraglutide, which is reported to be 4.0, is prone for promoting aggregation of liraglutide.

An embodiment of the invention is a method for manufacturing a precipitated mixture PrecMixt-1, which contains liraglutide or a salt thereof as component (A), comprising the steps
(i) providing a solid phase conjugated protected liraglutide precursor;
(ii) treating the solid phase conjugated protected liraglutide precursor with a cleavage composition CleaComp-1, which comprises the components
   (a) 75 to 98.7 parts by volume of trifluoroacetic acid,
   (b) 1 to 14 parts by volume of water,
   (c) optionally one or more further scavengers different to water, wherein parts by volume are based on the volume of the cleavage composition CleaComp-1, which is 100 parts by volume, and the sum of the components (a), (b) and (c) is smaller than or exactly 100 parts by volume, at a temperature Temp-(ii) between 0 °C and 35 °C to obtain a cleavage suspension CleaSusp-1;
(iii) removing solid parts from the cleavage suspension CleaSusp-1 to obtain a cleavage solution CleaSolu-1;
(iv) mixing the cleavage solution CleaSolu-1 with an antisolvent to obtain a precipitation suspension PrecSusp-1;
(v) stirring the precipitation suspension PrecSusp-1 at a temperature Temp-(v) between 0 °C and 35 °C for a time period Time-(v), which is at least 7 h and lasts further until an analytical HPLC sample results in a HPLC-UV chromatogram with an area percentage of component (A)
   (A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof,
      and an area percentage of component (B)
   (B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO: 4) or a salt thereof,
      with a relative area percentage ratio (B) to (A) in the range of 0.080 to 0.010, to obtain a precipitation suspension PrecSusp-2,
      when the analytical HPLC sample is prepared by
         - taking a test sample from the stirred precipitation suspension,
         - filtering the test sample to obtain a test sample precipitate,
         - drying the test sample precipitate under vacuum,
         - dissolving 1 mg of the dried test sample precipitate in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample;
(vi) isolating the precipitation from the precipitation suspension PrecSusp-2 to obtain the precipitated mixture PrecMixt-1.

Liraglutide (CAS-No. 204656-20-2) is a polypeptide, i.e. His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (SEQ ID NO: 3). Liraglutide's amino acid sequence of its backbone respectively its plain peptide strand is written in one-letter code:
HAEGTFTSDVSSYLEGQAAKEFIAWLVRGRG (SEQ ID NO: 1).

A salt of liraglutide is for example liraglutide trifluoroacetate (CAS-No. 1860838-79-4), liraglutide acetate (CAS-No. 1997361-86-0), liraglutide tromethamine (CAS-No. 2080345-76-0), liraglutide phosphate, liraglutide chloride, liraglutide sulfate or liraglutide formate.

The solid phase conjugated protected liraglutide precursor possesses the amino acid sequence of liraglutide including a hexadecanoyl-gamma-glutamyl side chain substitution at the epsilon amino group of lysine, is conjugated to a linking group of a solid phase resin and at least one side chain of the amino acids His, Glu, Thr, Ser, Asp, Tyr, Gln, Trp or Arg carries a protecting group. Preferably, the solid phase conjugated protected liraglutide precursor is linked via the carboxylic acid of its C-terminal glycine to the linking group of the solid phase resin.

It is understood that the protection group as well as the linking group of the solid phase resin are acid labile. The protecting group as well as the linking group is cleavable by a composition, which comprises
(a) 75 to 98.7 parts by volume of trifluoroacetic acid,
(b) 1 to 14 parts by volume of water,
(c) optionally one or more further scavengers different to water,
wherein parts by volume are based on the volume of the composition, which is 100 parts by volume, and the sum of the components (a), (b) and (c) is smaller than or exactly 100 parts by volume.

A protecting group is for example an amine protecting group, a carboxylic acid protecting group, a hydroxy protecting group or an amide protecting group. An amine protecting group is for example tert-butyloxycarbonyl, trityl or 2,2,4,6,7-pentamethyl-2,3-dihydrobenzfuran-5-sulfonyl. A carboxylic acid protecting group is for example tert-butyl, trityl or 3-methylpent-3-yl. A hydroxyl protecting group is for example tert-butyl, 3-methylpent-3-yl, trityl or in case of serine or threonine 1,1-dimethylmethdiyl, which is covalently linked to the beta oxygen atom of serine or threonine and covalently linked to the alpha-nitrogen of serine or threonine (pseudoproline or Psi(Me,Me)pro). An amide protecting group is for example trityl.

Preferably, the solid phase conjugated protected liraglutide precursor is a compound of formula I
R^{N00}-His(R^{N01})-Ala-Glu(OR^{O03})-Gly-Thr(R^{O05})-Phe-Thr(R^{O07})-Ser(R^{O08})-Asp(OR^{O09})-Val-Ser(R^{O11})-Ser(R^{O12})-Tyr(R^{O13})-Leu-Glu(OR^{O15})-Gly-Gln(R^{N17})-Ala-Ala-Lys(palmitoyl-gamma-Glu(OR^{O20}))-Glu(OR^{O21})-Phe-Ile-Ala-Trp(R^{N25})-Leu-Val-Arg(R^{N28})-Gly-Arg(R^{N30})-Gly-[solid phase resin] (I),
wherein
R^{N00}, R^{N01}, R^{N25}, R^{N28} and R^{N30} are independently from each other and an amine protecting group or H,
R^{O03}, R^{O09}, R^{O15}, R^{O20} and R^{O21} are independently from each other and a carboxylic acid protecting group or H and at least one of R^{O03}, R^{O09}, R^{O15}, R^{O20} and R^{O21} is a carboxylic acid protecting group,
R^{O05}, R^{O07}, R^{O08}, R^{O11}, R^{O12} and R^{O13} are independently from each other and a hydroxy protecting group or H,
R^{N17} is an amide protecting group or H.

At formula I, H means a hydrogen atom as a substituent respectively that there is no protecting group. For example, 'R^{N00}-His(R^{N01})-Ala-Glu(OR⁰⁰³)-...' with R^{N00}, R^{N01} and R^{O03} being H is 'H-His(H)-Ala-Glu(OH)-...' respectively 'His-Ala-Glu-...'.

R^{N00} is preferably an amine protecting group different to trifluoroacetyl or H, very preferably tert-butyloxycarbonyl, H or trityl, very preferably tert-butyloxycarbonyl or H and particularly tert-butyloxycarbonyl.

R^{N01} is preferably tert-butyloxycarbonyl, trityl or H, very preferably tert-butyloxycarbonyl or trityl, and very preferably tert-butyloxycarbonyl.

R^{N25} is preferably tert-butyloxycarbonyl, trityl or H, more preferably tert-butyloxycarbonyl or H and very preferably tert-butyloxycarbonyl.

R^{N28} is preferably tert-butyloxycarbonyl, trityl or 2,2,4,6,7-pentamethyl-2,3-dihydrobenzfuran-5-sulfonyl, very preferably tert-butyloxycarbonyl or 2,2,4,6,7-pentamethyl-2,3-dihydrobenzfuran-5-sulfonyl and very preferably 2,2,4,6,7-pentamethyl-2,3-dihydrobenzfuran-5-sulfonyl.

R^{N30} is preferably tert-butyloxycarbonyl, trityl or 2,2,4,6,7-pentamethyl-2,3-dihydrobenzfuran-5-sulfonyl, very preferably tert-butyloxycarbonyl or 2,2,4,6,7-pentamethyl-2,3-dihydrobenzfuran-5-sulfonyl and very preferably 2,2,4,6,7-pentamethyl-2,3-dihydrobenzfuran-5-sulfonyl.

R^{O03} is preferably tert-butyl or 3-methylpent-3-yl and more preferably tert-butyl.

R^{O09} is preferably tert-butyl or 3-methylpent-3-yl and more preferably 3-methylpent-3-yl.

R^{O15} is preferably tert-butyl or 3-methylpent-3-yl and more preferably tert-butyl.

R^{O20} is preferably tert-butyl or 3-methylpent-3-yl and more preferably tert-butyl

R^{O21} is preferably tert-butyl or 3-methylpent-3-yl and more preferably tert-butyl.

R^{O05} is preferably tert-butyl, 3-methylpent-3-yl, trityl, 1,1-dimethylmethdiyl, which is covalently linked to the beta oxygen atom of threonine and covalently linked to the alpha-nitrogen of threonine, or H, more preferably tert-butyl or 1,1-dimethylmethdiyl, which is covalently linked to the beta oxygen atom of threonine and covalently linked to the alpha-nitrogen of threonine, and very preferably tert-butyl.

R^{O07} is preferably tert-butyl, 3-methylpent-3-yl, trityl, 1,1-dimethylmethdiyl, which is covalently linked to the beta oxygen atom of threonine and covalently linked to the alpha-nitrogen of threonine, or H, more preferably tert-butyl or 1,1-dimethylmethdiyl, which is covalently linked to the beta oxygen atom of threonine and covalently linked to the alpha-nitrogen of threonine, and very preferably 1,1-dimethylmethdiyl, which is covalently linked to the beta oxygen atom of threonine and covalently linked to the alpha-nitrogen of threonine.

R^{O08} is preferably tert-butyl, 3-methylpent-3-yl, trityl, 1,1-dimethylmethdiyl, which is covalently linked to the beta oxygen atom of serine and covalently linked to the alpha-nitrogen of serine, or H, more preferably tert-butyl or 1,1-dimethylmethdiyl, which is covalently linked to the beta oxygen atom of serine and covalently linked to the alpha-nitrogen of serine, and very preferably tert-butyl.

R^{O11} is preferably tert-butyl, 3-methylpent-3-yl, trityl, 1,1-dimethylmethdiyl, which is covalently linked to the beta oxygen atom of serine and covalently linked to the alpha-nitrogen of serine, or H, more preferably tert-butyl or 1,1-dimethylmethdiyl, which is covalently linked to the beta oxygen atom of serine and covalently linked to the alpha-nitrogen of serine, and very preferably tert-butyl.

R^{O12} is preferably tert-butyl, 3-methylpent-3-yl, trityl, 1,1-dimethylmethdiyl, which is covalently linked to the beta oxygen atom of serine and covalently linked to the alpha-nitrogen of serine, or H, more preferably tert-butyl or 1,1-dimethylmethdiyl, which is covalently linked to the beta oxygen atom of serine and covalently linked to the alpha-nitrogen of serine, and very preferably tert-butyl.

R^{O13} is preferably tert-butyl, 3-methylpent-3-yl, trityl or H, more preferably tert-butyl, 3-methylpent-3-yl or H, very preferably tert-butyl or 3-methylpent-3-yl and particularly tert-butyl.

R^{N17} is preferably trityl or H and more preferably trityl.

Preferably,
R^{N00} is an amine protecting group or H,
R^{N01} and R^{N25}, are independently from each other and an amine protecting group or H,
R^{N28} and R^{N30} are independently from each other and an amine protecting group, R^{O03}, R^{O09}, R^{O15}, R^{O20} and R^{O21} are independently from each other and a carboxylic acid protecting group,
R^{O05}, R^{O07}, R^{O08}, R^{O11}, R^{O12} and R^{O13} are independently from each other and a hydroxy protecting or H,
R^{N17} is an amide protecting group or H.

More preferably,
R^{N00} is an amine protecting group or H,
R^{N01}, R^{N25}, R^{N28} and R^{N30} are independently from each other and an amine protecting group,
R^{O03}, R^{O09}, R^{O15}, R^{O20} and R^{O21} are independently from each other and a carboxylic acid protecting group,
R^{O05}, R^{O07}, R^{O08}, R^{O11}, R^{O12} and R^{O13} are independently from each other and a hydroxy protecting group,
R^{N17} is an amide protecting group or H.

Preferably, the carboxylic acid protecting group is tert-butyl or 3-methylpent-3-yl.

The solid phase resin possesses a support part and a linking group, which is covalently bound to the support part. The support part is for example polystyrene, polystyrene-polyethylene glycol composites, polyethylene glycol, cross-linked ethoxylated polyacrylate, polyamide or polydimethylacrylamide. Preferably, the support part is a polystyrene, which is crosslinked with 1% divinylbenzene, very preferably in the form of beads, very preferably with a size distribution of 200 to 400 mesh or 100 to 200 mesh. The linking group is for example 2-chlorotrityl ((2-chlorophenyl)diphenylmethanol; in case of polystyrene as the support part, one of 2-chlorotrityl's two chloro-free phenyl groups being a phenyl group of polystyrene), 2-chlorotrityl amidomethyl (4-[(2-chlorophenyl)hydroxyphenyl-methy]-N-methylbenzamide), a Wang linker (4-hydroxy-benzylalcohol) or a Sasrin linker (4-hydroxy-2-methoxybenzylalcohol). The solid phase resin is for example a 2-chlorotrityl resin, a 2-chlorotrityl amidomethyl resin, a Sasrin resin or a Wang resin.

A resin load of the solid phase resin defines the number of conjugated protected liraglutide precursors per weight of solid phase resin prior to a conjugation of the first amino acid or the first peptide. The resin load can be varied by using a specific solid phase resin and by a full or submolar loading of the specific solid phase, i.e. not all available linking groups are used. The latter is typically accompanied by a blocking respectively capping of the idle linking groups, which avoids that the idle linking groups interfere with the progressing solid phase synthesis of the protected liraglutide precursor. Preferably, the resin load of the solid phase resin is between 0.2 mmol / g and 0.9 mmol / g, more preferably between 0.3 mmol / g and 0.8 mmol / g, very preferably 0.4 mmol / g to 0.7 mmol / g, particularly between 0.5 mmol / g to 0.7 mmol /g and more particularly between 0.6 mmol / g and 0.7 mmol / g.

Preferred is a method for manufacturing a precipitated mixture PrecMixt-1, wherein the solid phase conjugated protected liraglutide precursor is a compound of formula (I)
R^{N00}-His(R^{N01})-Ala-Glu(OR^{O03})-Gly-Thr(R^{O05})-Phe-Thr(R^{O07})-Ser(R^{O08})-Asp(OR^{O09})-Val-Ser(R^{O11})-Ser(R^{O12})-Tyr(R^{O13})-Leu-Glu(OR^{O15})-Gly-Gln(R^{N17})-Ala-Ala-Lys(palmitoyl-gamma-Glu(OR^{O20}))-Glu(OR^{O21})-Phe-Ile-Ala-Trp(R^{N25})-Leu-Val-Arg(R^{N28})-Gly-Arg(R^{N30})-Gly-[solid phase resin] (I),
wherein
R^{N00}, R^{N01}, R^{N25}, R^{N28} and R^{N30} are independently from each other and an amine protecting group or H,
R^{O03}, R^{O09}, R^{O15}, R^{O20} and R^{O21} are independently from each other and a carboxylic acid protecting group or H and at least one of R^{O03}, R^{O09}, R^{O15}, R^{O20} and R^{O21} is a carboxylic acid protecting group,
R^{O05}, R^{O07}, R^{O08}, R^{O11}, R^{O12} and R^{O13} are independently from each other and a hydroxy protecting group or H,
R^{N17} is an amide protecting group or H.

Preferred is a method for manufacturing a precipitated mixture PrecMixt-1, wherein
R^{N00} is an amine protecting group or H,
R^{N01}, R^{N25}, R^{N28} and R^{N30} are independently from each other and an amine protecting group,
R^{O03}, R^{O09}, R^{O15}, R^{O20} and R^{O21} are independently from each other and a carboxylic acid protecting group,
R^{O05}, R^{O07}, R^{O08}, R^{O11}, R^{O12} and R^{O13} are independently from each other and a hydroxy protecting group,
R^{N17} is an amide protecting group or H.

Preferred is a method for manufacturing a precipitated mixture PrecMixt-1, wherein the carboxylic acid protecting group is tert-butyl or 3-methylpent-3-yl.

Treating the solid phase conjugated protected liraglutide precursor with a cleavage composition CleaComp-1 at step (ii) is typically conducted by adding the cleavage composition CleaComp-1, preferably in a liquid form, to the solid phase conjugated protected liraglutide precursor, or adding the solid phase conjugated protected liraglutide precursor to the cleavage composition CleaComp-1, and stirring the resulting mixture at the temperature Temp-(ii) for a time period Time-(ii). The time period Time-(ii) is chosen sufficiently long to assure an essentially complete cleavage of a protected liraglutide precursor from the solid phase by breaking the covalent bond to the linking group of the solid phase resin and an essentially complete removal of the protecting groups of the solid phase conjugated protected liraglutide precursor or the protected liraglutide precursor already cleaved from the solid phase resin. The cleavage from the solid phase resin and the removal of the protecting groups can occur simultaneously. Often, the cleavage from the solid phase resin is already completed, while the removal of all protecting groups takes more time. The result is the cleavage suspension CleaSusp-1. The solid phase resin, from which the protected liraglutide precursor is cleaved, forms the solid parts of the CleaSusp-1, whereas liraglutide-containing materials are dissolved in the solution part of the CleaSusp-1. The temperature Temp-(ii) is preferably between 4 °C and 33 °C, more preferably between 8 °C and 32 °C, very preferably between 12 °C and 31 °C, particularly between 16 °C and 30 °C, more particularly between 20 °C and 29 °C and very particularly between 24 °C and 28 °C. The time period Time-(ii) is preferably between 20 minutes and 360 minutes, more preferably between 30 minutes and 300 minutes, very preferably between 40 minutes and 240 minutes, particularly between 50 minutes and 180 minutes, more particularly between 60 minutes and 120 minutes and very particularly between 80 minutes and 100 minutes. The weight of the solid phase conjugated protected liraglutide precursor, i.e. the weight of the material to be cleaved, includes the solid phase resin and the covalently linked protected liraglutide precursor. The weight to volume ratio between the solid phase conjugated protected liraglutide precursor and the cleavage composition CleaComp-1 is preferably 1 g of the solid phase conjugated protected liraglutide precursor to 5 to 18 mL of CleaComp-1, more preferably to 6 to 15 mL of CleaComp-1, very preferably 6.5 to 12 mL of CleaComp-1, particularly 7 to 10 mL of CleaComp-1, more particularly 7.5 to 9 mL of CleaComp-1 and very particularly to 8 mL of CleaComp-1. Preferably, the treating at step (ii) occurs under a chemically inert gas atmosphere, for example under nitrogen.

Preferred is a method for manufacturing a precipitated mixture PrecMixt-1, wherein step (ii) takes place at a time period Time-(ii), which is between 20 and 360 minutes.

A scavenger herein is understood as a compound, which is added to the cleavage composition CleaComp-1 in order to suppress side reactions during cleavage from the solid phase resin and/or during removal of protecting groups. Water, i.e. component (b), acts as a scavenger, i.e. a first scavenger.

The cleavage composition CleaComp-1 is for example a composition consisting of triflouroacetic acid / water / ethane-1,2-dithiol (87.6:6.0:6.4 vol/vol/vol), a composition consisting of triflouroacetic acid / water / ethane-1,2-dithiol (90:5:5 vol/vol/vol), a composition consisting of trifluoroacetic acid / water/ triisopropylsilane (90:5:5 vol/vol/vol), a composition consisting of trifluoroacetic acid / water / phenol (90:5:5 vol/vol/vol), a composition consisting of trifluoroacetic acid / water / ethane-1,2-dithiol / triisopropylsilane (90:4:3:3 vol/vol/vol/vol), a composition consisting of trifluoroacetic acid / water / ethane-1,2-dithiol / triisopropylsilane (90:5:2.5:2.5 vol/vol/vol/vol) or a composistion consisting of trifluoroacetic acid / water / thioanisole / phenol / ethane-1,2-dithiol (82.5:5:5:5:2.5 vol/vol/vol/vol/vol).

The cleavage composition comprises preferably as component (c) a scavenger, which is different to water. The scavenger, which is different to water, i.e. component (c), acts also as a scavenger, i.e. a second scavenger. A scavenger different to water is for example a thiol, a silane, phenol, a cresol, anisole, thioanisole, 3-methylindole, N-acetylindole, tryptamine, methyl N-acetyl-tryptophanate or a mixture thereof. The thiol is for example ethane-1,2-dithiol, 1,4-dithiothreitol, 1,4-dithioerythrit, 2,2'-ethylendioxy-diethanthiol, thioglycol, 1-octanethiol or 1-dodecanethiol. The thiol is preferably ethane-1,2-dithiol. The silane is for example triisopropylsilane or triethylsilane. The silane is preferably triisopropylsilane. The cresole is for example ortho-cresol, meta-cresol or para-cresol. The cresol is preferably meta-cresol.

The cleavage composition CleaComp-1 comprises preferably as component (c) a thiol, a silane, phenol, a cresol, anisole, thioanisole, 3-methylindole, N-acetylindole, tryptamine, methyl N-acetyl-tryptophanate or a mixture thereof. More preferably, the cleavage composition CleaComp-1 comprises as component (c) a thiol, a silane, phenol, anisole, thioanisole or a mixture thereof. Very preferably, the cleavage composition CleaComp-1 comprises as component (c) a thiol, triisopropylsilane or a mixture thereof. More particularly, the cleavage composition CleaComp-1 comprises as component (c) a thiol. More particularly, the cleavage composition comprises as component (c) triisopropylsilane. Very particularly, the cleavage composition CleaComp-1 comprises as component (c) ethane-1,2-dithiol. Especially, the cleavage composition CleaComp-1 comprises as component (c) a mixture of ethane-1,2-dithiol and triisopropylsilane.

The amount of trifluoroacetic acid as component (a) in the cleavage composition CleaComp-1 is preferably 80 to 96.5 parts by volume based on 100 parts by volume of the cleavage composition CleaComp-1, more preferably 82 to 96 parts by volume, very preferably 84 to 95 parts by volume, particularly 85 to 93 parts by volume, more particularly 86 to 91 parts by volume and very particularly 87 to 89 parts by volume. The amount of water as component (b) in the cleavage composition CleaComp-1 is preferably 2 to 10 parts by volume based on 100 parts by volume of the cleavage composition CleaComp-1, more preferably 2 to 9 parts by volume, very preferably 3 to 8 parts by volume, particularly 5 to 7 parts by volume and more particularly 6 parts by volume. The amount of the scavenger different to water as component (c) - if present - is preferably 0.1 to 15 parts by volume based on 100 parts of volume of the cleavage composition CleaComp-1, more preferably 1 to 13 parts by volume, very preferably 1 to 10 parts by volume, particularly 2 to 9 parts by volume, more particularly 4 to 8 parts by volume, very particularly 5 to 7 parts by volume and especially 6 to 7 parts by volume.

Preferably, the cleavage composition CleaComp-1 comprises
(a) 80 to 96.5 parts by volume of trifluoroacetic acid,
(b) 2 to 10 parts by volume of water,
(c) 1 to 10 parts by volume of a thiol,
wherein parts by volume are based on the volume of the cleavage composition CleaComp1, which is 100 parts by volume, and the sum of the components (a), (b) and (c) is below or exactly 100 parts by volume.

More preferably, the cleavage composition CleaComp-1 comprises
(a) 80 to 95 parts by volume of trifluoroacetic acid,
(b) 2 to 9 parts by volume of water,
(c) 2 to 9 parts by volume of ethane-1,2-dithiol.

Very preferably, the cleavage composition CleaComp-1 comprises
(a) 84 to 95 parts by volume of trifluoroacetic acid,
(b) 3 to 8 parts by volume of water,
(c) 4 to 8 parts by volume of ethane-1,2-dithiol.

Preferred is a method for manufacturing a precipitated mixture PrecMixt-1, wherein at step (ii) the cleavage composition CleaComp-1 comprises
(c) 0.1 to 15 parts by volume of a scavenger different to water, which is a thiol, a silane, phenol, a cresol, anisole, thioanisole, 3-methylindole, N-acetylindole, tryptamine, methyl N-acetyl-tryptophanate or a mixture thereof,
wherein parts by volume are based on the volume of the cleavage composition CleaComp1, which is 100 parts by volume, and the sum of the components (a), (b) and (c) is below or exactly 100 parts by volume.

Removing solid parts from the cleavage suspension CleaSusp-1 at step (iii) is typically filtering the CleaSusp-1 to separate into a solid filter residue and a liquid filtrate. Optionally, the solid filter residue is washed with trifluoroacetic acid or a solution of trifluoroacetic acid and a scavenger, for example with the cleavage composition CleaComp-1 and thus a washing liquid is obtained. The liquid filtrate, which is combined with the optional washing liquid, is the cleavage solution CleaSolu-1. Preferably, removing solid parts from the cleavage suspension CleaSusp-1 comprises a washing of the removed solid parts, more preferably the washing occurs with trifluoroacetic acid or a solution of trifluoroacetic acid and a scavenger, very preferably the washing occurs with trifluoroacetic acid. The amount of the trifluoroacetic acid or the solution of trifluoroacetic acid and a scavenger, which is employed for the washing of the removed solid parts, is preferably in a volume ratio of 0.08 mL to 0.4 mL for 1.0 mL of cleavage composition CleaComp-1 employed at step (ii), more preferably in a volume ratio of 0.10 mL to 0.35 mL for 1.0 mL of cleavage composition CleaComp-1 employed at step (ii) and very preferably in a volume ratio of 0.12 mL to 0.28 mL for 1.0 mL of cleavage composition CleaComp-1 employed at step (ii). The amount of the trifluoroacetic acid or the solution of trifluoroacetic acid and a scavenger, which is employed for the washing of the removed solid parts, is preferably in a volume to weight ratio of 0.64 mL to 3.2 mL for 1.0 g of the solid phase conjugated protected liraglutide precursor employed at step (ii), more preferably in a volume to weight ratio of ratio of 0.8 mL to 2.8 mL for 1.0 g of the solid phase conjugated protected liraglutide precursor employed at step (ii) and very preferably in a volume to weight ratio of ratio of 0.96 mL to 2.2 mL for 1.0 g of the solid phase conjugated protected liraglutide precursor employed at step (ii). Preferably, the washing of the removed solid parts is divided at least in a first washing and a second washing, very preferably in a first washing and a second washing. Preferably, the removing at step (iii) occurs under a chemically inert gas atmosphere, for example under nitrogen.

Mixing the cleavage solution CleaSolu-1 with an antisolvent at step (iv) is typically conducted by adding the antisolvent to the cleavage solution CleaSolu-1 under stirring or by adding the cleavage solution CleaSolu-1 to the antisolvent under stirring. An antisolvent is herein understood as a liquid, which induces precipitation of peptides when added to the cleavage solution CleaSolu-1. A suitably chosen temperature Temp-(iv) during the mixing supports the formation of precipitation. The temperature Temp-(iv) during the mixing is preferably kept below 0 °C, more preferably kept below -5 °C, very preferably kept below -8 °C and particularly kept below -10 °C. The temperature Temp-(iv) during the addition is preferably kept between -1 °C and -30 °C, more preferably kept between -6 °C and -29 °C, very preferably kept between -8 °C and -28 °C, particularly kept between -9 °C and -27 °C, more particularly kept between -10 °C and -26 °C and very particularly kept between below -10 °C and -25 °C. Technically, the temperature Temp-(iv) can be maintained by pre-cooling the cleavage solution CleaSolu-1 in a vessel or the antisolvent in a vessel and choosing of the addition rate to keep the desired Temp-(iv). Once the mixing is finished, i.e. essentially all or all of the antisolvent has been added, the temperature of the formed precipitation suspension PresSusp-1 is increased by a warming to at least 0 °C in case Temp-(iv) has been below 0 °C. The warming is relatively fast. This depends also on the size of the batch, i.e. a larger batch size cannot be as fast warmed as a smaller batch size. For example, the warming takes below 120 minutes, preferably between 5 minutes and 90 minutes, more preferably between 10 minutes and 70 minutes, very preferably between 15 minutes and 60 minutes. Preferably, the mixing at step (iv) occurs under a chemically inert gas atmosphere, for example under nitrogen.

The antisolvent is preferably a dialkyl ether or an antisolvent mixture AntiSolv-1, which comprises as component (anti-a) at least 50 parts by volume of a dialkyl ether based on 100 parts of volume for the antisolvent mixture Antisolv-1. More preferably, the antisolvent mixture AntiSolv-1 is a dialkyl ether. The dialkyl ether is for example diisopropyl ether, dipropyl ether, tert-butyl methyl ether, ethyl tert-butyl ether, tert-amyl methyl ether, cyclopentyl methyl ether, cyclohexyl methyl ether, diethyl ether or a mixture thereof. The dialkyl ether has preferably a total number of carbon atoms per molecule between 2 and 12, more preferably between 3 and 9, very preferably between 4 and 7, particularly between 4 and 6 and more particularly of 6. The dialkyl ether is preferably diisopropyl ether, dipropyl ether, tert-butyl methyl ether, ethyl tert-butyl ether, tert-amyl methyl ether or diethyl ether or a mixture thereof. The dialkyl ether is more preferably diisopropyl ether, diethylether or tert-butyl methyl ether or a mixture thereof. The dialkyl ether is very preferably diisopropyl ether. The antisolvent mixture AntiSolv-1 comprises as component (anti-b) preferably an aliphatic C₅-C₁₂ hydrocarbon or acetonitrile. The aliphatic C₅-C₁₂ hydrocarbon is for example pentane, hexane, cyclopentane, cyclohexane, heptane, cycloheptane, octane, nonane, decane or a mixture thereof. The aliphatic C₅-C₁₂ hydrocarbon is preferably saturated. The antisolvent mixture AntiSolv-1 comprises as component (anti-b) preferably an aliphatic C₅-C₉ hydrocarbon or acetonitrile. The aliphatic C₅-C₉ hydrocarbon is preferably saturated. Preferably, the antisolvent mixture AntiSolv-1 comprises the components
(anti-a) 50 to 99 parts by volume of dialkyl ether, and
(anti-b) 1 to 50 parts by volume of an aliphatic C₅-C₁₂ hydrocarbon or acetonitrile,
wherein parts by volume are based on the volume of the antisolvent mixture AntiSolv-1, which is 100 parts by volume, and the sum of the components (anti-a) and (anti-b) is below or exactly 100 parts by volume. More preferably, the antisolvent mixture AntiSolv-1 comprises the components
(anti-a) 50 to 99 parts by volume of dialkyl ether, and
(anti-b) 1 to 50 parts by volume of acetonitrile,
wherein parts by volume are based on the volume of the antisolvent mixture AntiSolv-1, which is 100 parts by volume, and the sum of the components (anti-a) and (anti-b) is below or exactly 100 parts by volume. Very preferably, the antisolvent mixture AntiSolv-1 comprises the components
   components
(anti-a) 75 to 83 parts by volume of dialkyl ether, and
(anti-b) 17 to 25 parts by volume of acetonitrile,
wherein parts by volume are based on the volume of the antisolvent mixture AntiSolv-1, which is 100 parts by volume, and the sum of the components (anti-a) and (anti-b) is below or exactly 100 parts by volume.

Preferred is a method for manufacturing a precipitated mixture PrecMixt-1, wherein at step (iv) the antisolvent is a dialkyl ether or an antisolvent mixture AntiSolv-1 comprising the components
(anti-a) 50 to 99 parts by volume of dialkyl ether, and
(anti-b) 1 to 50 parts by volume of an aliphatic C₅-C₁₂ hydrocarbon or acetonitrile,
wherein parts by volume are based on the volume of the antisolvent mixture AntiSolv-1, which is 100 parts by volume, and the sum of the components (anti-a) and (anti-b) is below or exactly 100 parts by volume.

An amount of the antisolvent mixture AntiSolv-1 at step (iv) is preferably in a range of 0.5 mL to 4.0 mL of antisolvent mixture AntiSolv-1 for 1.0 mL of cleavage solution CleaSolu-1 at step (iv), more preferably in a range of 1.0 mL to 4.0 mL for 1.0 mL of cleavage solution CleaSolu-1 at step (iv), very preferably in a range of 1.5 mL to 3.0 mL for 1.0 mL of cleavage solution CleaSolu-1 at step (iv), particularly in a range of 1.7 mL to 2.5 mL for 1.0 mL of cleavage solution CleaSolu-1 at step (iv) and more particularly in a range of 1.9 mL to 2.2 mL for 1.0 mL of cleavage solution CleaSolu-1 at step (iv).

The step (v) and thus the time period Time-(v) starts after essentially all or all of the antisolvent has been added and in case the temperature of the precipitation suspension PrecSusp-1 has been below 0 °C at step (iv), the precipitation suspension PrecSusp-1 is warmed and has reached a temperature of at least 0 °C. Preferably, the stirring at step (v) occurs under a chemically inert gas atmosphere, for example under nitrogen.

The time period Time-(v) is at least 7 h, i.e. irrespective of the results of the HPLC-UV chromatogram in regard to the relative area percentage ratio (B) to (A). The time period Time-(v) lasts longer than 7 h, i.e. the stirring is continued, until the relative area percentage (B) to (A) is in the stated range of the relative area percentage ratio (B) to (A). Preferably, the time period Time-(v) is at least 8 h, more preferably between 8 h and 72 h, very preferably between 9 h and 66 h, particularly between 10 h and 60 h, more particularly between 11 h and 56 h, very particularly between 12 h and 52 h, especially between 13 h and 50 h, more especially between 14 h and 49 h, very especially between 15 h and 48 h and most especially between 16 h and 28 h.

The Temp-(v) is preferably between 4 °C and 32 °C, more preferably between 6 °C and 31 °C, very preferably between 8 °C and 30 °C, particularly between 10 °C and 29 °C, more particularly between 12 °C and 28 °C, very particularly between 14 °C and 27 °C, especially between 16 °C and 26 °C, more especially between 18 °C and 25 °C and very especially between 20 °C and 24 °C.

Preferred is a method for manufacturing a precipitated mixture PrecMixt-1, wherein the time period Time-(v) is at least 8 h.

Preferred is a method for manufacturing a precipitated mixture PrecMixt-1, wherein the time period Time-(v) is between 8 h and 72 h.

Preferred is a method for manufacturing a precipitated mixture PrecMixt-1, wherein the temperature Temp-(v) is between 4 °C and 32 °C.

At step (v), the test sample, which is only a small part of the overall amount of the stirring precipitation suspension, is taken from the stirring precipitation suspension to determine and thus monitor the developing relative area percentage ratio (B) to (A) during the stirring. This allows to recognize the already achieved status and whether a continuation of stirring is indicated or whether the isolating of step (vi) is possible. Taking the test sample and the HPLC-UV chromatogram of the analytical HPLC sample prepared via the test sample is an in-process-control respectively an in-process-control. Accordingly, the method of manufacturing the precipitated mixture PrecMixt-1 might at the first conduction of a specific method of manufacturing the precipitated mixture PrecMixt-1 require two or more test samples respectively two or more analytical HPLC samples obtained from the two or more test samples, to depict the detailed kinetics of a specific method of manufacturing a precipitated mixture PrecMixt-1. It has surprisingly also been found that the prolonged stirring of the precipitated suspension at step (v) is benign, i.e. the relative area percentage ratio (B) to (A) develops relatively fast towards a tableau level and a peptide degradation remains not relevant for a relatively long time period. It is obvious that for the economic reason of a desirably short duration of a process step, the earlier part of the time period Time-(v) is of more interest, even if a certain longer stirring at the later part of the time period Time-(v) does not lead to a relevant peptide degradation. At least once a specific method of manufacturing a precipitated mixture PrecMixt-1 is again conducted after the first conduction, a taking of a test sample and the analytical HPLC sample prepared thereof is optional.

The taken test sample is worked-up to isolate the test sample precipitate. The work-up, which comprises filtrating to obtain a test sample precipitate, optionally including washing of the test sample precipitate, and drying of the test sample precipitate to obtain a dried test sample precipitate, reduces already the amount of trifluoroacetic acid. Filtering optionally includes a washing, preferably a washing with an antisolvent, more preferably with an antisolvent as described at step (iv), very preferably with an antisolvent which comprises a dialkyl ether, particularly with an antisolvent, which is a dialkyl ether with a total number of carbon atoms per molecule between 4 and 7, and more particularly with an antisolvent, which is diisopropyl ether. Preferably, the drying of the test sample precipitate is conducted under a vacuum, more preferably at a vacuum between 100 mbar (10000 Pa) to 0.5 mbar (50 Pa), very preferably at a vacuum between 50 mbar (5000 Pa) to 1.0 mbar (100 Pa), particularly at a vacuum between 25 mbar (2500 Pa) to 3 mbar (300 Pa) and more particularly at a vacuum between 6 mbar (600 Pa) to 4 mbar (400 Pa). Preferably, the drying of the test sample precipitate is conducted at a temperature between 0 °C and 35 °C, more preferably between 4 °C and 32 °C, very preferably between 6 °C and 31 °C, particularly between 10 °C and 29 °C, more particularly between 16 °C and 28 °C, very particularly between 20 °C and 27 °C, especially between 24 °C and 26 °C and more especially at 25 °C. Preferably, the drying of the test sample precipitate is conducted for a time period between 0.5 h and 24 h, more preferably between 1 h and 23 h, very preferably between 2 h and 22 h, particularly between 4 h and 21 h, more particularly between 6 h and 20 h and very particularly between 12 h and 18 h. Dissolving of the dried test sample precipitate in the solution for dissolving or diluting, which contains with around 50 vol.% acetonitrile a water-miscible organic solvent and also 1.0 M (NH₄)₃PO₄ with a pH of 7.8, allows an essentially non-acidic analytical HPLC sample. The essentially non-acidic analytical HPLC sample allows the determination of the relative area percentage ratio (B) to (A). It has surprisingly also been found that a preparation of the analytical HPLC sample with a buffer salt, which prevents an acidic pH value of the analytical HPLC sample, is decisive. This is surprising, since an applied reverse phase high performance liquid chromatography as shown in the experimental part, which uses two eluents containing both trifluoroacetic acid, does not lead to a disappearance of component (B) in the HPLC-UV chromatogram.

The HPLC-UV chromatogram is obtained from a high performance liquid chromatography with an UV detection, which allows a baseline of the peaks of component (A) and of component (B). The intensity of the UV absorption defines the area percentage of the respective peak. The high performance liquid chromatography is preferably a reverse phase high performance liquid chromatography, more preferably a reverse phase high performance liquid chromatography with a column containing a hydrophobically modified silica as the stationary phase, very preferably a reverse phase high performance liquid chromatography with a column containing C18-, C8-, C4- or phenyl-modified silica as the stationary phase, and particularly a reverse phase high performance liquid chromatography with a column containing C18-modified silica as the stationary phase. Preferably, the reverse phase high performance liquid chromatography uses an eluent A, which comprises more than 50 vol.% water based on the overall volume of the eluent A, and trifluoroacetic acid, and an eluent B, which comprises more than 50 vol.% acetonitrile based on the volume of the eluent B, and trifluoroacetic acid. More preferably, the concentration of trifluoroacetic acid in eluent A is between 0.01 vol.% and 0.10 vol.% based on the overall volume of the eluent A, and the concentration of trifluoroacetic acid in eluent B is between 0.01 vol.% and 0.1 vol.% trifluoroacetic acid based on the overall volume of the eluent B. Very preferably, the concentration of trifluoroacetic acid in eluent A is between 0.03 vol.% and 0.07 vol.% and the concentration of trifluoroacetic acid in eluent B is between 0.03 vol.% and 0.07 vol.%. Preferably, the reverse phase high performance liquid chromatography uses a gradient elution, which starts with a high content of eluent A and a low content of eluent B and with an increase of the amount of eluent B. The UV detection is preferably conducted at a wavelength of 220 nm. A column temperature of the reverse phase high performance liquid chromatography is preferably above room temperature, more preferably between 30 °C and 65 °C, more preferably between 40 °C and 60 °C, very preferably between 45 °C and 55 °C and particularly is 50 °C. Preferably, the HPLC-UV chromatogram is obtained from a reverse phase high performance liquid chromatography, which comprises an injection of 0.5 to 3 µL of the analytical HPLC sample into a column with C18-modified silica as a stationary phase, a column temperature of 50 °C, an UV detection at 220 nm, an eluent A prepared by adding 0.05 vol.% trifluoroacetic acid based on the overall volume of a mixture of 98 vol.% deionized water and 2 vol.% acetonitrile, an eluent B obtained by adding 0.05 vol.% trifluoroacetic acid to acetonitrile and a gradient elution of the two eluents A and B by a start with 85 vol.% eluent A and 15 vol.% eluent B and an increase of the amount of eluent B to 100 vol.%.

Preferred is a method for manufacturing a precipitated mixture PrecMixt-1, wherein the HPLC-UV chromatogram is obtained from a reverse phase high performance liquid chromatography, which comprises an injection of 0.5 to 3 µL of the analytical HPLC sample into a column with C18-modified silica as a stationary phase, a column temperature of 50 °C, an UV detection at 220 nm, an eluent A prepared by adding 0.05 vol.% trifluoroacetic acid based on the overall volume of a mixture of 98 vol.% deionized water and 2 vol.% acetonitrile, an eluent B obtained by adding 0.05 vol.% trifluoroacetic acid to acetonitrile and a gradient elution of the two eluents A and B by a start with 85 vol.% eluent A and 15 vol.% eluent B and an increase of the amount of eluent B to 100 vol.%.

The relative area percentage ratio (B) to (A) is preferably in the range of 0.075 to 0.015, more preferably in the range of 0.070 to 0.018, very preferably in the range of 0.065 to 0.019, particularly in the range of 0.060 to 0.020, more particularly in the range of 0.055 to 0.021 and very particularly in the range of 0.050 to 0.022.

Preferred is a method for manufacturing a precipitated mixture PrecMixt-1, wherein the HPLC-UV chromatogram with the area percentage of component (A) and the area percentage of component (B) has a relative area percentage ratio (B) to (A) in the range of 0.075 to 0.015.

Isolating the precipitation from the precipitation suspension PrecSusp-2 at step (vi) is typically conducted by filtering the precipitation suspension PrecSusp-2 to obtain a separated precipitation, by centrifuging the precipitation suspension PrecSusp-2 to obtain a separated precipitation or by letting rest the precipitation suspension PrecSusp-2 without stirring to obtain a separated precipitation. At filtering, the separated precipitation is the filter residue. At centrifuging, the separated precipitation is a concentrate. At letting rest, the separated precipitation is a sediment. The separated precipitation is optionally washed with an antisolvent. Washing with an antisolvent is typically conducted by re-suspending the separated precipitation and conducting again a filtering, a centrifuging or a letting rest. At letting rest, the separated concentrate is optionally washed with an antisolvent by re-suspending. Preferably, the antisolvent at the optional washing of the separated precipitation is an antisolvent as described at step (iv), more preferably an antisolvent, which comprises a dialkyl ether, very preferably with an antisolvent, which is a dialkyl ether with a total number of carbon atoms per molecule between 4 and 7, particularly with an antisolvent, which is diisopropyl ether. Preferably isolating at step (vi) is conducted by filtering or by centrifuging, more preferably by filtering followed by washing or by centrifuging followed by washing. Preferably, the isolating at step (vi) occurs under a chemically inert gas atmosphere, for example under nitrogen.

Isolating the precipitation from the precipitation suspension PrecSusp-2 comprises preferably drying of the separated precipitation at step (vi), which has optionally been washed. Preferably, the drying of the separated precipitation at step (vi), which has optionally been washed, is conducted under a vacuum, more preferably at a vacuum between 100 mbar (10000 Pa) to 0.5 mbar (50 Pa), very preferably at a vacuum between 50 mbar (5000 Pa) to 1.0 mbar (100 Pa), particularly at a vacuum between 25 mbar (2500 Pa) to 3 mbar (300 Pa) and more particularly at a vacuum between 6 mbar (600 Pa) to 4 mbar (400 Pa). Preferably, the drying of the separated precipitation at step (vi), which has optionally been washed, is conducted at a temperature between 0 °C and 35 °C, more preferably between 4 °C and 32 °C, very preferably between 6 °C and 31 °C, particularly between 10 °C and 29 °C, more particularly between 16 °C and 28 °C, very particularly between 20 °C and 27 °C, especially between 24 °C and 26 °C and more especially at 25 °C. Preferably, the drying of the separated precipitation at step (vi), which has optionally been washed, is conducted for a time period between 0.5 h and 48 h, more preferably between 1 h and 36 h, very preferably between 2 h and 24 h, particularly between 4 h and 21 h, more particularly between 6 h and 20 h and very particularly between 12 h and 18 h.

Preferred is a method for manufacturing a precipitated mixture PrecMixt-1, wherein at step (vi) isolating is conducted by filtering or by centrifuging.

Preferred is a method for manufacturing a precipitated mixture PrecMixt-1, wherein at step (vi) the precipitation is dried in vacuum.

A higher purity according to the HPLC-UV chromatogram of the precipitated mixture PrecMixt-1 means a lower amount of components different to component (A), for example peptidic components different to component (A) that afterwards have to be removed at a purification of the precipitated mixture PrecMixt-1 to obtain a purified composition PuriComp-1, which comprises liraglutide or a salt thereof. Preferably, the sum of the area percentage of component (A) and the area percentage of component (B) is at least 65 % based on the overall area percentages of the HPLC-UV chromatogram under exclusion of area percentages caused by solvent peaks, more preferably, in the range 66 % to 95 %, very preferably in the range of 67 % to 93 %, particularly in the range of 68 % to 90 %, more particularly in the range of 70 % to 85 %, very particularly in the range of 72 % to 82 % and especially in the range of 73 % to 81 %. Preferably, the area percentage of component (A) is at least 64 % based on the overall area percentages of the HPLC-UV chromatogram under exclusion of area percentages caused by solvent peaks, more preferably in the range of 65 % to 94 %, very preferably in the range of 66 % to 92 %, particularly in the range of 67 % to 89 %, more particularly in the range of 69 % to 84 %, very particularly in the range of 71 % to 81 % and especially in the range of 72 % to 80 %.

Preferred is a method for manufacturing a precipitated mixture PrecMixt-1, wherein the sum of the area percentage of component (A) and the area percentage of component (B) is at least 65 % based on the overall area percentages of the HPLC-UV chromatogram under exclusion of area percentages caused by solvent peaks.

Preferred is a method for manufacturing a precipitated mixture PrecMixt-1, wherein the area percentage of component (A) is at least 64 % based on the overall area percentages of the HPLC-UV chromatogram under exclusion of area percentages caused by solvent peaks.

A water content of the precipitated mixture PrecMixt-1 is for example determined according to the Karl Fischer method, preferably with volumetric tritration. The water content of the precipitated mixture PrecMixt-1 is preferably less than 20 wt.% based on the weight of the precipitated mixture PrecMixt-1, more preferably less than 10 wt.%, very preferably less than 5 wt.%, particularly between 0.1 wt.% and 5 wt.%, more particularly between 0.2 wt.% and 4 wt.%, very particularly between 0.5 wt.% and 3.5 wt.%, especially between 0.8 wt.% and 3 wt.%, more especially between 1.0 wt.% and 2 wt.%.

Preferred is a method for manufacturing a precipitated mixture PrecMixt-1, wherein the precipitated mixture PrecMixt-1 has a water content of less than 20 wt.% based on the weight of the precipitated mixture PrecMixt-1.

A trifluoroacetic acid content of the precipitated mixture PrecMixt-1 is for example determined by HPLC with a UV detector at 210 nm by injecting an aqueous solution, which is obtained by adding precipitated mixture PrecMixt-1 into water, 0.01 M aqueous hydrochloric acid or 0.1 M hydrochloric acid at a concentration of 5 or 10 mg of precipitated mixture PrecMixt-1 in 1.0 mL of water, of 0.01 M aqueous hydrochloric acid or of 0.1 M hydrochloric acid and mixing. Preferably, the trifluoroacetic acid content of the precipitated mixture PrecMixt-1 is determined by HPLC with a UV detector at 210 nm by injecting an aqueous solution, which is obtained by adding precipitated mixture PrecMixt-1 into water at a concentration of 10 mg of precipitated mixture PrecMixt-1 in 1.0 mL of water and mixing. The precipitated mixture PrecMixt-1 can contain trifluoroacetic acid itself, for example by entrapped residual triflouroacetic acid in the precipitated mixture, trifluoroacetate as a counterion of a protonated liraglutide salt or a protonated Tfa-liraglutide salt and trifluoroacetic acid, which originates from a potential hydrolysis of Tfa-liraglutide or a salt thereof in the acidic aqueous environment into liraglutide or a salt thereof and trifluoroacetic acid. A calculation as provided in C-7 of the experimental part reveals that in case of a mixture of 0.92 g of liraglutide and 0.08 g Tfa-liraglutide, the maximum trifluoroacetic acid weight resulting from a complete hydrolysis of Tfa-liraglutide is 0.00237 g. This represents 0.237 wt.%. Accordingly, the quantitative effect of the extend of hydrolysis of Tfa-liraglutide or a salt thereof as component (B) in the precipitated mixture is limited in view of a preferred minimum content of 6 wt.% of trifluoroacetic acid based on the weight of the precipitated mixture PrecMixt-1. Preferably, the precipitated mixture PrecMix-1 has a content of trifluoroacetic acid between 6 wt.% and 24 wt.% based on the overall weight of the precipitated mixture PrecMixt-1. More preferably, the content is between 7 wt.% and 21 wt.%, very preferably between 8 wt.% and 18 wt.%, particularly between 9 wt.% and 16 wt.% and more particularly between 10 wt.% and 14 wt.%.

Preferred is a method for manufacturing a precipitated mixture PrecMixt-1, wherein the precipitated mixture PrecMixt-1 has a content of trifluoroacetic acid between 6 wt.% and 24 wt.% based on the weight of the precipitated mixture PrecMixt-1.

The precipitated mixture PrecMixt-1 can contain residual amounts of the antisolvent added at step (iv) or in case of a washing with an antisolvent at step (vi), the antisolvent used at step (vi). The content is for example determined by gas chromatography. The stated content means the sum of the weight of the antisolvent added at step (iv) and in case of a washing with an antisolvent at step (vi), the weight of the antisolvent used at step (vi). Preferably, the content of diisopropyl ether of the precipitated mixture PrecMixt-1 is stated. Preferably, the precipitated mixture PrecMixt-1 has a content of the antisolvent added at step (iv) and in case of a washing with an antisolvent at step (vi), the antisolvent used at step (vi), between 0.2 wt.% and 1.0 wt.% based on the weight of the precipitated mixture PrecMixt-1. More preferably, the content is between 0.3 wt.% and 0.8 wt.% and very preferably between 0.4 wt.% and 0.6 wt.%.

Preferred is a method for manufacturing a precipitated mixture PrecMixt-1, wherein the precipitated mixture PrecMixt-1 has a content of the antisolvent added at step (iv) and in case of a washing with an antisolvent at step (vi), the antisolvent used at step (vi), between 0.2 wt.% and 1.0 wt.% based on the weight of the precipitated mixture PrecMixt-1.

Preferably, the precipitated mixture PrecMixt-1 is free of glycine. Preferably, the precipitated mixture PrecMixt-1 is free of piperidine. Preferably, the precipitated mixture PrecMixt-1 is free of tetrahydrofuran. More preferably, the precipitated mixture PrecMixt-1 is free of glycine and piperidine. More preferably, the precipitated mixture PrecMixt-1 is free of glycine and tetrahydrofuran. More preferably, the precipitated mixture PresMixt-1 is free of piperidine and tetrahydrofuran. Very preferably, the precipitated mixture PrecMixt-1 is free of glycine, piperidine and tetrahydrofuran.

The precipitated mixture PrecMixt-1 obtained at step (vi) can be further purified to an extend that a pharmaceutically acceptable, very high degree of purity is achieved. Accordingly in addition to steps (i) to (vi), one or more steps for a purification of the precipitated PrecMixt-1 are preferably conducted at the method for manufacturing of a precipitated mixture PrecMixt-1. The one or more further steps comprise at least one purifying by a preparative liquid chromatography. The preparative liquid chromatography differs from an analytical high performance liquid chromatography by collecting fractions from the elution and combining selected collected fractions. By the further steps, the method for manufacturing of a precipitated mixture PrecMixt-1 becomes a method for manufacturing of a precipitated mixture PrecMixt-1 and for manufacturing a purified composition PuriComp-1, which contains as component (A) liraglutide or a salt thereof. The purified composition PuriComp-1 differs from the precipitated mixture PrecMixt-1 by being purified by a preparative liquid chromatography and by possessing a higher area percentage of component (A) in its HPLC-UV chromatogram than the combined area percentages of components (A) and (B) in the HPLC-UV chromatogram of the precipitated mixture PrecMixt-1. Preferably, both HPLC-UV chromatograms are obtained by the same analytical high performance liquid chromatography. Preferably, the purified composition PuriComp-1 results in a HPLC UV-chromatogram with an area percentage of liraglutide or a salt thereof as component (A) of at least 90 %, more preferably in the range of 95 % to 100 %, very preferably in the range of 96 % to 99.99 %, particularly in the range of 97 % to 99.90 %, more particularly in the range of 98.0 % to 99.88 %, very particularly in the range of 98.5 % to 99.86 % and especially in the range of 99.0 % to 99.85 %. Preferably, the HPLC UV-chromatogram of the purified composition PuriComp-1 is obtained from a reverse phase high performance liquid chromatography, which comprises an injection of 0.5 to 3 µL of an analytical HPLC sample, which is obtained by dissolving a part of the purified composition PuriComp-1 at a concentration of 1 mg 11 mL in a mixture of water and acetonitrile, into a column with C18-modified silica as a stationary phase, a column temperature of 50 °C, an UV detection at 220 nm, an eluent A prepared by adding 0.05 vol.% trifluoroacetic acid based on the overall volume of a mixture of 98 vol.% deionized water and 2 vol.% acetonitrile, an eluent B obtained by adding 0.05 vol.% trifluoroacetic acid to acetonitrile and a gradient elution of the two eluents A and B by a start with 85 vol.% eluent A and 15 vol.% eluent B and an increase of the amount of eluent B to 100 vol.%. The purified composition PuriComp-1 is preferably in the form of a lyophilizate.

For example, the method for manufacturing a precipitated mixture PrecMixt-1 and for manufacturing a purified composition PuriComp-1, which contains liraglutide or a salt thereof, comprises the steps
(vii) dissolving the precipitated mixture PrecMixt-1 to obtain a solution for preparative liquid chromatography SoluChro-1;
(viii-a) purifying the solution for a preparative liquid chromatography SoluChro-1 by a first preparative liquid chromatography including collecting fractions and combining selected fractions,
(viii-b) optionally purifying by a second preparative liquid chromatography including collecting fractions and combining selected fractions,
(viii-c) optionally desalting of combined selected fractions from step (viii-a) or optionally step (viii-b);
(ix) lyophilizing to obtain the purified composition PuriComp-1.

At step (vii), the solution for preparative liquid chromatography SoluChro-1 is preferably an aqueous solution, more preferably an aqueous solution containing at least 50 vol.% of water based on the overall volume of the solution for preparative liquid chromatography SoluChro-1, very preferably at least 60 vol.% of water, particularly 65 vol.% to 98 vol.% of water and acetonitrile, more particularly 80 vol.% to 97 vol.% water and acetonitrile. Preferably, the dissolving of the precipitated mixture PrecMixt-1 occurs under control of the pH value of the forming solution for preparative liquid chromatography, more preferably by keeping the pH value in a range of 6.8 to 8.5, very preferably in a range of 6.9 to 8.2 and particularly in a range of 7.0 to 8.1. The pH value is kept for example by addition of a base, preferably an inorganic base, more preferably by addition of sodium hydroxide or ammonia. The solution for preparative liquid chromatography SoluChro-1 contains preferably a buffer salt, for example (NH₄)₃PO₄, (NH₄)HCO₃ or ammonium acetate. The buffer salt supports keeping of the pH value in the desired range. The solution for preparative liquid chromatography SoluChro-1 contains preferably a weight amount of precipitated PrecMixt-1 in a range of 2 to 50 g / L, more preferably in a range of 4 to 40 g / L, very preferably in a range of 10 to 30 g / L and particularly in a range of 15 to 25 g / L. The solution for preparative liquid chromatography SoluChro-1 is preferably filtered, for example filtered with a 0.2 µm filter.

At step (viii-a), the first preparative liquid chromatography is preferably a preparative high performance liquid chromatography, more preferably a preparative reverse phase high performance liquid chromatography, very preferably a preparative reverse phase high performance liquid chromatography with a column containing a hydrophobically modified silica as the stationary phase, particularly a preparative reverse phase high performance liquid chromatography with a column containing C18-, C8-, C4- or phenyl-modified silica as the stationary phase, more particularly a preparative reverse phase high performance liquid chromatography with a column containing C8-modified silica as the stationary phase. Preferably, the preparative reverse phase high performance liquid chromatography uses an eluent A, which comprises more than 50 vol.% water based on the overall volume of the eluent A, and a buffer salt, and an eluent B, which comprises more than 50 vol.% acetonitrile based on the volume of the eluent B, and a buffer salt. The buffer salt is preferably (NH₄)₃PO₄, (NH₄)HCO₃ or ammonium acetate, more preferably (NH₄)₃PO₄. The aqueous part of eluent A, i.e. prior to addition of an optional organic water-miscible solvent, for example acetonitrile, is preferably set to a pH value in a range of 6.8 to 8.5, more preferably in a range of 6.9 to 8.2, very preferably in a range of 7.0 to 8.1, particularly in a range of 7.1 to 8.0, more particularly in a range of 7.2 to 7.9 and very particularly in a range of 7.4 to 7.8. The aqueous part of eluent B, i.e. prior to addition of acetonitrile, is preferably set to a pH value in a range of 6.8 to 8.5, more preferably in a range of 6.9 to 8.2, very preferably in a range of 7.0 to 8.1, particularly in a range of 7.1 to 8.0, more particularly in a range of 7.2 to 7.9 and very particularly in a range of 7.4 to 7.8. Preferably, the first preparative reverse phase high performance liquid chromatography uses a gradient elution, which starts with a high content of eluent A and a low content of eluent B and with an increase of the amount of eluent B. The first preparative reverse phase high performance liquid chromatography, which uses a gradient elution, is for example conducted in the mode of a multicolumn countercurrent solvent gradient purification. The liquid eluting from the first preparative liquid chromatography at step is collected as fractions. A fraction, which contains liraglutide or a salt thereof in a high purity, is selected and combined with other fractions of similar or higher purity. The combined selected fractions are the main-cut of the first preparative liquid chromatography.

At optional step (viii-b), the main-cut of the first preparative liquid chromatography is purified by the second preparative liquid chromatography. The preferences for the first preparative liquid chromatography apply similarly to the second preparative liquid chromatography except for the buffer salt. The buffer salt of the second preparative liquid chromatography is preferably (NH₄)₃PO₄, (NH₄)HCO₃ or ammonium acetate, more preferably (NH₄)HCO₃ or ammonium acetate and very preferably (NH₄)HCO₃. Preferably, the stationary phase of the first preparative liquid chromatography and the stationary phase of the second preparative liquid chromatography are the same. The combined selected fractions of the second preparative liquid chromatography are the main-cut of the second preparative liquid chromatography.

At optional step (viii-c), the combined selected fractions respectively the main-cut of the first preparative liquid chromatography or in case optional step (viii-b) is conducted, the combined selected fractions respectively the main-cut of the second preparative liquid chromatography are desalted. Desalting is for example conducted by a third preparative reverse phase liquid chromatography with a column, which includes preferably loading of the respective main-cut, which is optionally reduced in its acetonitrile content, onto the column, washing with a mainly aqueous eluent with a low or no salt content and eluting liraglutide or a salt thereof relatively fast, i.e. in a short period, respectively in a comparatively high concentration of the relevant eluting liquid. Desalting is for example conducted by an ion exchange chromatography, which includes optionally that the respective main-cut is reduced in its acetonitrile content prior to loading onto the ion exchange chromatography itself. Desalting is for example conducted by a size exclusion chromatography with a column, which includes optionally that the respective main-cut is reduced in its acetonitrile content prior to loading onto the column. Desalting is for example conducted by an ultrafiltration, which includes optionally that the respective main-cut is reduced in its acetonitrile content. The respective main-cut can be reduced in its acetonitrile content by dilution, for example by dilution with water, or by vacuum evaporation of acetonitrile form the respective main-cut, for example by using a vacuum rotary evaporator.

At step (ix), the combined selected fractions respectively the main-cut of the first preparative liquid chromatography, in case optional step (viii-b) is conducted, the combined selected fractions respectively the main-cut of the second preparative liquid chromatography, or in case step (viii-c) is conducted, the desalted respective main-cut are lyophilized respectively freeze-dried. The purified composition PuriComp-1 is obtained in the form of a lyophilizate respectively in a freeze-dried form.

Preferred is a method for manufacturing a precipitated mixture PrecMixt-1 and for manufacturing a purified composition PuriComp-1 comprising liraglutide or a salt thereof, which comprises the steps
(vi) dissolving the precipitated mixture PrecMixt-1 to obtain a solution for preparative liquid chromatography SoluChro-1;
(vii-a) purifying the solution for preparative liquid chromatography SoluChro-1 by a first preparative liquid chromatography including collecting fractions and combining selected fractions,
(vii-b) optionally purifying by a second preparative liquid chromatography including collecting fractions and combining selected fractions,
(vii-c) optionally desalting of combined selected fractions from step (vii-a) or optionally step (vii-b);
(viii) lyophilizing to obtain the purified composition PuriComp-1 comprising liraglutide or a salt thereof.

The explanations and preferences described above for a method of manufacturing a precipitated mixture PrecMixt-1, including the explanations and preferences for individual technical features contained in the method of manufacturing a precipitated mixture PrecMixt-1, apply similarly to further embodiments of the invention.

Further disclosed is a precipitated mixture PrecMixt-1 (not forming a part of the invention as such), which contains the components
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof, and
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO: 4) or a salt thereof,

wherein an analytical HPLC sample of the precipitated mixture PrecMixt-1 results in a HPLC-UV chromatogram with an area percentage of component (A) and an area percentage of component (B) with a relative area percentage ratio (B) to (A) in the range of 0.080 to 0.010, when the analytical HPLC sample is prepared by
   - dissolving 1 mg of the precipitated mixture PrecMixt-1 in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample,
which has a water content of less than 20 wt.% based on the weight of the precipitated mixture PrecMixt-1.

Preferred is a precipitated mixture PrecMixt-1, wherein the sum of the area percentage of component (A) and the area percentage of component (B) is at least 65% based on the overall area percentages of the HPLC-UV chromatogram under exclusion of area percentages caused by solvent peaks.

Preferred is a precipitated mixture PrecMixt-1, wherein the precipitated mixture PrecMixt-1 has a content of trifluoroacetic acid between 6 wt.% and 24 wt.% based on the weight of the precipitated mixture PrecMixt-1.

Preferred is a precipitated mixture PrecMixt-1, wherein the precipitated mixture PrecMixt-1 has a content of diisopropyl ether between 0.2 wt.% and 1.0 wt.% based on the weight of the precipitated mixture PrecMixt-1.

Preferred is a precipitated mixture PrecMixt-1, wherein the HPLC-UV chromatogram is obtained from a reverse phase high performance liquid chromatography, which comprises an injection of 0.5 to 3 µL of the analytical HPLC sample into a column with C18-modified silica, a column temperature of 50 °C, an UV detection at 220 nm, an eluent A obtained by adding 0.05 vol.% trifluoroacetic acid to a mixture of 98 vol.% deionized water and 2 vol.% acetonitrile, an eluent B obtained by adding 0.05 vol.% trifluoroacetic acid to acetonitrile and a gradient elution by a start with 85 vol.% eluent A and 15 vol.% eluent B and an increase of the amount of eluent B to 100 vol.%.

A further embodiment of the invention is a method for manufacturing a purified liraglutide composition PuriComp-1, which contains liraglutide or a salt thereof, which comprises the steps
(I) dissolving a precipitated mixture PrecMixt-1, which contains the components
   (A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof, and
   (B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO: 4) or a salt thereof,
      wherein an analytical HPLC sample of the precipitated mixture PrecMixt-1 results in a HPLC-UV chromatogram with an area percentage of component (A) and an area percentage of component (B) with a relative area percentage ratio (B) to (A) in the range of 0.080 to 0.010, when the analytical HPLC sample is prepared by
         - dissolving 1 mg of the precipitated mixture PrecMixt-1 in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample,
      which has a water content of less than 20 wt.% based on the weight of the precipitated mixture PrecMixt-1, to obtain a solution for a preparative liquid chromatography SoluChro-1;
(II-a) purifying the solution for a preparative liquid chromatography SoluChro-1 from step (I) by a first preparative liquid chromatography including collecting fractions and combining selected fractions,
(II-b) optionally purifying by a second preparative liquid chromatography including collecting fractions and combining selected fractions,
(II-c) optionally desalting of combined selected fractions from step (II-a) or optionally from (II-b);
(III) lyophilizing to obtain the purified liraglutide composition PuriComp-1.

Further disclosed is a use of a precipitated mixture PrecMixt-1 (not forming part of the invention as such), which contains the components
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof, and
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO: 4) or a salt thereof,
   wherein an analytical HPLC sample of the precipitated mixture PrecMixt-1 results in a HPLC-UV chromatogram with an area percentage of component (A) and an area percentage of component (B) with a relative area percentage ratio (B) to (A) in the range of 0.080 to 0.010, when the analytical HPLC sample is prepared by
      - dissolving 1 mg of the precipitated mixture PrecMixt-1 in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample,
   which has a water content of less than 20 wt.% based on the weight of the precipitated mixture PrecMixt-1,
in the manufacturing of a purified liraglutide composition PuriComp-1, which contains liraglutide or a salt thereof.

Further disclosed is a method for analyzing a precipitated liraglutide mixture (not forming part of the invention as such), which contains liraglutide or a salt thereof as component (A)
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof,
   which comprises the steps
   (an-i) providing the precipitated liraglutide mixture, which is a precipitate obtained from a solid phase conjugated protected liraglutide precursor by cleavage with trifluoroacetic acid;
   (an-ii) dissolving the precipitated liraglutide mixture in a solution for dissolving or diluting, which is obtained by mixing a solvent basis and a buffer salt solution,
      the solvent basis comprises
         - water and a water-soluble organic solvent,
      the buffer salt solution comprises
         - water and a buffer salt
      the buffer salt solution is adjusted to a pH value between 7.0 and 8.5,
      and the content of the water-soluble organic solvent in the solution for dissolving or diluting is at least 15 vol.% based on 100 vol.% of the solution for dissolving or diluting, or
      which is dimethylformamide,
      to obtain an analytical HPLC sample;
   (an-iii) conducting a reverse phase high performance liquid chromatography including an injection of an appropriate amount of the analytical HPLC sample;
   (an-iv) determining a content of component (B)
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO: 4) or a salt thereof.

At step (an-i), the precipitated liraglutide mixture is preferably the precipitated mixture PrecMixt-1. The precipitated liraglutide mixture is more preferably the precipitated mixture PrecMixt-1, which is obtained by the method for manufacturing a precipitated mixture PrecMixt-1.

At step (an-ii), the precipitated liraglutide mixture is dissolved in a concentration of 0.5 mg to 2.0 mg per 1 mL of the solution for dissolving or diluting, more preferably in a concentration of 0.8 mg to 1.5 mg per 1 mL, very preferably in a concentration of 0.9 mg to 1.1 mg per 1 mL and more particularly in a concentration of 1 mg per 1 mL.

At step (an-ii), the water-soluble organic solvent is for example acetone, acetonitrile, gamma-butyrolactone, dimethyl sulfoxide, dimethylformamide, dioxane, ethanol, propanol, iso-propanol, methanol, N-methyl-2-pyrrolidone, sulfolane or tetrahydrofuran. Preferably, the water-soluble organic solvent is soluble in water in any ratio at 20 °C and 101,325 kPa. Preferably, the water-soluble solvent example acetone, acetonitrile, gamma-butyrolactone, dimethyl sulfoxide, dimethylformamide, dioxane, ethanol, propanol, iso-propanol, methanol, N-methyl-2-pyrrolidone, sulfolane or tetrahydrofuran, more preferably acetone, acetonitrile, gamma-butyrolactone, dimethyl sulfoxide, dimethylformamide, dioxane, N-methyl-2-pyrrolidone, sulfolane or tetrahydrofuran, very preferably acetone, acetonitrile, dimethyl sulfoxide or dimethylformamide, particularly acetonitrile, dimethyl sulfoxide or dimethylformamide, more particularly acetonitrile or dimethyl sulfoxide and very particularly acetonitrile.

At step (an-ii), the buffer salt is suitable to buffer at a pH value in the pH range between 7.0 and 8.5. More preferably, the buffer salt is an acetate, a carbonate, a formate, a phosphate or a proprionate. Very preferably, the buffer salt is ammonium acetate, ammonium carbonate, ammonium formate or ammonium phosphate. Particularly, the buffer salt is ammonium phosphate. The amount of the buffer salt in the solution for dissolving or diluting is chosen to keep a pH value of the analytical HPLC preferably in a range of 6.8 to 8.5, very preferably in a range of 6.9 to 8.2 and particularly in a range of 7.0 to 8.1.

At step (an-ii), the content of the water-soluble organic solvent in the solution for dissolving or diluting is preferably between 15 vol.% and 90 vol.% based on 100 vol.% of the solution for dissolving or diluting, more preferably between 20 vol.% and 85 vol.%, very preferably between 25 vol.% and 80 vol.%, particularly between 30 vol.% and 75 vol.%, more particularly between 35 vol.% and 70 vol.%, very particularly between 40 vol.% and 65 vol.%, especially between 45 vol.% and 50 vol.% and more especially between 48 vol.% and 52 vol.%. It is understood herein that if the volume of the buffer salt solution is relatively small in comparison to the volume of the solvent basis, then the volume content of the water-soluble organic solvent in the solvent basis is approximately the vaolume ontent of the water-soluble organic solvent in the solution for dissolving or diluting. Preferably, the volume of the buffer salt solution is between 0.5 vol.% and 8 vol.% based on 100 vol.% of solvent basis, more preferably between 0.6 vol.% and 5 vol.%, very preferably between 0.7 vol.% and 3 vol.%, particularly between 0.8 vol.% and 2 vol.%, more particularly between 0.9 vol.% and 1.5 vol.% and very particularly between 1.0 vol.% and 1.3 vol.%.

At step (an-iii), the reverse phase high performance liquid chromatography is preferably an analytical reverse phase high performance liquid chromatography. More preferably an analytical reverse phase high performance liquid chromatography with a column containing a hydrophobically modified silica as the stationary phase, very preferably an analytical reverse phase high performance liquid chromatography with a column containing C18-, C8-, C4- or phenyl-modified silica as the stationary phase, and particularly an analytical reverse phase high performance liquid chromatography with a column containing C18-modified silica as the stationary phase. The reverse phase high performance liquid chromatography uses preferably a gradient elution. For example, the reverse phase high performance liquid chromatography comprises an injection of 0.5 to 3 µL of the analytical HPLC sample into a column with C18-modified silica as a stationary phase, a column temperature of 50 °C, an UV detection at 220 nm, an eluent A prepared by adding 0.05 vol.% trifluoroacetic acid based on the overall volume of a mixture of 98 vol.% deionized water and 2 vol.% acetonitrile, an eluent B obtained by adding 0.05 vol.% trifluoroacetic acid to acetonitrile and a gradient elution of the two eluents A and B by a start with 85 vol.% eluent A and 15 vol.% eluent B and an increase of the amount of eluent B to 100 vol.%.

At step (an-vi), the content of component (B) is preferably determined as the area percentage of the respective peak of a HPLC-UV chromatogram, more preferably the area percentage of the respective peak of the HPLC-UV chromatogram at a detection wavelength between 200 nm and 290 nm, very preferably between 205 nm and 280 nm, particularly between 210 nm and 260 nm and more particularly at 220 nm.

### Brief description of the Figures

Fig. 1 depicts an extract of the LC-MS chromatogram from D-2-3. The peaks representing liraglutide and Tfa-liraglutide are marked.
Fig. 2 depicts the MS chromatogram of the peak marked with liraglutide at the LC-MS chromatogram from Fig. 1.
Fig. 3 depicts the MS chromatogram of the peak marked with Tfa-liraglutide at the LC-MS chromatogram from Fig. 1.
Fig. 4 depicts a complete HPLC-UV chromatogram from D-4-5-01. The peaks representing liraglutide and Tfa-liraglutide are marked and their area percentages are stated.
Fig. 5 depicts an extract of the HPLC-UV chromatogram from D-4-5-01 of Fig. 4 between 4 and 28 minutes.
Fig. 6 depicts an extract of the HPLC-UV chromatogram from D-4-5-01 of Fig. 4 between 14 and 20.5 minutes.
Fig. 7 depicts a complete HPLC-UV chromatogram from D-4-5-02. The peaks representing liraglutide and Tfa-liraglutide are marked and their area percentages are stated.
Fig. 8 depicts an extract of the HPLC-UV chromatogram from D-4-5-02 of Fig. 7 between 4 and 28 minutes.
Fig. 9 depicts an extract of the HPLC-UV chromatogram from D-4-5-02 of Fig. 7 between 14 and 20.5 minutes.
Fig. 10 depicts an extract of the HPLC-UV chromatogram from D-3-2-03 between 4 and 28 minutes.
Fig. 11 depicts an extract of the HPLC-UV chromatogram from D-3-2-03 of Fig. 10 between 14 and 20.5 minutes.
Fig. 12 depicts an extract of the HPLC-UV chromatogram from D-3-2-09 between 4 and 28 minutes.
Fig. 13 depicts an extract of the HPLC-UV chromatogram from D-3-2-09 of Fig. 12 between 14 and 20.5 minutes.
Fig. 14 depicts an extract of the HPLC-UV chromatogram from D-3-2-11 between 4 and 28 minutes.
Fig. 15 depicts an extract of the HPLC-UV chromatogram from D-3-2-11 of Fig. 14 between 14 and 20.5 minutes.
Fig. 16 depicts an extract of the HPLC-UV chromatogram from D-4-5-03 between 4 and 28 minutes.
Fig. 17 depicts an extract of the HPLC-UV chromatogram from D-4-5-03 of Fig. 16 between 14 and 20.5 minutes.
Fig. 18 depicts a complete HPLC-UV chromatogram from D-4-5-07. The peaks representing DMSO and liraglutide are marked and the area percentage of liraglutide is stated.
Fig. 19 depicts an extract of the HPLC-UV chromatogram from D-4-5-07 of Fig. 18 between 4 and 28 minutes. The peaks representing liraglutide and Tfa-liraglutide are marked and the area percentages are stated.
Fig. 20 depicts an extract of the HPLC-UV chromatogram from D-4-5-07 of Fig. 18 between 14 and 20.5 minutes. The peaks representing liraglutide and Tfa-liraglutide are marked and the area percentages are stated.
Fig. 21 depicts a complete HPLC-UV chromatogram from D-4-5-08. The peaks representing DMSO, liraglutide and Tfa-liraglutide are marked and the area percentages of liraglutide and Tfa-liraglutide are stated.
Fig. 22 depicts an extract of the HPLC-UV chromatogram from D-4-5-08 of Fig. 21 between 4 and 28 minutes.
Fig. 23 depicts an extract of the HPLC-UV chromatogram from D-4-5-08 of Fig. 21 between 14 and 20.5 minutes.
Fig. 24 depicts an extract of the HPLC-UV chromatogram from D-4-5-10 between 4 and 28 minutes.
Fig. 25 depicts an extract of the HPLC-UV chromatogram from D-4-5-10 of Fig. 24 between 14 and 20.5 minutes.
Fig. 26 depicts an extract of the HPLC-UV chromatogram from D-4-5-11 between 4 and 28 minutes.
Fig. 27 depicts an extract of the HPLC-UV chromatogram from D-4-5-11 of Fig. 26 between 14 and 20.5 minutes.
Fig. 28 depicts an extract of the HPLC-UV chromatogram from D-2-4-01 between 4 and 28 minutes.
Fig. 29 depicts an extract of the HPLC-UV chromatogram from D-2-4-01 of Fig. 28 between 14 and 20.5 minutes.
Fig. 30 depicts an extract of the HPLC-UV chromatogram from D-2-5-01 between 4 and 28 minutes.
Fig. 31 depicts an extract of the HPLC-UV chromatogram from D-2-5-01 of Fig. 30 between 14 and 20.5 minutes.

### Sequence Listing

Liraglutide plain peptide strand
   HAEGTFTSDVSSYLEGQAAKEFIAWLVRGRG (SEQ ID NO: 1)
N-terminally trifluoroacetylated liraglutide precursor: 
Tfa-His-Ala-Glu-Gly-Thr-Phe-Thr-Ser-Asp-Val-Ser-Ser-Tyr-Leu-Glu-Gly-Gln-Ala-
Ala-Lys(gamma-Glu-OH)-Glu-Phe-Ile-Ala-Trp-Leu-Val-Arg-Gly-Arg-Gly-OH
(SEQ ID NO: 2),
Liraglutide: 
His-Ala-Glu-Gly-Thr-Phe-Thr-Ser-Asp-Val-Ser-Ser-Tyr-Leu-Glu-Gly-Gln-Ala-Ala-
Lys(palmitoyl-gamma-Glu)-Glu-Phe-Ile-Ala-Trp-Leu-Val-Arg-Gly-Arg-Gly
(SEQ ID NO: 3)
N-terminally trifluoroacetylated liraglutide:

CF3CO-His-Ala-Glu-Gly-Thr-Phe-Thr-Ser-Asp-Val-Ser-Ser-Tyr-Leu-Glu-Gly-Gln-
Ala-Ala-Lys(palmitoyl-gamma-Glu)-Glu-Phe-Ile-Ala-Trp-Leu-Val-Arg-Gly-Arg-Gly
(SEQ ID NO: 4)

### Examples

The following examples illustrate further the invention without limiting it.
A) abbreviations
   - %: if not stated differently, a percentage value refers to weight percent
   - ACN: acetonitrile
   - Boc: tert-butyloxycarbonyl
   - DEPBT: 3-(diethoxyphosphoryloxy)-3H-benzo[d][1,2,3]triazin-4-one
   - DIC: diisopropylcarbodiimide
   - DIPEA: diisopropylethylamine
   - DMF: N,N-dimethylformamide
   - DMSO: dimethyl sulfoxide
   - EDT: ethane-1,2-dithiol
   - Fmoc: 9-fluorenylmethyloxycarbonyl
   - HPLC: high performance liquid chromatography
   - int.%: signal intensity percents
   - IPE: diisopropyl ether
   - Mpe: 3-methylpent-3-yl
   - MS: mass spectrometry
   - NMP: N-methylpyrrolidone
   - OxymaPure^{™}: cyano-hydroxyimino-acetic acid ethyl ester
   - Pbf: 2,2,4,6,7-pentamethyl-2,3-dihydrobenzfuran-5-sulfonyl
   - Psi(Me,Me): pro 1,1-dimethylmethdiyl, which is covalently linked to the beta oxygen atom of serine or threonine and covalently linked to the alpha-nitrogen of serine or threonine (pseudoproline)
   - TBTU: (benzotriazolyl)tetramethyluronium tetraflouroborate
   - tBu: tert-butyl
   - Tfa: trifluoroacetyl
   - TFA: trifluoroacetic acid
   - Trt: trityl
   - vol.%: volume percents
   - vol.-pph: additional volume parts based on hundred parts of previous volume
B) materials
   The employed materials are commercially available ones (e.g. from Bachem AG) unless a reference to a literature procedure is provided.
C) methods
   C-1: analytical HPLC sample

A solution of the material to be analyzed is needed for a conduction of an analytical HPLC method. The solution of the material to be analyzed allows the injection into a HPLC system. The targeted concentration of the material to be analyzed in the solution is 1 mg of material to be analyzed in 1 mL of the solution of the material to be analyzed. A material to be analyzed in a solid form is dissolved to obtain a solution of the material to be analyzed. If a material to be analyzed is already present in a dissolved form, a respective dilution towards the targeted concentration of the material to be analyzed of 1 mg in 1 mL is conducted. The solution of the material to be analyzed is prepared by combining the material to be analyzed and a solution for dissolving or diluting. The solution for dissolving or diluting contains one or more solvents. Optionally, a content of a buffer salt is desirable in the solution of the material to be analyzed. Accordingly, the solution for dissolving or diluting contains optionally the buffer salt or the buffer salt is added to the combination of the material to be analyzed and the solution for dissolving or diluting. Optionally, a certain pH value is desirable in the solution of the material to be analyzed. Accordingly, a base or an acid is added to the solution for dissolving or diluting or a base or an acid is added to the combination of the material to be analyzed and the solution for dissolving or diluting. Accordingly, a solution of a material to be analyzed contains the material to be analyzed in a targeted concentration of 1 mg in 1 mL, one or more solvents, optionally a buffer salt and optionally a base or an acid respectively the cations of the base or the anions of the acid. There might be situations, where a suspension of the material to be analyzed is obtained instead of an intended solution of the material to be analyzed. For example, a component of the material to be analyzed does not completely dissolve in the solution for dissolving or diluting. For example, a precipitation forms in an initial solution of the material to be analyzed after a certain time. In case of a suspension of the material to be analyzed, a treatment with ultrasonic is an option for trying to obtain a solution of the material to be analyzed. In case of a suspension of the material to be analyzed, a removal of the precipitate, for example by filtration with a 0.2 µm syringe filter, is an option to obtain a solution of the material to be analyzed. In case of a filtration, awareness that and what might have been removed is intrinsic. The solution of the material to be analyzed, is herein called an analytical HPLC sample.

Typically, 3.0 mL of a solution for dissolving or diluting are prepared and optionally, a pH-value of the solution for dissolving or diluting is measured. For dissolving a material to be analyzed, which is in solid form, 1.0 mg of the material to be analyzed is put into a HPLC vial and 1.0 mL of the solution for dissolving or diluting is added. For an acceleration of a dissolution, the HPLC vial is put into an ultrasonic bath for a short time. Optionally, a pH value of the analytical HPLC sample is measured.

### C-2: solutions for dissolving or diluting

At a solution for dissolving or diluting of the material to be analyzed, a solvent basis is defined by one solvent in case of one solvent or by two or more solvents in case of two or more solvents. The solvent basis of the solution for dissolving or diluting is set as 100 volume parts. In case a solution for dissolving or diluting contains two or more solvents, then the respective volume parts of the two or more solvents are mixed. A volume percentage of one of the two or more solvents is stated based on its mixed respective volume parts in relation to the volume parts of the solvent basis. In case of a desired content of a buffer salt in the solution for dissolving or diluting, the addition of the buffer salt is typically conducted by an addition of a relatively concentrated aqueous buffer salt solution to the solvent basis. Accordingly, the solution for dissolving or diluting contains a volume part of a solvent, i.e. water, originating from the relatively concentrated aqueous buffer salt solution in addition to the solvent basis. In view of the relatively small change, the solvent composition of the solution for dissolving or diluting is still stated based on the solvent basis if not reported differently. As an example, 3.0 mL of a solution for dissolving or diluting stated as 5 vol.% ACN and 1 vol.-pph 1 M (NH₄)₃PO₄ (pH 7.8) is prepared by mixing 2850 µL deionized water and 150 µL ACN and addition of 30 µL aqueous 1.0 M (NH₄)₃PO₄ solution adjusted to pH 7.8. The same principle of stating the solvent basis is applied in case of an addition of a base or an acid to the solvent basis or to a combination of the solvent basis and a relatively concentrated aqueous buffer salt solution.

A parent buffer salt solution I of an aqueous 1.7 M (NH₄)₃PO₄ solution with pH 7.8 is prepared by mixing 2.51 mL of an aqueous 25% NH₃ solution with 1.21 mL of an aqueous 85% H₃PO₄ solution and filling up with deionized water to 10 mL followed by pH adjustment to pH 7.8 by the aqueous 85% H₃PO₄ solution or the aqueous 25% NH₃ solution.

Buffer salt solution A-buff is prepared by 95 mL deionized water plus 3.8 mL ACN plus 1.06 mL of the parent buffer salt solution I (1.7 M (NH₄)₃PO₄ adjusted to pH 7.8). Buffer salt solution A-Buff is stated as 4 vol.% ACN and 1.07 vol.-pph 1.7 M (NH₄)₃PO₄ (pH 7.8). The amount of phosphoric acid anions is 0.0182 mmol / mL of solvent basis obtained by mixing 95 mL deionized water and 3.8 mL ACN.

Buffer salt solution B-buff is prepared by 33 mL deionized water plus 67 mL ACN plus 0.35 mL of the parent buffer solution I (1.7 M (NH₄)₃PO₄ adjusted to pH 7.8). Buffer salt solution B-Buff is stated as 67 vol.% ACN and 0.35 vol.-pph 1.7 M (NH₄)₃PO₄ pH 7.8. The amount of phosphoric acid anions is 0.00595 mmol / mL of solvent basis obtained by mixing 33 mL deionized water and 67 mL ACN.

Some solutions for dissolving or diluting are prepared by mixing buffer salt solution A-buff and buffer salt solution B-buff in different ratios as described in table C-2-1. For comparison, a solution for dissolving or diluting from deionized water plus ACN without a buffer salt is prepared.

**Table C-2-1**

| solution for dissolving or diluting No. | mixing ratio [vol.%] | | calculated content ACN in solvent basis of solution for dissolving or diluting [vol.%] | calculated content in solution for dissolving or diluting of 1.7 M (NH₄)₃PO₄ pH 7.8 [vol.-pph] |
|---|---|---|---|---|
| | A-buff | B-buff | | |
| C-2-1-01 | 80 | 20 | 17 | 0.93 |
| C-2-1-02 | 60 | 40 | 29 | 0.78 |
| C-2-1-03 | 50 | 50 | 35 | 0.71 |
| C-2-1-04 | 40 | 60 | 42 | 0.64 |
| C-2-1-05 | 20 | 80 | 54 | 0.49 |
| | | | | |
| C-2-1-06 | 50 vol.% ACN and 50 vol.% H₂O | | 50 | - |

A parent buffer solution II of an aqueous 1.0 M (NH₄)₃PO₄ solution with pH 7.8 is prepared by mixing 3.75 mL aqueous 25% NH₃ solution with 1.65 mL aqueous 85% H₃PO₄ solution and filling up with deionized water to 25 mL, followed by pH adjustment to pH 7.8 by the aqueous 85% H₃PO₄ solution or the aqueous 25% NH₃ solution.

Several series of solutions for dissolving or diluting are prepared via addition of parent buffer solution II, i.e. aqueous 1.0 M (NH₄)₃PO₄ solution with pH 7.8, to a solvent basis with deionized water and ACN. The solutions for dissolving or diluting are described in table C-2-1. For example, solution for dissolving or diluting No. C-2-2-02 is prepared by mixing 2.85 mL deionized water plus 0.15 mL ACN plus 15 µL of the parent buffer solution II (1.0 M (NH₄)₃PO₄ adjusted to pH 7.8). The solution for dissolving or diluting No. C-2-2-02 is stated as 5 vol.% ACN and 0.5 vol.-pph 1.0 M (NH₄)₃PO₄ pH 7.8. For example, solution for dissolving or diluting No. C-2-2-09 is prepared by mixing 1.5 mL deionized water plus 1.5 mL ACN plus 30 µL of the parent buffer solution II (1.0 M (NH₄)₃PO₄ adjusted to pH 7.8). The solution for dissolving or diluting No. C-2-2-09 is stated as 50 vol.% ACN and 1.0 vol.-pph 1.0 M (NH₄)₃PO₄ pH 7.8. For example, solution for dissolving or diluting No. C-2-2-19 is prepared by mixing 1.5 mL deionized water plus 1.5 mL ACN plus 300 µL of the parent buffer solution II (1.0 M (NH₄)₃PO₄ adjusted to pH 7.8). The solution for dissolving or diluting No. C-2-2-19 is stated as 50 vol.% ACN and 10.0 vol.-pph 1.0 M (NH₄)₃PO₄ pH 7.8.

**Table C-2-2**

| solution for dissolving or diluting No. ^{a)} | H₂O in solvent basis of solution for dissolving or diluting | ACN in solvent basis of solution for dissolving or diluting | content of 1.0 M (NH₄)₃PO₄ pH 7.8 in solution for dissolving or diluting [vol.-pph] | pH value ^{b), c)} of solution for dissolving or diluting |
|---|---|---|---|---|
| C-2-2-01 | 95 vol.% H₂O | 5 vol.% ACN | - | 7.3 (7.27) |
| C-2-2-02 | 95 vol.% H₂O | 5 vol.% ACN | 0.5 | 7.8 (7.75) |
| C-2-2-03 | 95 vol.% H₂O | 5 vol.% ACN | 1.0 | 7.9 (7.86) |
| C-2-2-04 | 95 vol.% H₂O | 5 vol.% ACN | 2.0 | 7.9 (7.90) |
| C-2-2-05 | 95 vol.% H₂O | 5 vol.% ACN | 5.0 | 7.9 (7.91) |
| | | | | |
| C-2-2-06 | 95 vol.% H₂O | 5 vol.% ACN | 1.0 | 7.9 (7.86) |
| C-2-2-07 | 90 vol.% H₂O | 10 vol.% ACN | 1.0 | 7.9 (7.91) |
| C-2-2-08 | 75 vol.% H₂O | 25 vol.% ACN | 1.0 | 8.0 (8.02) |
| C-2-2-09 | 50 vol.% H₂O | 50 vol.% ACN | 1.0 | 8.1 (8.10) |
| C-2-2-10 | 40 vol.% H₂O | 60 vol.% ACN | 1.0 | 8.1 (8.10) |
| | | | | |
| C-2-2-11 | 50 vol.% H₂O | 50 vol.% ACN | 1.0 | 8.1 (8.05) |
| C-2-2-12 | 40 vol.% H₂O | 60 vol.% ACN | 1.0 | 8.1 (8.05) |
| C-2-2-13 | 30 vol.% H₂O | 70 vol.% ACN | 1.0 | 8.1 (8.05) |
| C-2-2-14 | 20 vol.% H₂O | 80 vol.% ACN | 1.0 ^{d)} | 8.0 (7.97) |
| | | | | |
| C-2-2-15 | 50 vol.% H₂O | 50 vol.% ACN | 0.5 | 8.0 (8.06) |
| C-2-2-16 | 50 vol.% H₂O | 50 vol.% ACN | 1.0 | 8.1 (8.08) |
| C-2-2-17 | 50 vol.% H₂O | 50 vol.% ACN | 2.0 | 8.1 (8.08) |
| C-2-2-18 | 50 vol.% H₂O | 50 vol.% ACN | 5.0 | 8.1 (8.08) |
| C-2-2-19 | 50 vol.% H₂O | 50 vol.% ACN | 10.0 | 8.1 (8.08) |

| | | | | |
|---|---|---|---|---|
| Footnotes: a) solutions for dissolving or diluting of a jointly prepared series are grouped in table C-2-2-1, e.g. series C-2-2-01 to C-2-2-05 b) only tentative pH value in view of a measurement by a pH electrode in an aqueous mixture with a high content of organic solvent c) values in brackets without rounding towards one decimal provided in view of separately prepared solutions for dissolving or diluting with the same recipe, e.g. C-2-2-03 / C-2-2-06 or C-2-2-09 / C-2-2-11 / C-2-2-16 d) addition of the parent buffer solution II to the solvent basis leads to a cloudiness | | | | |

A further series of solutions for dissolving or diluting are prepared. The solvent basis of the solutions for dissolving or diluting are with deionized water or without deionized water. The solutions for dissolving or diluting are with an addition of parent buffer solution II, i.e. aqueous 1.0 M (NH₄)₃PO₄ solution with pH 7.8, to the solvent basis or without an addition. The solutions for dissolving or diluting are described in table C-2-3.

**Table C-2-3**

| solution for dissolving or diluting No. | H₂O in solvent basis of solution for dissolving or diluting | organic solvent in solvent basis of solution for dissolving or diluting | content of 1.0 M (NH₄)₃PO₄ pH 7.8 in solution for dissolving or diluting [vol.-pph] | pH value ^{a), b)} of solution for dissolving or diluting |
|---|---|---|---|---|
| C-2-3-01 | 50 vol.% H₂O | 50 vol.% ACN | - | 7.2 (7.20) |
| C-2-3-02 | 50 vol.% H₂O | 50 vol.% ACN | 1.0 | 8.1 (8.07) |
| C-2-3-03 | - | 100 vol.% DMF | - | - |
| C-2-3-04 | 50 vol.% H₂O | 50 vol.% DMF | - | 7.5 (7.54) |
| C-2-3-05 | 50 vol.% H₂O | 50 vol.% DMF | 1.0 | 8.8 (8.75) |
| C-2-3-06 | - | 100 vol. % DMSO | - | - |
| C-2-3-07 | 50 vol.% H₂O | 50 vol.% DMSO | - | 7.8 (7.77) |
| C-2-3-08 | 50 vol.% H₂O | 50 vol.% DMSO | 1.0 | 9.2 (9.18) |
| C-2-3-09 | - | 100 vol. % acetone | - | - |
| C-2-3-10 | 50 vol.% H₂O | 50 vol.% acetone | - | 7.7 (7.70) |
| C-2-3-11 | 50 vol.% H₂O | 50 vol.% acetone | 1.0 | 8.3 (8.32) |

| | | | | |
|---|---|---|---|---|
| Footnotes: a) only tentative pH value in view of a measurement by a pH electrode in an aqueous mixture with a high content of organic solvent b) values in brackets without rounding towards one decimal provided in view of separately prepared solutions for dissolving or diluting with the same recipe, e.g. C-2-3-02 in view of C-2-2-09 / C-2-2-11 / C-2-2-16 | | | | |

### C-3: analytical HPLC method

An analytical HPLC is conducted on a Dionex UltiMate 3000 Rapid Separation LC system from Thermo Scientific Inc. equipped with an Acquity UPLC BEH C18 column (130 Angstrom, 1.7 µm, 150 x 2.1 mm) from Waters Corporation. Eluent A is 0.05 vol.% of TFA in a mixture of deionized water: ACN = 98 : 2 (vol.% : vol.%) and eluent B is 0.05% vol.% of TFA in ACN. The applied gradient is depicted in table C-3-1. The flow rate is 0.4 mL / min and the column temperature is 50 °C. Detection occurs via ultraviolet irradiation at 220 nm. Injection volume of the analytical HPLC sample, i.e. the solution of the material to be analyzed, is between 0.5 and 3.0 µL. A HPLC-UV chromatogram is obtained.

**Table C-3-1**

| time [min] | eluent A [% volume] | eluent B [% volume] |
|---|---|---|
| 0.0 | 85.0 | 15.0 |
| 30.00 | 0.0 | 100.0 |
| 30.01 | 85.0 | 15.0 |
| 35.01 | 85.0 | 15.0 |

The peak at retention time 16.42 min and its area percentage, which is caused by the UV absorption, is counted as liraglutide respectively H-His¹-liraglutide.

The peak at retention time 17.30 min and its area percentage, which is cause by the UV absorption, is counted as Tfa-liraglutide respectively CF₃CO-His¹-liraglutide. An early eluting peak, for example from a solvent in the solution for dissolving or diluting, is not integrated for a calculation of area percentages.

### C-4: identification of Tfa-liraglutide

A liquid chromatography mass spectrum is conducted to identify Tfa-liraglutide. 1 mg of the final precipitate from example D-2-3 is dissolved in 1 mL of the solution for dissolving or diluting C-2-2-11 (50% ACN : 50 % H₂O; 1.0 vol.-pph 1.0 M (NH₄)₃PO₄ pH 7.8). 1 µL of the obtained solution is injected in a LC-MS system equipped with a Waters Acquity BEH C18 50x2.1 mm column from Waters Corporation. The flow rate is 0.4 mL / min and the column temperature is 50 °C. Eluent A is 0.05 vol.% of TFA in a mixture of deionized water: ACN = 99 : 1 (vol.% : vol.%) and eluent B is 0.05 vol.% of TFA in ACN. The applied gradient starts from 15 vol.% eluent B towards 30 vol. % and then continues to 100 vol.%.

An extract of the obtained LC-MS chromatogram is depicted at Fig. 1.

In the obtained LC-MS chromatogram, the peak at retention time 17.2 min is identified by its mass spectrum, which is depicted at Fig. 2, to represent liraglutide (Mw 3751.25 g/mol).

m/z: 751.1987 ([liraglutide+5H]⁵⁺), 938.7462 ([liraglutide+4H]⁴⁺), 1251.3259 ([liraglutide+3H]³⁺), 1876.4849 ([liraglutide+2H]²⁺).

In the obtained LC-MS chromatogram, the peak at retention time 18.1 min is identified by its mass spectrum, which is depicted at Fig. 3, to represent Tfa-liraglutide (Mw 3847.26 g/mol).

m/z: 770.3953 ([Tfa-liraglutide+5H]⁵⁺), 962.7415 ([Tfa-liraglutide+5H]⁵⁺), 1283.3194 ([Tfa-liraglutide+3H]³⁺), 1924.4745 ([Tfa-liraglutide+2H]²⁺).

### C-5: pH measurement

A pH meter from Metrohm Inc. is used for measurement of pH values. Its pH electrode is calibrated prior to its use with buffer calibration solutions of pH 7.01 and pH 10.00 from Hamilton Inc. It is noted that a measurement by a pH electrode in an aqueous solution containing an organic solvent provides only a tentative pH value. Despite of this, tentative pH values are still measured herein to indicate tendencies. For the same reason, tentative pH values are partly provided with three digits and without a rounding towards one decimal.

### C-6: water content

A water content is determined according to a Karl Fischer method with volumetric titration. The result is stated as a weight percentage, i.e. parts by weight of water in 100 parts by weight of material measured.

### C-7: TFA content

A TFA content is determined by HPLC with a UV detector at 210 nm. The material to be measured is dissolved at a concentration of 5 or 10 mg / mL in water or - if incompletely dissolved in water alone - in 0.01 M or 0.1 M aqueous hydrochloric acid. 20 µL of the obtained solution is injected onto a column, for example Spherisorb ODS2 (5 µm, 80 Angstrom, 4.6 x 250 mm) from Waters Corporation, at a column temperature of 25 °C and isocratic elution with a suitable eluent, for example a mixture of water, methanol, aqueous ammonia and phosphoric acid. A quantification occurs via a calibration by aqueous potassium trifluoroacetate solutions of known concentrations and by a respective fitting of the response curves of the UV detector. The result is stated as a weight percentage, i.e. parts by weight of TFA in 100 parts by weight of material measured.

The maximum influence in case of a hydrolysis of Tfa-liraglutide into liraglutide and TFA, is calculated to be 3.0 wt.%. Assuming 1 g of pure Tfa-liraglutide, this represents 0.2599 mmol of Tfa-liraglutide (1 g / 3847.26 g/mol). Accordingly, 1 g of pure Tfa-liraglutide releases in case of a complete hydrolysis 0.030 g of TFA (0.2599 mmol x 114.02 mg / mmol). The TFA content from a complete hydrolysis of Tfa-liraglutide would be 3.0 wt.%. Assuming a mixture of 0.8 g of pure liraglutide and 0.2 g of pure Tfa-liraglutide, this represents 0.052 mmol of Tfa-liraglutide. In case of a complete hydrolysis, the TFA content from a complete hydrolysis of Tfa-liraglutide is 0.6 wt.%.

### C-8: IPE content

An IPE content is determined by gas chromatography. The material to be measured is dissolved in water or DMSO. The result is stated as a weight percentage, i.e. parts by weight of IPE in 100 parts by weight of material measured.

### D) examples

### D-1: synthesis of solid-phase conjugated protected liraglutide precursor

### Example D-1-1: synthesis of solid-phase conjugated protected liraglutide precursor

Stepwise Fmoc-SPPS of Liraglutide is performed on a 100-200 or 200-400 mesh H-Gly-2-chlorotrityl resin (Bachem no. 4092098 or 4026823) or a H-Gly-2-chlorotritylamidomethyl resin using the standard Fmoc amino acid derivatives Fmoc-Ala-OH, Fmoc-Arg(Pbf)-OH, Fmoc-Asp(OtBu)-OH, Fmoc-Asp(OMpe)-OH, Fmoc-Gly-OH, Fmoc-Gln(Trt)-OH, Fmoc-Glu(OtBu)-OH, Boc-His(Boc)-OH, Fmoc-Ile-OH, Fmoc-Leu-OH, Fmoc-Phe-OH, Fmoc-Ser(tBu)-OH, Fmoc-Thr(tBu)-OH, Fmoc-Trp(Boc)-OH, Fmoc-Tyr(tBu)-OH, and Fmoc-Val-OH, as well as the previously described building block Fmoc-Lys(N-epsilon-(gamma-glutamyl(OtBu)-(N-alpha-hexadecanoyl))) (= Fmoc-Lys(palmitoyl-Glu-OtBu)-OH, see WO 2013-171135) and at least one pseudoproline dipeptide selected from Fmoc-Gly-Thr(Psi(Me,Me)pro)-OH, Fmoc-Phe-Thr(Psi(Me,Me)pro)-OH, Fmoc-Thr(tBu)-Ser(Psi(Me,Me)pro)-OH, Fmoc-Val-Ser(Psi(Me,Me)pro)-OH, and Fmoc-Ser(tBu)-Ser(Psi(Me,Me)pro)-OH. Coupling reactions are executed either with DIC/OxymaPure (RTM), TBTU/DIPEA or DEPBT/DIPEA with appropriate coupling (1.5-24.0 h) and Fmoc deprotection (0.5-4.0 h) times.

When desired, 20% piperidine in NMP was used instead of 20% piperidine in DMF to enhance deprotection rates. After coupling steps, an acetylation was optionally performed using acetic anhydride. DMF and IPA were used as solvents for the washing steps after acetylation or Fmoc deprotection.

The chemical structure of the solid-phase conjugated protected liraglutide precursor used at D-2-1, D-2-2, D-2-3, D-3-1 and D-3-2 is Boc-His(Boc)-Ala-Glu(OtBu)-Gly-Thr(tBu)-Phe-Thr(Psi(Me,Me)pro)-Ser(tBu)-Asp(OMpe)-Val-Ser(tBu)-Ser(tBu)-Tyr(tBu)-Leu-Glu(OtBu)-Gly-Gln(Trt)-Ala-Ala-Lys(palmitoyl-gamma-Glu(OtBu))-Glu(OtBu)-Phe-Ile-Ala-Trp(Boc)-Leu-Val-Arg(Pbf)-Gly-Arg(Pbf)-Gly-[2-chlorotritylamidomethyl resin].

The material of the solid-phase conjugated protected liraglutide precursor used at D-2-1, D-2-2 and D-3-1 is from the same synthetic batch. The material of the solid-phase conjugated protected liraglutide precursor used at D-2-3 and D-3-2 is from the same synthetic batch.

The chemical structure of the solid-phase conjugated protected liraglutide precursor used at D-2-4 and D-2-5 is Boc-His(Boc)-Ala-Glu(OtBu)-Gly-Thr(tBu)-Phe-Thr(Psi(Me,Me)pro)-Ser(tBu)-Asp(OMpe)-Val-Ser(tBu)-Ser(tBu)-Tyr(tBu)-Leu-Glu(OtBu)-Gly-Gln(Trt)-Ala-Ala-Lys(palmitoyl-gamma-Glu(OtBu))-Glu(OtBu)-Phe-Ile-Ala-Trp(Boc)-Leu-Val-Arg(Pbf)-Gly-Arg(Pbf)-Gly-[2-chlorotrityl resin].

The material of the solid-phase conjugated protected liraglutide precursor used at D-2-4 and D-2-5 is from the same synthetic batch.

### D-2: cleavage of solid-phase conjugated protected liraglutide precursor with precipitation

### Example D-2-1: prolonged stirring of precipitation slurry

30.0 g material of a solid-phase conjugated protected liraglutide precursor as obtainable from example D-1-1 is cleaved by addition to 240 mL of a cleavage composition of TFA / EDT / H₂O (87.6 / 6.4 / 6.0 in volume % based on volume of cleavage composition; 8 mL cleavage composition per 1 g of material to be cleaved, i.e. the weight of the material to be cleaved includes resin and covalently linked protected liraglutide precursor) at 26 °C. After 90 minutes of stirring, the cleavage suspension is filtered with a POR 0 reverse frit and the resin is washed with 2 x 15 mL TFA. The filtrates are combined and the overall volume is measured as 260 mL. The combined filtrates are cooled to < -10 °C and under stirring, 520 mL IPE are added at such a rate that a temperature of -10 °C is not exceeded. A precipitation slurry is formed. The temperature of the precipitation slurry is raised to 0 °C in 15 minutes and the precipitation slurry is stirred for 30 minutes at 0 °C. This represents in table D-2-1-1 the formal start time. 100 mL of the precipitation slurry are removed and stored at -20 °C. The remaining amount of the precipitation slurry is warmed to a temperature of 25 °C und stirring is continued at 25 °C. A test sample in the size of 5 mL precipitation slurry is taken at the time periods and from the precipitation slurries as stated in table D-2-1-1. The test sample is filtered with a POR 4 frit. The obtained initial precipitate of the test sample is twice re-suspended in 3 mL IPE and re-filtered. The obtained intermediate precipitate of the test sample is dried overnight for 17 h at 25°C under a vacuum of 5 mbar to obtain a final precipitate of the test sample. An analytical HPLC sample is prepared from the final precipitate of the test sample with solution for dissolving or diluting No. C-2-1-03 (35 vol.% ACN with 0.71 vol.-pph 1.7 M (NH₄)₃PO₄ pH 7.8). The analytical HPLC sample is measured according to the analytical HPLC method. Table D-2-1-1 depicts the determined area percentage of liraglutide and Tfa-liraglutide from the HPLC-UV chromatogram of the analytical HPLC of the final precipitate of the test sample.

**Table D-2-1-1**

| test sample No. | temperature ^{c)} | time period d) | area percentage liraglutide [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| D-2-1-01 a) | 0 °C | 0 ^{e)} | 59.23 | 13.93 | 0.235 | 73.16 |
| | | | | | | |
| D-2-1-02 a) | -20 °C | 15 h | 66.50 | 7.17 | 0.107 | 73.67 |
| | | | | | | |
| D-2-1-03 b) | 25 °C | 17 h | 69.47 | 2.07 | 0.030 | 71.54 |
| D-2-1-04 b) | 25 °C | 24 h | 71.50 | 1.94 | 0.027 | 73.44 |
| D-2-1-05 b) | 25 °C | 4 d | 66.91 | 2.95 | 0.044 | 69.86 |
| D-2-1-06 b) | 25 °C | 5 d | 65.26 | 3.54 | 0.054 | 68.80 |
| D-2-1-07 b) | 25 °C | 6 d | 66.66 | 3.44 | 0.052 | 70.10 |

| | | | | | | |
|---|---|---|---|---|---|---|
| Footnotes: a) comparative precipitated mixture b) inventive precipitated mixture c) temperature of the precipitation slurry at the time when the test sample is taken and also the temperature, which is kept during the time period from the start time until taking of the test sample d) time period of storage at -20 °C or stirring at 25 °C from the start time until taking of the test sample e) time period at the start time set to 0, i.e. point of time after stirring the precipitation slurry for 30 minutes at 0 °C | | | | | | |

The results of table D-2-1-1 show:
- by a comparison of example D-2-1-02 versus example D-2-1-01 that a storage at
- 20 °C for 15 h leads to a reduction of the area percentage of Tfa-liraglutide, but the area percentage of Tfa-liraglutide remains relatively high. The sum of the area percentages of liraglutide and Tfa-liraglutide indicate that there is no overall degradation at -20 °C;
- by a comparison of examples D-2-1-03 to D-2-1-07 versus example D-2-1-01 that a prolonged time of stirring at 25 °C leads to a significant reduction of the area percentage of Tfa-liraglutide;
- by a comparison of examples D-2-1-03 to D-2-1-04 versus examples D-2-1-05 to D-2-1-07 that a very prolonged time of stirring at 25 °C leads to a type of equilibrium with the area percentage of Tfa-liraglutide not being reduced to zero;
- by a comparison of examples D-2-1-05 to D-2-1-07 versus examples D-2-1-03 to D-2-1-04 that after a very prolonged time of stirring at 25 °C, a slight reduction of the sum of the area percentages of Tfa-liraglutide and liraglutide occurs. The latter might indicate that an overall degradation starts taking place under the very prolonged time of stirring of the precipitation slurry at its still acidic environment.

### Example D-2-2: prolonged stirring of precipitation slurry

7.0 g material of a solid-phase conjugated protected liraglutide precursor as obtainable from example D-1-1 is put in a 100 mL round bottom flask. At 26 °C, 56 mL of a cleavage composition of TFA / EDT / H₂O (87.6 / 6.4 / 6.0 in vol.% based on volume of cleavage composition; 8 mL cleavage composition per 1 g of material to be cleaved, i.e. weight of the material to be cleaved includes resin and covalently linked protected liraglutide precursor) are added. After 90 minutes of stirring at 26 °C, the cleavage suspension is filtered with a POR 3 frit and the resin is washed with 2 x 7 mL TFA. The filtrates are combined and the overall volume is measured as around 70 mL. The combined filtrates are transferred to a 250 mL reactor with a cooling jacket, which is cooled with a cooling liquid at a temperature of -30 °C. Once the internal temperature in the reactor is below -15 °C, 140 mL IPE are added under stirring via a dropping funnel. The rate of IPE addition is adjusted to avoid that the internal temperature exceeds -10 °C. A precipitation slurry is formed. After completion of the IPE addition, the temperature of the cooling liquid in the cooling jacket is raised to 22 °C. After 20 minutes, the internal temperature has reached 20 °C. This represents in table D-2-2-1 the formal start time. Stirring is continued at a temperature of 22 °C. A test sample in the size of around 15 mL precipitation slurry is taken at the time periods as stated in table D-2-2-1. The test sample is filtered with a POR 4 frit. The obtained initial precipitate of the test sample is three times re-suspended in around 5 mL IPE and re-filtered. The obtained intermediate precipitate of the test sample is dried overnight for 17 h at 25°C under a vacuum of 5 mbar to obtain a final precipitate of the test sample. An analytical HPLC sample is prepared from the final precipitate of the test sample with solution for dissolving or diluting No. C-2-2-09 (50 vol.% ACN with 1.0 vol.-pph 1.0 M (NH₄)₃PO₄ pH 7.8). The analytical HPLC sample is measured according to the analytical HPLC method. Table D-2-2-1 depicts the determined area percentage of liraglutide and Tfa-liraglutide from the HPLC-UV chromatogram of the analytical HPLC sample of the final precipitate of the test sample.

**Table D-2-2-1**

| test sample No. | temperature ^{c)} | time period d) | area percentage liraglutide [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| D-2-2-01 a) | 20 °C | 0 e) | 63.45 | 13.16 | 0.207 | 76.61 |
| D-2-2-02 a) | 22 °C | 1 h | 65.24 | 11.73 | 0.180 | 76.97 |
| D-2-1-03 a) | 22 °C | 2 h | 64.61 | 9.69 | 0.150 | 74.30 |
| D-2-2-04 a) | 22 °C | 3 h | 65.16 | 8.40 | 0.129 | 73.56 |
| D-2-2-05 a) | 22 °C | 4 h | 68.25 | 7.98 | 0.117 | 76.23 |
| D-2-2-06 b) | 22 °C | 19h | 69.01 | 2.52 | 0.037 | 71.53 |
| D-2-2-07 b) | 22 °C | 1 d | 71.31 | 2.61 | 0.037 | 73.92 |
| D-2-2-08 b) | 22 °C | 2 d | 74.54 | 2.15 | 0.029 | 76.69 |
| D-2-2-09 b) | 22 °C | 3 d | 71.49 | 1.56 | 0.022 | 73.05 |
| D-2-2-10 b) | 22 °C | 4 d | 69.88 | 1.54 | 0.022 | 71.42 |
| D-2-1-11 b) | 22 °C | 7 d | 68.95 | 1.41 | 0.020 | 70.36 |

| | | | | | | |
|---|---|---|---|---|---|---|
| Footnotes: a) comparative precipitated mixture b) inventive precipitated mixture c) temperature of the precipitation slurry at the time when the test sample is taken and also the temperature, which is kept during the time period from the start time until taking of the test sample d) time period of stirring at 22 °C from the start time until taking of the test sample e) time period at the start time set to 0, i.e. point of time after having reached 20 °C internal temperature f) the pH values of the analytical HPLC samples (but only tentative ones in view of a measurement by a pH electrode in an aqueous mixture with a high content of organic solvent) are: D-2-2-1 - 8.1, D-2-2-2 - 8.0, D-2-2-3 - 8.0, D-2-2-4 - 8.0, D-2-2-5 - 8.0, D-2-2-6 - 7.8, D-2-2-7 - 8.0, D-2-2-8 - 7.9, D-2-2-9 - 7.9, D-2-2-10 - 8.0, D-2-2-11 - 8.0 | | | | | | |

The results of table D-2-2-1 show:
- by a comparison of examples D-2-2-06 to D-2-2-11 versus examples D-2-2-01 to D-2-2-05
   that a prolonged time of stirring at 22 °C leads to a significant reduction of the area percentage of Tfa-liraglutide;

- by a comparison of examples D-2-2-09 to D-2-2-11 versus examples D-2-2-06 to D-2-2-08 that a very prolonged time of stirring at 22 °C leads to a type of equilibrium with the area percentage of Tfa-liraglutide not being reduced to zero;
- by a comparison of example D-2-2-11 to D-2-2-01 to D-2-2-10 that after a very prolonged time of stirring at 22 °C, a slight reduction of the sum of the area percentages of Tfa-liraglutide and liraglutide occurs. The latter might indicate that an overall degradation starts taking place under the very prolonged time of stirring of the precipitation slurry at its still acidic environment.

### Example D-2-3: short time stirring of precipitation slurry

7.0 g material of a solid-phase conjugated protected liraglutide precursor as obtainable from example D-1-1 is cleaved at 26 °C. 56 mL of a cleavage composition of TFA / EDT / H₂O (87.6 / 6.4 / 6.0 in vol.% based on volume of cleavage composition; 8 mL cleavage composition per 1 g of material to be cleaved, i.e. the weight of the material to be cleaved includes resin and covalently linked protected liraglutide precursor) are added. After 90 minutes of stirring at 26 °C, the cleavage suspension is filtered with a POR 3 frit and the resin is washed with 2 x 7 mL TFA. The filtrates are combined and the overall volume is measured as around 70 mL of a cloudy yellow liquid. The combined filtrates are transferred to a 250 mL OptiMax reactor from Mettler Toledo Inc. with a cooling jacket and stirred. The cooling jacket is cooled with a cooling liquid at a temperature of -30 °C. Once the internal temperature in the reactor is below -15 °C, 140 mL IPE of room temperature are added under stirring via a dropping funnel. The rate of IPE addition is adjusted to avoid that the internal temperature exceeds -10 °C. A precipitation slurry is formed. The IPE addition is completed after around 20 minutes. After completion of the IPE addition, the temperature of the cooling liquid in the cooling jacket is raised to 25 °C. After 15 minutes, the internal temperature has reached 20 °C. Directly after the internal temperature has reached 20 °C, the precipitation slurry is filtered with a POR 4 frit. The obtained initial precipitate is three times re-suspended in 5 mL IPE and re-filtered. The obtained intermediate precipitate is dried for 2 days at 25°C under a vacuum of 5 mbar to obtain a final precipitate in a yield of 3.92 g. Its water content is 3.4 wt.%, its IPE content is 0.478 wt.% and its TFA content is 9.0 wt.%.

A part of the obtained final precipitate is used as a material for LC-MS described at C-4. A part of the obtained final precipitate is used as a material for some of the investigations described at D-4.

### Example D-2-4: 18 h stirring time of precipitation slurry

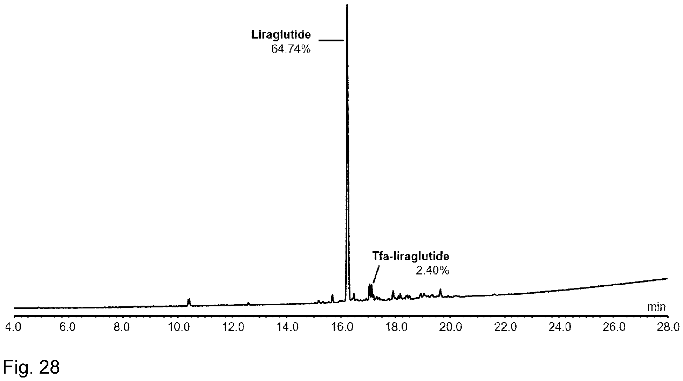

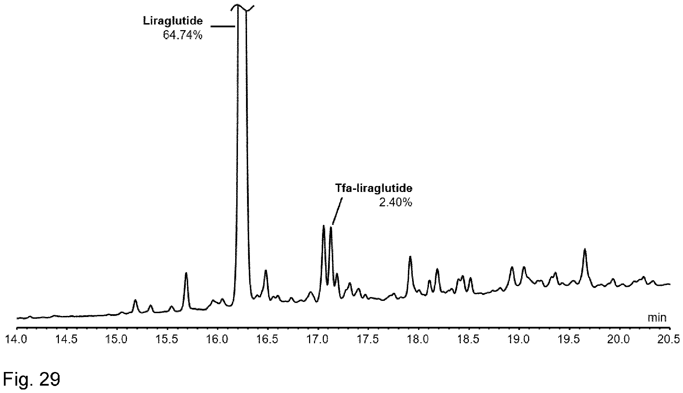

25 g material of a solid-phase conjugated protected liraglutide precursor as obtainable from example D-1-1 is cleaved at 26 °C. 200 mL of a cleavage composition of TFA / EDT / H₂O (87.6 / 6.4 / 6.0 in vol.% based on volume of cleavage composition; 8 mL cleavage composition per 1 g of material to be cleaved, i.e. the weight of the material material to be cleaved includes resin and covalently linked protected liraglutide precursor) are added. After 90 minutes of stirring at 26 °C, the cleavage suspension is filtered with a POR 0 reverse frit and the resin is washed with 2 x 25 mL TFA. The filtrates are combined and the overall volume is measured as around 240 mL of a cloudy yellow liquid. The combined filtrates are transferred to a temperature-controlled 1 L double jacketed glass reactor and stirred. The cooling jacket is cooled with a cooling liquid at a temperature of -25 °C. Once the internal temperature in the reactor is below -15 °C, 480 mL IPE (precooled to - 20°C in a second temperature controlled 1 L double jacketed glass reactor) are added under stirring via a connector. The rate of IPE addition is adjusted to avoid that the internal temperature exceeds -10 °C. A precipitation slurry is formed. The IPE addition is completed after around 20 minutes. After completion of the IPE addition, the temperature of the cooling liquid in the cooling jacket is raised to 22 °C. After 20 minutes, the internal temperature has reached 20 °C. The precipitation slurry is stirred at an internal temperature of 20-22°C for 18 h. After the stirring time is passed, the precipitation slurry is transferred into a 1 L nutsche filter, equipped with two PTFE filter cloth with a mesh size of 8 µm lying on top of each other. The precipitation slurry is directly filtered with a nitrogen overpressure of up to 1.3 bar. The obtained initial precipitate is three times re-suspended in 75 mL IPE and re-filtered. The obtained intermediate precipitate is dried for 1 day at 25 °C under a vacuum of 5 mbar to obtain a final precipitate in a yield of 11.50 g. Its water content is 1.5 wt.%, its IPE content is 0.467 wt.% and its TFA content is 13.9 wt.%. An analytical HPLC sample D-2-4-01 is prepared from the final precipitate with solution for dissolving or diluting No. C-2-2-09 (50 vol.% ACN with 1.0 vol.-pph 1.0 M (NH₄)₃PO₄ pH 7.8). Table D-2-5-1 depicts the determined area percentage of liraglutide and Tfa-liraglutide from the HPLC-UV chromatogram of the analytical HPLC sample D-2-4-01. Fig. 28 to 29 depict extracts of the HPLC-UV chromatogram of D-2-4-01. A part of the final precipitate is further purified at D-6-1.

### Example D-2-5: 3 h of stirring time of precipitation slurry

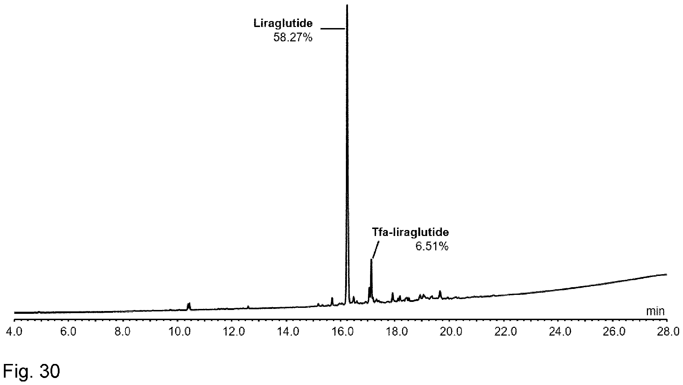

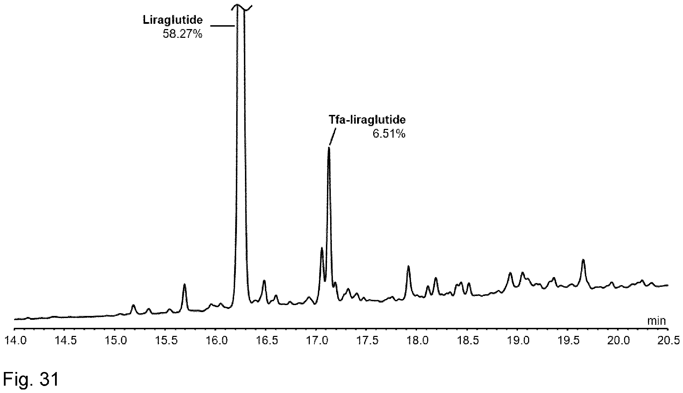

25 g material of a solid-phase conjugated protected liraglutide precursor as obtainable from example D-1-1 is cleaved at 26 °C. 200 mL of a cleavage composition of TFA / EDT / H₂O (87.6 / 6.4 / 6.0 in vol.% based on volume of cleavage composition; 8 mL cleavage composition per 1 g of material to be cleaved, i.e. the weight of the material to be cleaved includes resin and covalently linked protected liraglutide precursor) are added. After 90 minutes of stirring at 26 °C, the cleavage suspension is filtered with a POR 0 reverse frit and the resin is washed with 2 x 25 mL TFA. The filtrates are combined and the overall volume is measured as around 240 mL of a cloudy yellow liquid. The combined filtrates are transferred to a temperature controlled 1 L double jacketed glass reactor and stirred. The cooling jacket is cooled with a cooling liquid at a temperature of -25 °C. Once the internal temperature in the reactor is below -15 °C, 480 mL IPE (precooled to -20°C in a second temperature controlled 1 L double jacketed glass reactor) are added under stirring via a connector. The rate of IPE addition is adjusted to avoid that the internal temperature exceeds -10 °C. A precipitation slurry is formed. The IPE addition is completed after around 20 minutes. After completion of the IPE addition, the temperature of the cooling liquid in the cooling jacket is raised to 22 °C. After 20 minutes, the internal temperature has reached 20 °C. The precipitation slurry is stirred at an internal temperature of 20-22°C for 3 h. After the stirring time is passed, the precipitation slurry is transferred into a 1 L nutsche filter, equipped with two PTFE filter cloth with a mesh size of 8 µm lying on top of each other. The precipitation slurry is directly filtered with a nitrogen overpressure of up to 1.3 bar. The obtained initial precipitate is three times re-suspended in 75 mL IPE and re-filtered. The obtained intermediate precipitate is dried for 1 day at 25°C under a vacuum of 5 mbar to obtain a final precipitate in a yield of 11.32 g. Its water content is 2.2 wt.%, its IPE content is 0.553 wt.% and its TFA content is 13.5 wt.%. An analytical HPLC sample D-2-5-01 is prepared from the final precipitate with solution for dissolving or diluting No. C-2-2-09 (50 vol.% ACN with 1.0 vol.-pph 1.0 M (NH₄)₃PO₄ pH 7.8). Table D-2-5-1 depicts the determined area percentage of liraglutide and Tfa-liraglutide from the HPLC-UV chromatogram of the analytical HPLC sample D-2-5-01. Fig. 30 to 31 depict extracts of the HPLC-UV chromatogram of D-2-5-01.

**Table D-2-5-1**

| test sample No. | temperature ^{c)} | stirring time ^{d)} | area percentage liraglutide [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| D-2-5-01 ^{a)} | 20-22 °C | 3 h | 58.27 | 6.51 | 0.112 | 64.78 |
| D-2-4-01 ^{b)} | 20-22 °C | 18 h | 64.74 | 2.40 | 0.037 | 67.14 |

| | | | | | | |
|---|---|---|---|---|---|---|
| Footnotes: a) comparative precipitated mixture b) inventive precipitated mixture b) temperature, which is kept during the stirring time of the precipitated slurry d) stirring time of the precipitated slurry | | | | | | |

The results of table D-2-5-1 show:
- by test sample No. D-2-4-01 versus test sample No. D-2-5-01 that a prolonged stirring time of the precipitated slurry leads in the final precipitate to a significantly reduced area percentage of Tfa-liraglutide.

### D-3: cleavage of solid-phase conjugated protected liraglutide precursor without precipitation

### Example D-3-1: stirring of cleavage slurry for 90 minutes

5.0 g material of the solid-phase conjugated protected liraglutide precursor as obtainable from example D-1-1 is cleaved by addition to 40 mL of a cleavage composition of TFA / EDT / H₂O (87.6 / 6.4 / 6.0 in volume % based on volume of cleavage composition; 8 mL cleavage composition per 1 g of material to be cleaved, i.e. the weight of the material to be cleaved includes resin and covalently linked protected liraglutide precursor) at 26 °C. The addition to the cleavage composition represents the start time in table D-3-1-1. After 90 minutes of stirring at 26 °C, a test sample in the size of 4 mL of the cleavage suspension is removed via a pipette. The test sample is put into a disposable syringe equipped with a frit. A plunger is mounted to the disposable syringe and the test sample is pressed through the frit by the plunger of the disposable syringe into 8 mL of IPE, which is cooled to a temperature < -10 °C, to obtain a precipitation slurry of the test sample with a temperature around 0 °C. The precipitation slurry is stirred for 30 minutes and is warmed during that time to room temperature. Afterwards, the precipitation slurry of the test sample is filtered with a POR 4 frit. The obtained initial precipitate of the test sample is twice re-suspended in 5 mL IPE and re-filtered. The obtained intermediate precipitate of the test sample is dried overnight for 17 h at 25 °C under a vacuum of 5 mbar to obtain a final precipitate of the test sample. An analytical HPLC sample is prepared from the final precipitate of the test sample with solution for dissolving or diluting No. C-2-1-03 (35 vol.% ACN with 0.71 vol.-pph 1.7 M (NH₄)₃PO₄ pH 7.8). The analytical HPLC sample is measured according to the analytical HPLC method. Table D-3-1-1 depicts the determined area percentage of liraglutide and Tfa-liraglutide from the HPLC-UV chromatogram of the analytical HPLC sample of the final precipitate of the test sample. The obtained final precipitate of the test sample is used as a material for some of the investigations described at D-4.

**Table D-3-1-1**

| test sample No. | temperature ^{b)} | time period ^{c)} from start time ^{d)} [minutes] | area percentage liraglutide [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| D-3-1-01 ^{a)} | 26 °C | 90 | 65.60 | 10.75 | 0.164 | 76.35 |

| | | | | | | |
|---|---|---|---|---|---|---|
| Footnotes: a) comparative precipitated mixture b) temperature, which is kept during the time period from the start time until taking of the test sample c) time period from the start time until taking of the test sample d) addition of cleavage composition to solid-phase conjugated protected liraglutide precursor | | | | | | |

The results of table D-3-1-1 show:
- by example D-3-1-01 a situation after 90 minutes exposure to the cleavage composition at 26 °C.

### Example D-3-2: prolonged stirring of cleavage slurry

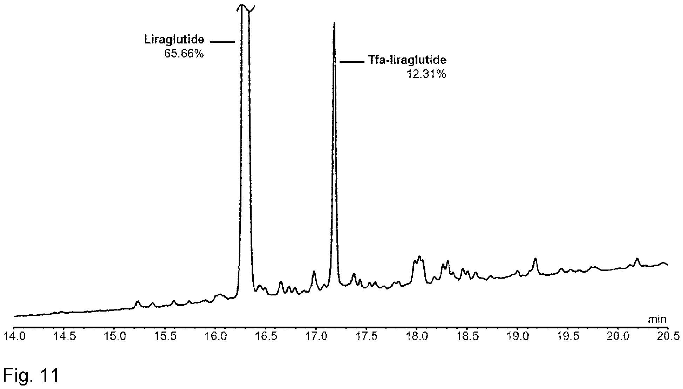

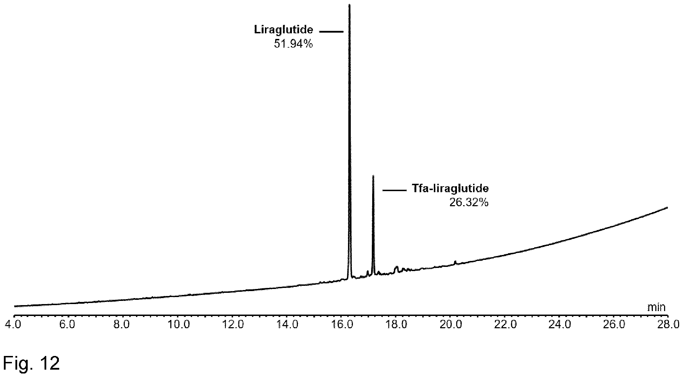

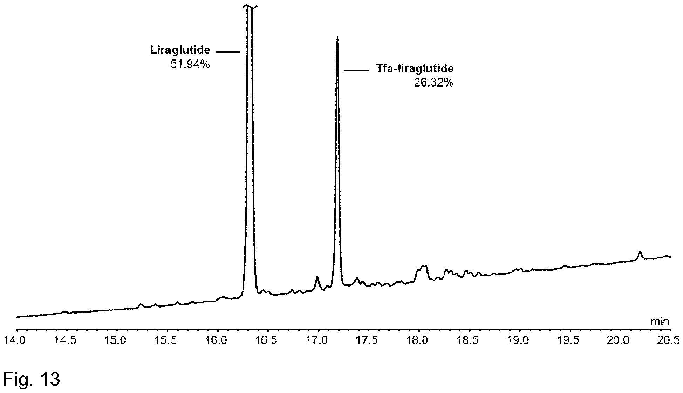

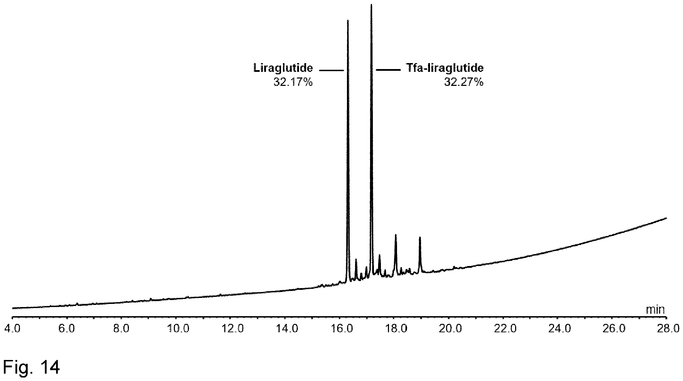

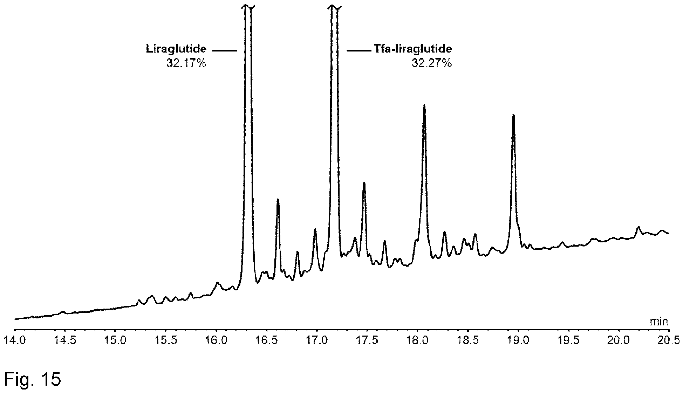

7.0 g material of a solid-phase conjugated protected liraglutide precursor as obtainable from example D-1-1 is put in a 100 mL round bottom flask. At 26 °C, 56 mL of a cleavage composition of TFA / EDT / H₂O (87.6 / 6.4 / 6.0 in vol.% based on volume of cleavage composition; 8 mL cleavage composition per 1 g of material to be cleaved, i.e. the weight of the material to be cleaved includes resin and covalently linked protected liraglutide precursor) are added. The cleavage slurry is stirred at 26 °C. At each time period described in table D-3-2-1, a test sample of around 5 mL of cleavage slurry is removed. The test sample is directly after removal filtered with a POR 3 frit and the resin is washed with 2 x 0.6 mL TFA. The filtrates are combined and the overall volume of around 6 mL is put into a 30 mL pill jar. The pill jar is put into a cooling bath of ethanol and dry ice with a temperature of around -40 °C and the content of the pill jar is stirred. After around 1 minute of cooling time, 12 mL IPE, which are not cooled, are slowly added under stirring to the pill jar. The whole addition time of the IPE is around 5 minutes. A precipitation slurry is formed. Directly after the end of the IPE addition, the pill jar is removed from the cooling bath of ethanol and dry ice and put into a water bath at room temperature. After around 2 minutes stirring at the water bath for warming fastly to room temperature, the precipitation slurry from the pill jar is filtered with a POR 4 frit. The obtained initial precipitate of the test sample is twice re-suspended in around 5 mL IPE and re-filtered. The obtained intermediate precipitate of the test sample is dried overnight for 17 h at 25°C under a vacuum of 5 mbar to obtain a final precipitate of the test sample. A first analytical HPLC sample is prepared from the final precipitate of the test sample with solution for dissolving or diluting No. C-2-2-09 (50 vol.% ACN with 1.0 vol.-pph 1.0 M (NH₄)₃PO₄ pH 7.8). A second analytical HPLC sample is prepared from the final precipitate of the test sample with solution for dissolving or diluting No. C-2-2-10 (60 vol.% ACN with 1.0 vol.-pph 1.0 M (NH₄)₃PO₄ pH 7.8). The analytical HPLC samples are measured according to the analytical HPLC method. After a storage time of 7.5 h at 10 °C, the analytical HPLC samples are re-measured except those after 1440 minutes. Table D-3-2-1 depicts the determined area percentage of liraglutide and Tfa-liraglutide from the HPLC-UV chromatogram of the analytical HPLC sample of the final precipitate of the test sample. Fig. 10 to 11 depict extracts of the HPLC-UV chromatogram of D-3-2-03. Fig. 12 to 13 depict extracts of the HPLC-UV chromatogram of D-3-2-09. Fig. 14 to 15 depict extracts of the HPLC-UV chromatogram of D-3-2-11.

**Table D-3-2-1**

| | temperature ^{b)} | time period ^{c)} from start time ^{d)} [minutes] | | | | |
|---|---|---|---|---|---|---|
| analytical HPLC sample No. | ACN in solvent basis | pH value ^{e), f)} of analytical HPLC sample | area percentage liraglutide ^{g)} [%] | area percentage Tfa-liraglutide ^{g)} [%] | ratio of area percentages Tfa-liraglutide / liraglutide ^{g)} | sum of area percentages of Tfa-liraglutide and liraglutide ^{g)} [%] |
| | 26 °C | 45 | | | | |
| D-3-2-01 ^{a)} | 50 vol.% ACN | 7.74 | 64.30 (63.89) | 6.54 (6.53) | 0.102 (0.102) | 70.84 (70.42) |
| D-3-2-02 ^{a)} | 60 vol.% ACN | 7.67 | 63.37 (63.82) | 6.42 (6.42) | 0.101 (0.101) | 69.79 (70.24) |
| | | | | | | |
| | 26 °C | 90 | | | | |
| D-3-2-03 ^{a)} | 50 vol.% ACN | 7.75 | 65.66 (64.95) | 12.31 (12.02) | 0.187 (0.185) | 77.97 (76.97) |
| D-3-2-04 ^{a)} | 60 vol.% ACN | 7.81 | 64.43 (65.39) | 12.68 (12.85) | 0.197 (0.197) | 77.11 (78.24) |
| | | | | | | |
| | 26 °C | 135 | | | | |
| D-3-2-05 ^{a)} | 50 vol.% ACN | 7.79 | 62.17 (62.94) | 16.05 (15.75) | 0.258 (0.250) | 78.22 (78.69) |
| D-3-2-06 ^{a)} | 60 vol.% ACN | 7.82 | 62.34 (63.31) | 15.75 (15.81) | 0.253 (0.250) | 78.09 (79.12) |
| | | | | | | |
| | 26 °C | 180 | | | | |
| D-3-2-07 ^{a)} | 50 vol.% ACN | 7.93 | 60.70 (60.15) | 20.25 (19.84) | 0.334 (0.330) | 80.95 (79.99) |
| D-3-2-08 ^{a)} | 60 vol.% ACN | 7.78 | 59.32 (59.95) | 18.88 (18.97) | 0.318 (0.316) | 78.20 (78.92) |
| | | | | | | |
| | 26 °C | 360 | | | | |
| D-3-2-09 ^{a)} | 50 vol.% ACN | 7.85 | 51.94 (51.04) | 26.32 (25.07) | 0.507 (0.491) | 78.26 (76.11) |
| D-3-2-10 ^{a)} | 60 vol.% ACN | 7.89 | 53.43 (52.46) | 25.45 (25.23) | 0.476 (0.481) | 77.88 (77.69) |
| | | | | | | |
| | 26 °C | 1440 | | | | |
| D-3-2-11 ^{a)} | 50 vol.% ACN | 7.61 | 32.17 | 32.27 | 1.003 | 64.44 |
| D-3-2-12 ^{a)} | 60 vol.% ACN | 7.80 | 32.10 | 31.79 | 0.990 | 63.89 |

| | | | | | | |
|---|---|---|---|---|---|---|
| Footnotes: a) comparative precipitated mixture b) temperature, which is kept during the time period from the start time until taking of the test sample c) time period from the start time until taking of the test sample d) addition of cleavage composition to solid-phase conjugated protected liraglutide precursor e) only tentative pH value in view of a measurement by a pH electrode in an aqueous mixture with a high content of organic solvent f) values without rounding towards one decimal provided in view of tendency with a tentative pH value of solution for dissolving or diluting No. C-2-2-09 (50 vol.% ACN) of 8.08 and a tentative pH value of solution for dissolving or diluting No. C-2-2-10 (60 vol.% ACN) of 8.09 g) area percentage values from re-measurement of the analytical HPLC sample after a storage time of 7.5 h | | | | | | |

The results of table D-3-2-1 show:
- that the area percentage of Tfa-liraglutide increases continuously during the observed stirring time;
- that an almost linear increase of the area percentage of Tfa-liraglutide occurs until a stirring time of 180 minutes;
- that an overall degradation takes places by a stirring time of 1440 minutes (= 24 h);
- that the results of the analytical HPLC samples prepared with the two solutions for dissolving or diluting are comparable;
- that a storage for 7.5 h of the analytical HPLC samples does not lead to a significant change of the initial area percentages in the analytical HPLC samples.

### D-4: variation of analytical HPLC samples

### Example D-4-1: analytical HPLC samples of a final precipitate

1 mg of the final precipitate of the test sample from example D-3-1 is dissolved in 1 mL solution for dissolving or diluting No. C-2-1-06 (50 vol.% ACN and 50 vol.% deionized water). The obtained analytical HPLC sample is measured according to the analytical HPLC method. Table D-4-1-1 depicts the determined area percentage of liraglutide and Tfa-liraglutide from the HPLC-UV chromatogram of the analytical HPLC sample of the final precipitate of the test sample. For comparison, the area percentage of analytical HPLC sample D-3-1-01, which is prepared from the same final precipitate but with solution for dissolving or diluting No. C-2-1-03 (calculated 35 vol.% ACN and 65 vol.% H₂O and 0.71 vol.-pph 1.7 M (NH₄)₃PO₄ pH 7.8), is also depicted.

**Table D-4-1-1**

| analytical HPLC sample No. | ACN in solvent basis of solution for dissolving or diluting 1.7 M (NH₄)₃PO₄ pH 7.8 [vol.-pph] | pH value ^{b)} of the analyt-ical HPLC sample | area percentage liraglutid e [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| D-3-1-01 ^{a)} | 35 vol.% ACN | 7.7 | 65.60 | 10.75 | 0.164 | 76.35 |
| | 0.71 | | | | | |
| 0-4-1-01 ^{a)} | 50 vol.% ACN | 2.3 | 69.01 | 1.31 | 0.020 | 70.32 |
| | - | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent | | | | | | |

The results of table D-4-1-1 show:
- by a comparison of example D-4-1-01 versus example D-3-1-01 that a dissolving of the final precipitate of the test sample in a solution for dissolving or diluting without a buffer salt leads to an almost complete disappearance of the area percentage of Tfa-liraglutide in favor of the area percentage of liraglutide.

The analytical HPLC sample No. D-3-1-01 is stored at 20 °C for 8 h. The obtained analytical HPLC sample, which is named D-3-1-01-8h, is measured according to the analytical HPLC method. Table D-4-1-2 depicts the determined area percentage of liraglutide and Tfa-liraglutide from the HPLC-UV chromatogram of the analytical HPLC sample of the final precipitate of the test sample. For comparison, the area percentage of analytical HPLC sample D-3-1-01 is also depicted.

**Table D-4-1-2**

| analytical HPLC sample No. | ACN in solvent basis of solution for dissolving or diluting | pH value ^{b)} of the analytical HPLC sample | area percentage liraglutide [%] | area percentage Tfa-liraglutide [%] | ratio of area percentages Tfa-liraglutide / liraglutide | sum of area percentages of Tfa-liraglutide and liraglutide [%] |
|---|---|---|---|---|---|---|
| | 1.7 M (NH₄)₃PO₄ pH 7.8 [vol.-pph] | | | | | |
| | | | | | | |
| D-3-1-01 ^{a)} | 35 vol.% ACN | 7.7 | 65.60 | 10.75 | 0.164 | 76.35 |
| | 0.71 | | | | | |
| | | | | | | |
| D-3-1-01-8h ^{a), c)} | 35 vol.% ACN | - ^{d)} | 66.74 | 9.99 | 0.149 | 76.73 |
| | 0.71 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) analytical HPLC sample after storage of 8 h at 20 °C d) not determined | | | | | | |

The results of table D-4-1-2 show:
- by a comparison of example D-3-1-01-8h versus example D-3-1-01 that the area percentage of Tfa-liraglutide has slightly decreased during the 8 hours of storage of the analytical HPLC sample.

### Example D-4-2: analytical HPLC samples with low ACN content

1 mg of the final precipitate from example D-2-3 is dissolved in 1 mL of a respective solution for dissolving or diluting as described in table D-4-2-1. The obtained analytical HPLC sample is measured according to the analytical HPLC method. The obtained analytical HPLC sample is stored for 9.5 hours at 10 °C and re-measured. Table D-4-2-1 depicts the determined area percentage of liraglutide and Tfa-liraglutide from the HPLC-UV chromatogram of the analytical HPLC sample of the final precipitate.

**Table D-4-2-1**

| analytical HPLC sample No. | ACN in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| | | | | | | |
| 0-4-2-01 ^{a), f)} | 5 vol.% ACN | 2.92 ^{e)} {7.27} | - ^{e)} | - ^{e)} | - ^{e)} | - ^{e)} |
| | - | | | | | |
| | | | | | | |
| D-4-2-02 ^{a), f)} | 5 vol.% ACN | 7.75 {6.86} | 75.53 (74.90) | 2.15 (2.08) | 0.028 (0.028) | 77.68 (76.98) |
| | 0.5 | | | | | |
| | | | | | | |
| D-4-2-03 ^{a), f)} | 5 vol.% ACN | 7.40 {7.86} | 75.12 (74.54) | 3.77 (3.55) | 0.050 (0.048) | 78.89 (78.09) |
| | 1.0 | | | | | |
| | | | | | | |
| D-4-2-04 ^{a), f)} | 5 vol.% ACN | 7.57 {7.90} | 74.16 (74.15) | 3.90 (3.84) | 0.053 (0.052) | 78.06 (77.99) |
| | 2.0 | | | | | |
| | | | | | | |
| D-4-2-05 ^{a), f)} | 5 vol.% ACN | 7.91 {7.74} | 74.63 (74.16) | 3.63 (3.38) | 0.049 (0.046) | 78.26 (77.54) |
| | 5.0 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets e) final precipitate has not completely dissolved - no HPLC measurement conducted f) comparative analytical HPLC sample | | | | | | |

The results of table D-4-2-1 show:
- that a very high content of H₂O in the analytical HPLC samples leads to a significantly reduced area percentage of Tfa-liraglutide.

### Example D-4-3: analytical HPLC samples with increasing ACN content

1 mg of the final precipitate from example D-2-3 is dissolved in 1 mL of a respective solution for dissolving or diluting as described in table D-4-3-1. The obtained analytical HPLC sample is measured according to the analytical HPLC method. The obtained analytical HPLC sample is stored for 9.5 hours at 10 °C and re-measured. Table D-4-3-1 depicts the determined area percentage of liraglutide and Tfa-liraglutide from the HPLC-UV chromatogram of the analytical HPLC sample of the final precipitate.

**Table D-4-3-1**

| analytical HPLC sample No. | ACN in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| | | | | | | |
| 0-4-3-01 ^{a), e)} | 5 vol.% ACN | 7.39 {7.84} | 75.27 (75.10) | 2.26 (2.24) | 0.030 (0.030) | 77.53 (77.34) |
| | 1.0 | | | | | |
| | | | | | | |
| D-4-3-02 ^{a), e)} | 10 vol.% ACN | 7.46 {7.91} | 74.68 (74.19) | 2.99 (2.93) | 0.040 (0.039) | 77.67 (77.12) |
| | 1.0 | | | | | |
| | | | | | | |
| D-4-3-03 ^{a)} | 25 vol.% ACN | 7.66 {8.02} | 71.21 (70.71) | 7.16 (6.89) | 0.101 (0.097) | 78.37 (77.60) |
| | 1.0 | | | | | |
| | | | | | | |
| D-4-3-04 ^{a)} | 50 vol.% ACN | 7.82 {8.10} | 66.68 (67.04) | 11.06 (10.97) | 0.166 (0.165) | 77.74 (78.01) |
| | 1.0 | | | | | |
| | | | | | | |
| D-4-3-05 ^{a)} | 60 vol.% ACN | 7.86 {8.10} | 66.71 (66.38) | 11.15 (11.02) | 0.167 (0.166) | 77.86 (77.40) |
| | 1.0 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets e) comparative analytical HPLC sample | | | | | | |

The results of table D-4-3-1 show:
- that a content in the range of 50 vol.% ACN together with a buffer salt in the analytical HPLC samples leads to a determination of comparably high area percentages of Tfa-liraglutide.

A further series of analytical HPLC samples is prepared 5 days after the series described in table D-4-3-1. 1 mg of the final precipitate from example D-2-3 is dissolved in 1 mL of a respective solution for dissolving or diluting as described in table D-4-3-2. The obtained analytical HPLC sample is measured according to the analytical HPLC method. The obtained analytical HPLC sample is stored for 8 hours at 10 °C and re-measured. Table D-4-3-2 depicts the determined area percentage of liraglutide and Tfa-liraglutide from the HPLC-UV chromatogram of the analytical HPLC sample of the final precipitate.

**Table D-4-3-2**

| analytical HPLC sample No. | ACN in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| | | | | | | |
| D-4-3-06 ^{a)} | 50 vol.% ACN | 7.76 {8.05} | 67.99 (68.46) | 9.69 (9.62) | 0.143 (0.141) | 77.68 (78.08) |
| | 1.0 | | | | | |
| | | | | | | |
| D-4-3-07 ^{a)} | 60 vol.% ACN | 7.78 {8.05} | 65.51 (65.00) | 11.19 (11.07) | 0.171 (0.170) | 76.70 (76.07) |
| | 1.0 | | | | | |
| | | | | | | |
| D-4-3-08 ^{a)} | 70 vol.% ACN | 7.84 {8.05} | 67.41 (68.54) | 10.90 (11.05) | 0.162 (0.161) | 78.31 (79.59) |
| | 1.0 | | | | | |
| | | | | | | |
| D-4-3-09 ^{a)} | 80 vol.% ACN | - ^{e)} {7.97 ^{e)}} | - ^{e)} | - ^{e)} | - ^{e)} | - ^{e)} |
| | 1.0 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets e) addition of the parent buffer solution II to the solvent basis leads to a cloudiness - no preparation of an analytical HPLC sample conducted | | | | | | |

The results of table D-4-3-2 show:
- that a content in the range of 50 vol.% ACN in the analytical HPLC samples together with a buffer salt leads to a determination of comparably high area percentages of Tfa-liraglutide.

### Example D-4-4: analytical HPLC samples with increasing buffer salt content

1 mg of the final precipitate from example D-2-3 is dissolved in 1 mL of a respective solution for dissolving or diluting as described in table D-4-4-1. The obtained analytical HPLC sample is measured according to the analytical HPLC method. The obtained analytical HPLC sample is stored for 9.5 hours at 10 °C and re-measured. Table D-4-4-1 depicts the determined area percentage of liraglutide and Tfa-liraglutide from the HPLC-UV chromatogram of the analytical HPLC sample of the final precipitate.

**Table D-4-4-1**

| analytical HPLC sample No. | ACN in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| | amount of PO₄³⁻ added to 1 mL solvent basis ^{e)} | | | | | |
| | | | | | | |
| 0-4-4-01 ^{a)} | 50 vol.% ACN | 7.65 {8.06} | 68.70 (68.43) | 10.43 (10.26) | 0.152 (0.150) | 79.13 (78.69) |
| | 0.5 | | | | | |
| | 0.005 mmol ^{f)} | | | | | |
| | | | | | | |
| D-4-4-02 ^{a)} | 50 vol.% ACN | 7.83 {8.08} | 65.98 (67.06) | 10.82 (10.86) | 0.164 (0.160) | 76.80 (77.92) |
| | 1.0 | | | | | |
| | 0.01 mmol ^{f)} | | | | | |
| | | | | | | |
| D-4-4-03 ^{a)} | 50 vol.% ACN | 7.97 {8.08} | 68.51 (68.73) | 9.63 (9.51) | 0.141 (0.138) | 78.14 (78.24) |
| | 2.0 | | | | | |
| | 0.02 mmol ^{f)} | | | | | |
| | | | | | | |
| D-4-4-04 ^{a)} | 50 vol.% ACN | 8.02 {8.08} | 69.76 (69.87) | 8.22 (8.17) | 0.118 (0.117) | 77.98 (78.04) |
| | 5.0 | | | | | |
| | 0.05 mmol ^{f)} | | | | | |
| | | | | | | |
| D-4-4-05 ^{a)} | 50 vol.% ACN | 8.05 {8.08} | 71.82 (71.93) | 5.96 (5.99) | 0.083 (0.083) | 77.78 (77.92) |
| | 10.0 | | | | | |
| | 0.10 mmol ^{f)} | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets e) if the dissolved 1.0 mg of final precipitate are theoretically assumed to be pure liraglutide (Mw = 3751.2 g/mol), then around 0.00267 mmol of liraglutide are dissolved in 1.0 mL of the solution for dissolving or diluting. The latter one is approximately composed of 995 µL solvent basis and 5 µL buffer salt solution for D-4-4-01 respectively 990 µL and 10 µL for D-4-4-02, 980 µL and 20 µL for D-4-4-03, 950 µL and 50 µL for D-4-4-04 or 900 µL and 100 µL for D-4-4-05 f) equals the molar amount of (NH₄)₃PO₄, which is then - according to e) - present with theoretically up to around 0.00267 mmol of liraglutide, i.e. a formal molar ratio in relation to liraglutide of around 2 times buffer salt at D-4-4-01, around 4 times buffer salt at D-4-4-02, around 8 times buffer salt at D-4-4-03, around 20 times buffer salt at D-4-4-04 and around 40 times buffer salt at D-4-4-05 | | | | | | |

The results of table D-4-4-1 show:
- that at a range of 50 vol.% ACN in the analytical HPLC sample, a high molar amount of the buffer salt (NH₄)₃PO₄ in the analytical HPLC sample leads to a reduced area percentage of Tfa-liraglutide.

### Example D-4-5: analytical HPLC samples with different organic solvents

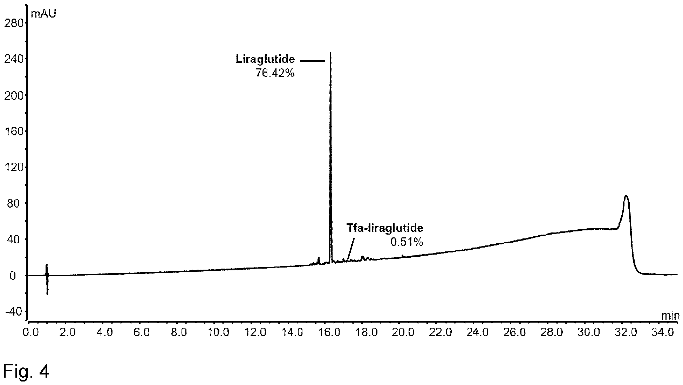

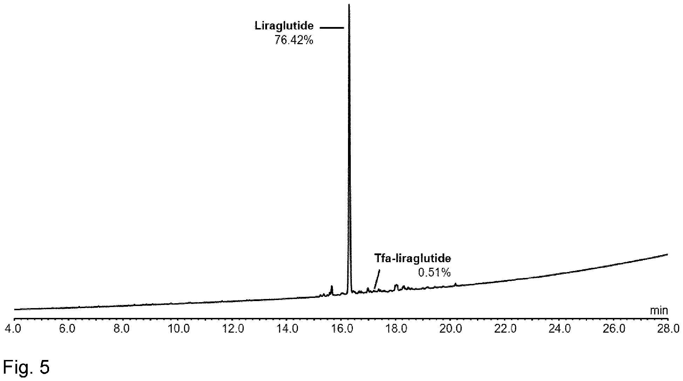

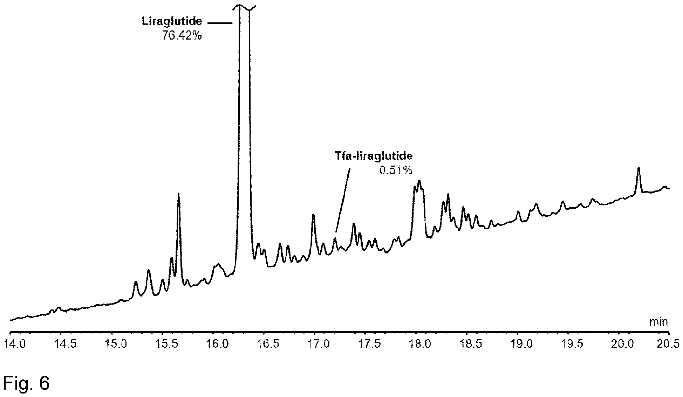

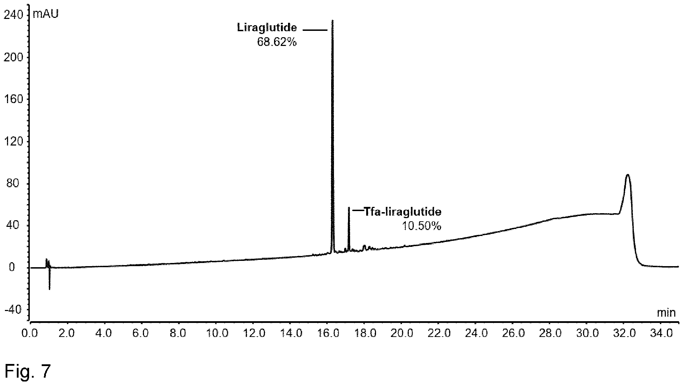

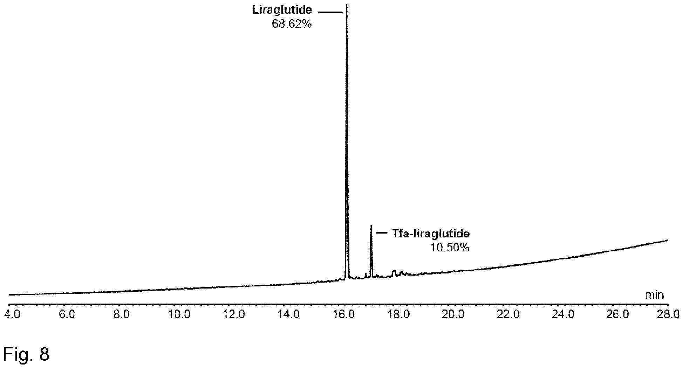

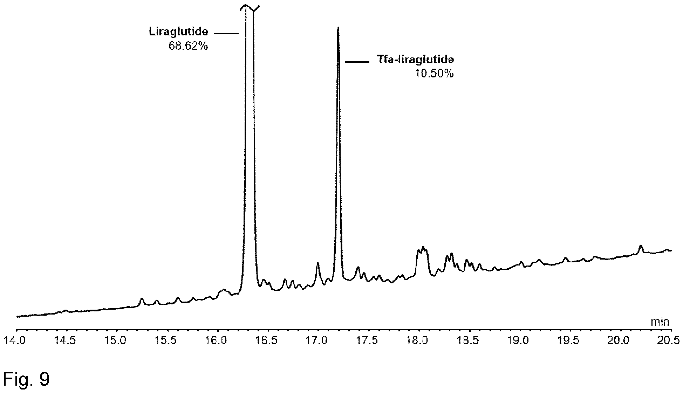

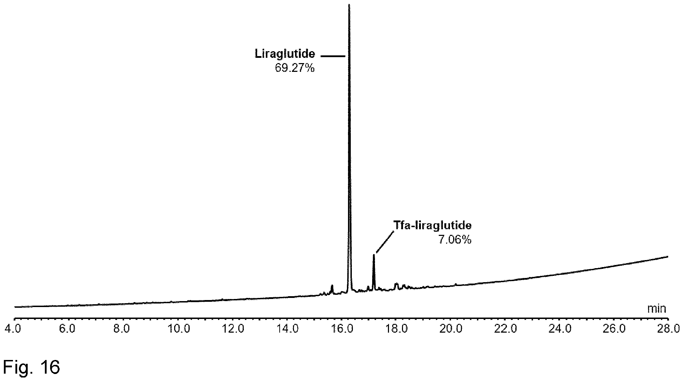

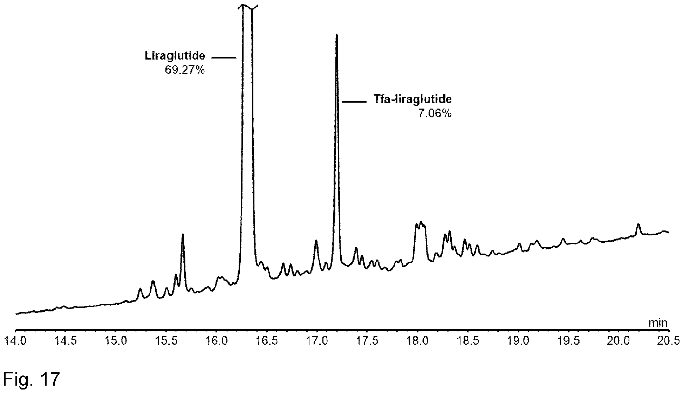

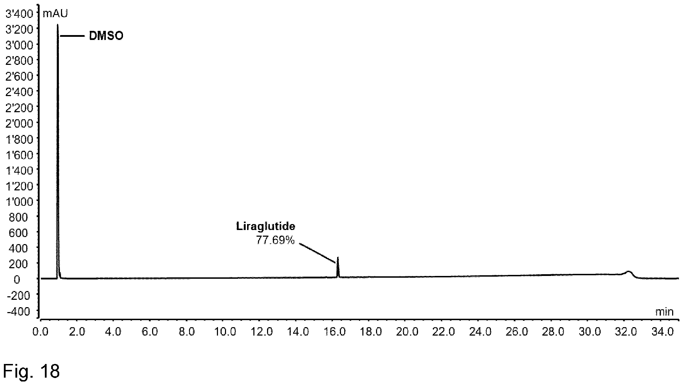

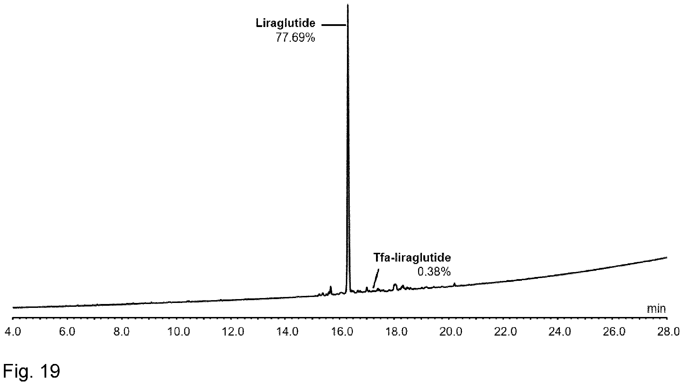

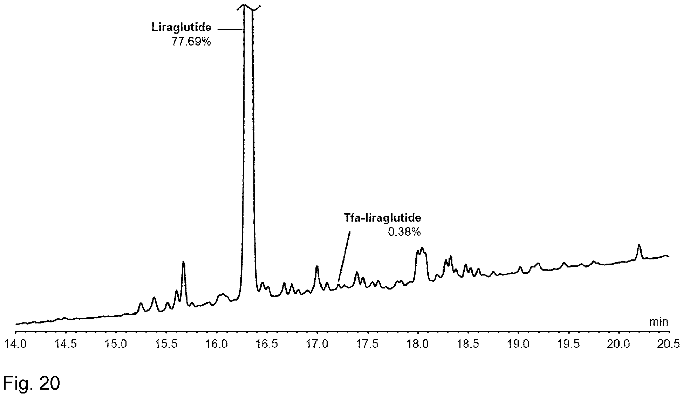

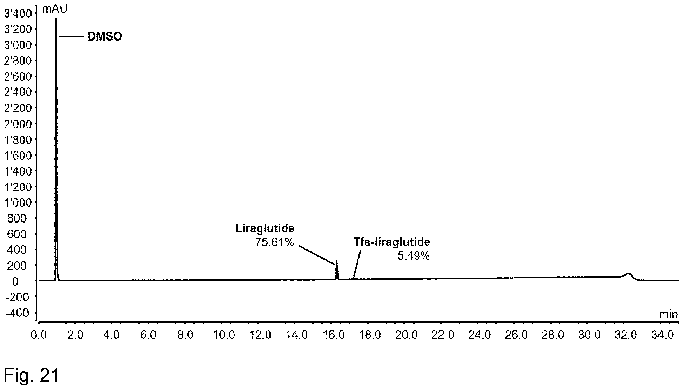

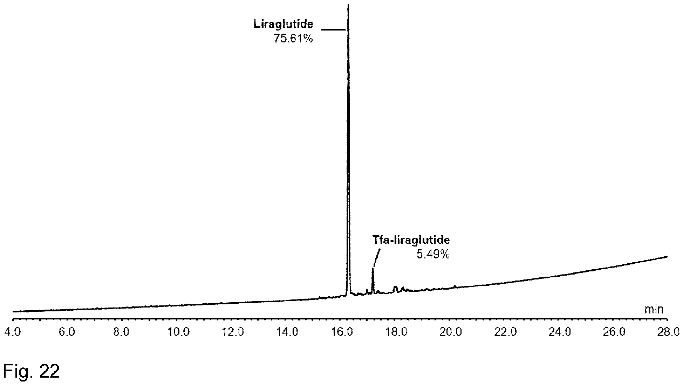

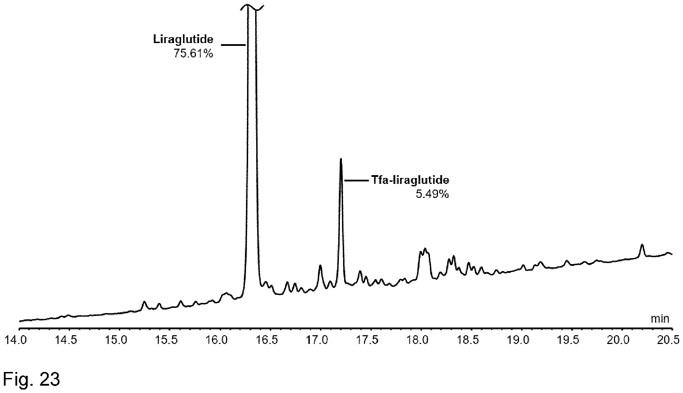

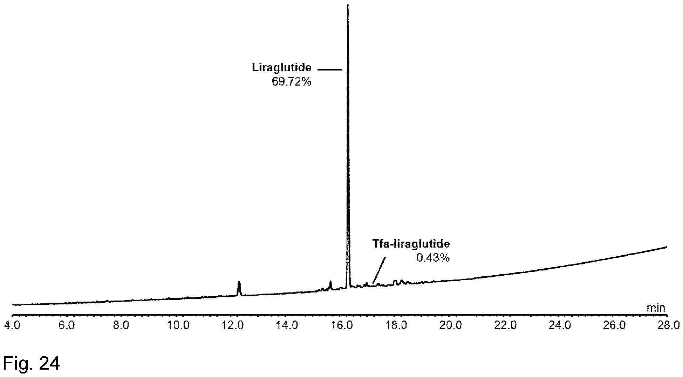

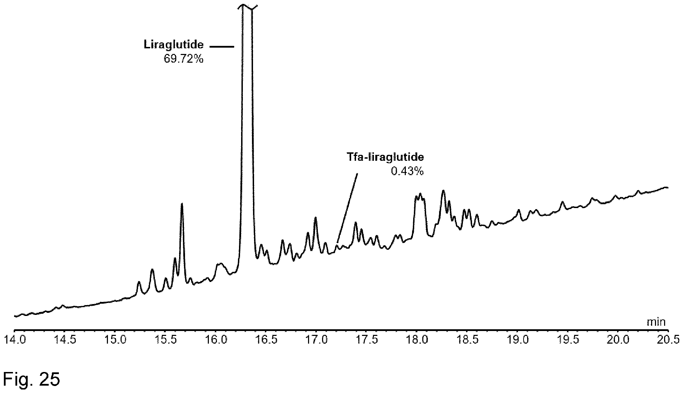

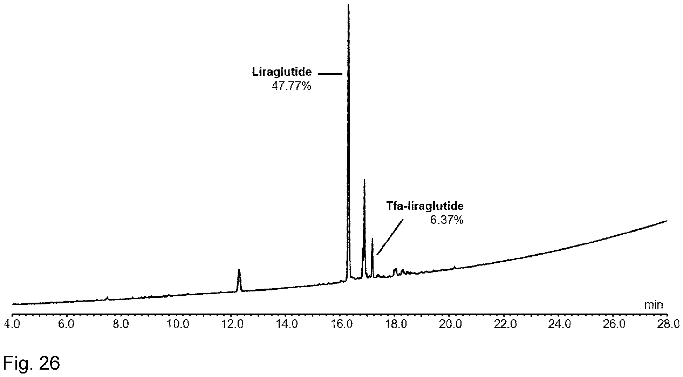

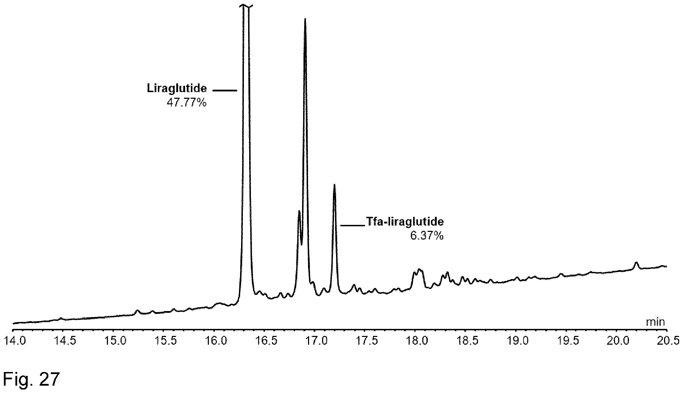

1 mg of the final precipitate from example D-2-3 is dissolved in 1 mL of a respective solution for dissolving or diluting as described in table D-4-5-1. The obtained analytical HPLC sample is measured according to the analytical HPLC method. The obtained analytical HPLC sample is stored for 9.5 hours at 10 °C and re-measured. Table D-4-5-1 depicts the determined area percentage of liraglutide and Tfa-liraglutide from the HPLC-UV chromatogram of the analytical HPLC sample of the final precipitate. Fig. 4 to 6 depict the complete HPLC-UV chromatogram of D-4-5-01 or extracts of it. Fig. 7 to 9 depict the complete HPLC-UV chromatogram of D-4-5-02 or extracts of it. Fig. 16 to 17 depict extracts of the HPLC-UV chromatogram of D-4-5-03. Fig. 18 to 20 depict the complete HPLC-UV chromatogram of D-4-5-07 or extracts of it. Fig. 21 to 23 depict the complete HPLC-UV chromatogram of D-4-5-08 or extracts of it. Fig. 24 to 25 depict extracts of the HPLC-UV chromatogram of D-4-5-10. Fig. 26 to 27 depict extracts of the HPLC-UV chromatogram of D-4-5-11.

**Table D-4-5-1**

| analytical HPLC sample No. | organic solvent in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| | | | | | | |
| D-4-5-01 ^{a), g)} | 50 vol.% ACN | 3.16 {7.20} | 76.42 (75.40) | 0.51 (0.53) | 0.007 (0.007) | 76.93 (75.93) |
| | - | | | | | |
| | | | | | | |
| D-4-5-02 ^{a)} | 50 vol.% ACN | 7.79 {8.07} | 68.62 (68.64) | 10.50 (10.39) | 0.153 (0.151) | 79.32 (79.03) |
| | 1.0 | | | | | |
| | | | | | | |
| D-4-5-03 ^{a)} | 100 vol.% DMF | - | 69.27 (66.17) | 7.06 (9.08) | 0.102 (0.137) | 76.33 (75.25) |
| | - | | | | | |
| | | | | | | |
| D-4-5-04 ^{a), g)} | 50 vol.% DMF | 3.63 {7.54} | 74.69 (77.12) | 0.65 (0.56) | 0.009 (0.007) | 75.34 (77.68) |
| | - | | | | | |
| | | | | | | |
| D-4-5-05 ^{a)} | 50 vol.% DMF | 8.41 {8.75} | 76.72 (73.50) | 4.95 (5.50) | 0.065 (0.075) | 81.67 (79.00) |
| | 1.0 | | | | | |
| | | | | | | |
| D-4-5-06 ^{a), g)} | 100 vol.% DMSO | - | 75.24 | 0.50 | 0.007 | 75.74 |
| | - | | | | | |
| | | | | | | |
| D-4-5-07 ^{a), g)} | 50 vol.% DMSO | 3.76 {7.77} | 77.69 (74.38) | 0.38 (0.49) | 0.005 (0.007) | 78.07 (74.87) |
| | - | | | | | |
| | | | | | | |
| D-4-5-08 ^{a)} | 50 vol.% DMSO | 8.84 {9.18} | 75.61 (72.97) | 5.49 (5.01) | 0.073 (0.069) | 81.10 (77.98) |
| | 1.0 | | | | | |
| | | | | | | |
| D-4-5-09 ^{a), g)} | 100 vol.% acetone | - ^{e)} | - | - | - | - |
| | - | | | | | |
| | | | | | | |
| D-4-5-1 0 ^{a), g)} | 50 vol.% acetone | 3.24 ^{f)} {7.70} | 69.72 (72.61) | 0.43 (0.26) | 0.006 (0.004) | 70.15 (72.87) |
| | - | | | | | |
| | | | | | | |
| D-4-5-11 ^{a)} | 50 vol.% acetone | 8.07 {8.32} | 47.77 (43.13) | 6.37 (6.63) | 0.133 (0.154) | 54.14 (49.76) |
| | 1.0 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets e) incomplete dissolution of the final precipitate - no measurement f) slightly cloudy - filtration with a 0.2 µm syringe filter to obtain the analytical HPLC sample g) comparative analytical HPLC sample | | | | | | |

The results of table D-4-4-1 show:
- that by a comparison of example D-4-5-02 versus example D-4-5-01, example D-4-5-05 versus example D-4-5-04, example D-4-5-08 versus D-4-5-07 and example D-4-5-10 versus D-4-5-11, that in an analytical HPLC sample, which comprises water as a solvent, a relevant Tfa-liraglutide area percentage is only determined if the buffer salt (NH₄)₃PO₄ is also present;
- that by a comparison of example D-4-5-03 versus example D-4-5-06, an analytical HPLC sample with only DMF as the solution for dissolving or diluting shows a relevant Tfa-liraglutide area percentage in contrast to an analytical HPLC sample with only DMSO as the solution for dissolving or diluting;
- that by a comparison of example D-4-5-02 versus example D-4-5-11, the ACN-containing analytical HPLC sample is prone to less relative change of Tfa-liraglutide area percentage after storage than the acetone-containing analytical HPLC sample;
- that by a comparison of example D-4-5-02 versus examples D-4-5-05, D-4-5-08 and D-4-5-11, the ACN-containing analytical HPLC sample shows the highest absolute Tfa-liraglutide area percentage.

### Example D-4-6: reproducibility of analytical HPLC sample

The analytical HPLC samples D-4-3-04, D-4-3-06, D-4-4-02 and D-4-5-02 are prepared from the same final precipitate of example D-2-3. The preparation procedure has been the same, i.e. 1.0 mg of final precipitate is dissolved in 1.0 mL of a solution for dissolving or diluting, which has been prepared by addition of 30 µL of an aqueous 1.0 M (NH₄)₃PO₄ solution adjusted to pH 7.8 to a solvent basis prepared by mixing 1.5 mL deionized water and 1.5 mL ACN. Table D-4-6-1 depicts the copied values of determined area percentage of liraglutide and Tfa-liraglutide from the HPLC-UV chromatogram of the analytical HPLC sample of the final precipitate and provides calculated averages and standard deviations.

**Table D-4-6-1**

| analytical HPLC sample No. | ACN in solvent basis of solution for dissolving or diluting | pH value ^{b), c)} of the analytical HPLC sample | area percentage liraglutide ^{d)} [%] | area percentage Tfa-liraglutide ^{d)} [%] | ratio of area percentages Tfa-liraglutide / liraglutide ^{d)} | sum of area percentages of Tfa-liraglutide and liraglutide ^{d)} [%] |
|---|---|---|---|---|---|---|
| | 1.0 M (NH₄)₃PO₄ pH 7.8 [vol.-pph] | | | | | |
| D-4-3-04 ^{a)} | 50 vol.% ACN | 7.82 {8.10} | 66.68 (67.04) | 11.06 (10.97) | 0.166 (0.165) | 77.74 (78.01) |
| | 1.0 | | | | | |
| | | | | | | |
| D-4-3-06 ^{a)} | 50 vol.% ACN | 7.76 {8.05} | 67.99 (68.46) | 9.69 (9.62) | 0.143 (0.141) | 77.68 (78.08) |
| | 1.0 | | | | | |
| | | | | | | |
| D-4-4-02 ^{a)} | 50 vol.% ACN | 7.83 {8.08} | 65.98 (67.06) | 10.82 (10.86) | 0.164 (0.160) | 76.80 (77.92) |
| | 1.0 | | | | | |
| | | | | | | |
| D-4-5-02 ^{a)} | 50 vol.% ACN | 7.79 {8.07} | 68.62 (68.64) | 10.50 (10.39) | 0.153 (0.151) | 79.32 (79.03) |
| | 1.0 | | | | | |
| | | | | | | |
| | | | | | | |
| average | 50 vol.% ACN | 7.81 {8.08} | 67.32 (67.80) | 10.52 (10.46) | 0.156 (0.154) | 77.84 (78.26) |
| | 1.0 | | | | | |
| | | | | | | |
| standard deviation | - | 0.04 {0.02} | 1.20 (0.87) | 0.60 (0.61) | 0.009 (0.008) | 0.96 (0.52) |
| | - | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) tentative pH value of the solution for dissolving or diluting prior to addition of the final precipitate provided in curly brackets d) values for analytical HPLC sample re-measured after storage for 9.5 h provided in brackets | | | | | | |

The results of table D-4-6-1 show:
- that preparation of several analytical HPLC samples from the same final precipitate results in reproducible area percentages.

### D-5: dissolving of more than 1.0 mg of final precipitate

### Example D-5-1: different modes of dissolving a final precipitate

Final precipitate of example D-2-3 is dissolved in an amount of more than 1.0 mg in different modes.

At example D-5-1-01 (mode 1), 100 mg of final precipitate of example D-2-3 are added to 5 mL of buffer salt solution A-buff (4 vol.% ACN, absolute amount of phosphoric acid anions is around 0.091 mmol if calculated based on its solvent basis, tentative pH value of 7.8) for a targeted concentration of 20 mg final precipitate in 1.0 mL of buffer salt solution A-buff under stirring at room temperature. After 1 h of stirring, there is still a suspension. A tentative pH value of 3.57 is measured at the suspension. The tentative pH value of the suspension is increased to 7.30 by addition of overall 100 µL of aqueous 1.5 M NaOH (absolute amount of around 0.15 mmol NaOH) in steps of 20 µL of aqueous 1.5 M NaOH. The suspension turns into a solution. For measuring a HPLC-UV chromatogram according to the analytical HPLC method, 50 µL of the stirred solution is diluted by addition of 950 µL buffer salt solution A-buff for a targeted concentration of 1.0 mg of final precipitate in 1 mL of diluted solution. A sample of this diluted solution is taken for an injection at the analytical HPLC method. After storage for 9.5 hours at 10 °C, the sample is measured again.

At example D-5-1-02 (mode 2), 100 µL of aqueous 1.5 M NaOH (absolute amount of around 0.15 mmol NaOH) is added to 5 mL of buffer salt solution A-buff (4 vol.% ACN, absolute amount of phosphoric acid anions is around 0.091 mmol if calculated based on its solvent basis, tentative pH value of 7.8). A tentative pH value of 9.84 is measured. Under stirring at room temperature, 100 mg of final precipitate of example D-2-3 is added. After several minutes, the final precipitate has dissolved and a tentative pH value of 6.95 is measured. After an addition of overall 20 µL of aqueous 1.5 M NaOH (absolute amount of around 0.03 mmol NaOH) in steps of 10 µL of aqueous 1.5 M NaOH, a tentative pH value of 7.32 is measured. For measuring a HPLC-UV chromatogram according to the analytical HPLC method, 50 µL of the stirred solution are diluted by addition of 950 µL buffer salt solution A-buff for a targeted concentration of 1.0 mg of final precipitate in 1 mL of diluted solution. A sample of this diluted solution is taken for an injection at the analytical HPLC method. After storage for 9.5 hours at 10 °C, the sample is measured again.

At example D-5-1-03 (mode 3), 10 mg of final precipitate of example D-2-3 are added to 10 mL of buffer salt solution A-buff (4 vol.% ACN, absolute amount of phosphoric acid anions is around 0.182 mmol if calculated based on its solvent basis, tentative pH value of 7.8) for a targeted concentration of 1.0 mg final precipitate in 1.0 mL of buffer salt solution A-buff under stirring at room temperature. After several minutes, the final precipitate has dissolved. A tentative pH value of 7.55 is measured. For measuring a HPLC-UV chromatogram according to the analytical HPLC method, a sample of the obtained solution is taken for an injection at the analytical HPLC method. After storage for 9.5 hours at 10 °C, the sample is measured again.

At example D-5-1-04 (mode 4), 10 mg of final precipitate of example D-2-3 are added to 10 mL of buffer salt solution B-buff (67 vol.% ACN, absolute amount of phosphoric acid anions is around 0.060 mmol if calculated based on its solvent basis, tentative pH value of 7.8) for a targeted concentration of 1.0 mg final precipitate in 1.0 mL of buffer salt solution B-buff under stirring at room temperature. After 1 h of stirring and despite of applying for some time periods ultrasonic, there is still a suspension. The suspension is filtered with a 0.2 µm syringe filter to obtain a solution. A tentative pH value of 7.76 is measured at the solution. For measuring a HPLC-UV chromatogram according to the analytical HPLC method, a sample of the obtained solution is taken for an injection at the analytical HPLC method. After storage for 9.5 hours at 10 °C, the sample is measured again.

Table D-5-1-1 depicts the determined area percentage of liraglutide and Tfa-liraglutide from the HPLC-UV chromatogram of injected solutions.

**Table D-5-1-1**

| example No. | ACN in liquid volume during example and in final solution for HPLC | pH value ^{b)} of the final solution for HPLC | area percentage liraglutide ^{c)} [%] | area percentage Tfa-liraglutide ^{c)} [%] | ratio of area percentages Tfa-liraglutide / liraglutide ^{c)} | sum of area percentages of Tfa-liraglutide and liraglutide ^{c)} [%] |
|---|---|---|---|---|---|---|
| | lowest pH value ^{b)} measured during example | | | | | |
| | | | | | | |
| D-5-1-01 ^{a)} | 4 vol.% ACN | 7.30 | 76.79 (76.87) | 0.95 (0.94) | 0.012 (0.012) | 77.74 (77.81) |
| | 3.57 ^{d)} | | | | | |
| | | | | | | |
| D-5-1-02 ^{a)} | 4 vol.% ACN | 7.32 | 71.69 (72.66) | 6.33 (5.92) | 0.088 (0.081) | 78.02 (78.58) |
| | 6.95 | | | | | |
| | | | | | | |
| D-5-1-03 ^{a)} | 4 vol.% ACN | 7.55 | 73.92 (74.40) | 4.65 (4.45) | 0.063 (0.060) | 78.57 (78.85) |
| | 7.55 | | | | | |
| | | | | | | |
| D-5-1-04 ^{a)} | 67 vol.% ACN ^{e)} | 7.76 | 70.10 (69.93) | 10.00 (9.90) | 0.143 (0.142) | 80.10 (79.83) |
| | 7.76 | | | | | |

| | | | | | | |
|---|---|---|---|---|---|---|
| Footnotes: a) comparative precipitated mixture b) only tentative pH value in view of a measurement by a pH electrode in an aqueous mixture with a high content of organic solvent c) values for analytical HPLC sample re-measured after storage for 9.5 h at 10 °C provided in brackets d) intermediary suspension for 1 h at this tentative pH value and the ACN content e) intermediary suspension for 1 h causes filtration for obtaining a solution | | | | | | |

The results of table D-5-1-1 show:
- that by a comparison of example D-5-1-01 versus examples D-5-1-02 to D-5-1-03, an intermediary low pH value for 1 h during dissolving leads to a very low area percentage of Tfa-liraglutide in the solution injected at the analytical HPLC method;
- that by a comparison of examples D-5-1-02 to D-5-1-03 versus example D-5-1-04, a low content of ACN during dissolving leads in a solution injected at the analytical HPLC method to a lower area percentage of Tfa-liraglutide.

### D-6: purifying dissolved crude liraglutide by preparative HPLC

### Example D-6-1: purification of crude liraglutide obtained with precipitation

9.6 g of final precipitate from example D-2-4 is dispersed in 480 mL of an aqueous solution, which contains around 5 vol.% acetonitrile and (NH₄)₃PO₄ in a concentration of 18 mM and possesses a pH value adjusted to 7.8. The dispersion is stirred, while the pH value of the dispersion is maintained between 7.0 and 7.4 by addition of 1 M NaOH solution (altogether 23 mL), until a solution is obtained. The solution is filtered with a 0.2 µm filter to result in a column feed solution. The column feed solution is fed to a first preparative reverse phase high performance liquid chromatography (column with a stationary phase of Kromasil (TM) C8 100 Angstrom 10 µm, eluent A: aqueous solution, which contains around 5 vol.% acetonitrile and (NH₄)₃PO₄ in a concentration of 18 mM and possesses a pH value adjusted to 7.8, eluent B: aqueous acetonitrile solution, which contains around 67 vol.% acetonitrile and (NH₄)₃PO₄ in a concentration of 5 mM and the pH value of the aqueous part prior to the presence of acetonitrile has been adjusted to 7.8, gradient elution starting with 100 % eluent A and increasing eluent B). The eluted liquid from the first preparative reverse phase high performance liquid chromatography is collected as fractions. A sample of each fraction is analyzed by an analytical HPLC and those fractions with a HPLC-UV chromatogram showing a high area percentage of liraglutide are combined to a first main-cut pool. The first main-cut pool is fed to a second preparative reverse phase high performance liquid chromatography (column with a stationary phase of Kromasil (TM) C8 100 Angstrom 10 µm, eluent A: aqueous solution, which contains around 5 vol.% acetonitrile and (NH₄)HCO₄ in a concentration of 10 mM and possesses a pH value adjusted to 7.8, eluent B: aqueous acetonitrile solution, which contains around 67 vol.% acetonitrile and (NH₄)HCO₃ and the pH value of the aqueous part prior to the presence of acetonitrile has been adjusted to 7.8, gradient elution starting with 100 % eluent A and increasing eluent B). The eluted liquid from the second preparative reverse phase high performance liquid chromatography is collected as fractions. A sample of each fraction is analyzed by an analytical HPLC and those fractions with a HPLC-UV chromatogram showing a high area percentage of liraglutide are combined to a second main-cut pool. The second main-cut pool is lyophilized to obtain a lyophilizate. The area percentage of liraglutide is 98.6 % based on a HPLC-UV chromatogram from an analytical HPLC of the lyophilizate.

## Claims

1. A method for manufacturing a precipitated mixture PrecMixt-1, which contains liraglutide or a salt thereof as component (A), comprising the steps
(i) providing a solid phase conjugated protected liraglutide precursor;
(ii) treating the solid phase conjugated protected liraglutide precursor with a cleavage composition CleaComp-1, which comprises the components
(a) 75 to 98.7 parts by volume of trifluoroacetic acid,
(b) 1 to 14 parts by volume of water,
(c) optionally one or more further scavengers different to water, wherein parts by volume are based on the volume of the cleavage composition CleaComp-1, which is 100 parts by volume, and the sum of the components (a), (b) and (c) is smaller than or exactly 100 parts by volume, at a temperature Temp-(ii) between 0 °C and 35 °C to obtain a cleavage suspension CleaSusp-1;
(iii) removing solid parts from the cleavage suspension CleaSusp-1 to obtain a cleavage solution CleaSolu-1;
(iv) mixing the cleavage solution CleaSolu-1 with an antisolvent to obtain a precipitation suspension PrecSusp-1;
(v) stirring the precipitation suspension PrecSusp-1 at a temperature Temp-(v) between 0 °C and 35 °C for a time period Time-(v), which is at least 7 h and lasts further until an analytical HPLC sample results in a HPLC-UV chromatogram with an area percentage of component (A)
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof,
and an area percentage of component (B)
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-I le-Ala-Trp-Leu-Val-Arg-Gly-Arg-Gly (Tfa-liraglutide) (SEQ ID NO: 4) or a salt thereof,
with a relative area percentage ratio (B) to (A) in the range of 0.080 to 0.010, to obtain a precipitation suspension PrecSusp-2,
when the analytical HPLC sample is prepared by
- taking a test sample from the stirred precipitation suspension,
- filtering the test sample to obtain a test sample precipitate,
- drying the test sample precipitate under vacuum,
- dissolving 1 mg of the dried test sample precipitate in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample;
(vi) isolating the precipitation from the precipitation suspension PrecSusp-2 to obtain the precipitated mixture PrecMixt-1.

2. The method according to claim 1, wherein step (ii) takes place at a time period Time-(ii), which is between 20 and 360 minutes.

3. The method according to claim 1 or 2, wherein the time period Time-(v) is at least 8 h.

4. The method according to claim 3, wherein the time period Time-(v) is between 8 h and 72 h.

5. The method according to any preceding claim, wherein the temperature Temp-(v) is between 4 °C and 32 °C.

6. The method according to any preceding claim, wherein at step (ii) the cleavage composition CleaComp-1 comprises
(c) 0.1 to 15 parts by volume of a scavenger different to water, which is a thiol, a silane, phenol, a cresol, anisole, thioanisole, 3-methylindole, N-acetylindole, tryptamine, methyl N-acetyl-tryptophanate or a mixture thereof,
wherein parts by volume are based on the volume of the cleavage composition CleaComp1, which is 100 parts by volume, and the sum of the components (a), (b) and (c) is below or exactly 100 parts by volume.

7. The method according to any preceding claim, wherein at step (iv) the antisolvent is a dialkyl ether or an antisolvent mixture AntiSolv-1 comprising the components
(anti-a) 50 to 99 parts by volume of dialkyl ether, and
(anti-b) 1 to 50 parts by volume of an aliphatic C₅-C₁₂ hydrocarbon or acetonitrile,
wherein parts by volume are based on the volume of the antisolvent mixture AntiSolv-1, which is 100 parts by volume, and the sum of the components (anti-a) and (anti-b) is below or exactly 100 parts by volume.

8. The method according to any preceding claim, wherein the HPLC-UV chromatogram with the area percentage of component (A) and the area percentage of component (B) has a relative area percentage ratio (B) to (A) in the range of 0.075 to 0.015.

9. The method according to any preceding claim, wherein the HPLC-UV chromatogram is obtained from a reverse phase high performance liquid chromatography, which comprises an injection of 0.5 to 3 µL of the analytical HPLC sample into a column with C18-modified silica as a stationary phase, a column temperature of 50 °C, an UV detection at 220 nm, an eluent A prepared by adding 0.05 vol.% trifluoroacetic acid based on the overall volume of a mixture of 98 vol.% deionized water and 2 vol.% acetonitrile, an eluent B obtained by adding 0.05 vol.% trifluoroacetic acid to acetonitrile and a gradient elution of the two eluents A and B by a start with 85 vol.% eluent A and 15 vol.% eluent B and an increase of the amount of eluent B to 100 vol.%.

10. The method according to any preceding claim for manufacturing a purified composition PuriComp-1, which contains liraglutide or a salt thereof, which comprises the steps
(vii) dissolving the precipitated mixture PrecMixt-1 to obtain a solution for a preparative liquid chromatography SoluChro-1;
(viii-a) purifying the solution for a preparative liquid chromatography SoluChro-1 by a first preparative liquid chromatography including collecting fractions and combining selected fractions,
(viii-b) optionally purifying by a second preparative liquid chromatography including collecting fractions and combining selected fractions,
(viii-c) optionally desalting of combined selected fractions from step (viii-a) or optionally step (viii-b);
(ix) lyophilizing to obtain the purified composition PuriComp-1.

11. The method according to any preceding claim, wherein the precipitated mixture PrecMixt-1 has a water content of less than 20 wt.% based on the weight of the precipitated mixture PrecMixt-1.

12. The method according to any preceding claim, wherein the precipitated mixture PrecMixt-1 has a content of trifluoroacetic acid between 6 wt.% and 24 wt.% based on the weight of the precipitated mixture PrecMixt-1.

13. A method for manufacturing a purified liraglutide composition PuriComp-1, which contains liraglutide or a salt thereof, which comprises the steps
(I) dissolving a precipitated mixture PrecMixt-1 , which contains the components
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO: 3) or a salt thereof, and
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala- Trp-Leu-Val-Arg-Gly-Arg-Gly (Tfa-liraglutide) (SEQ ID NO: 4) or a salt thereof,
wherein an analytical HPLC sample of the precipitated mixture PrecMixt-1 results in a HPLC-UV chromatogram with an area percentage of component (A) and an area percentage of component (B) with a relative area percentage ratio (B) to (A) in the range of 0.080 to 0.010, when the analytical HPLC sample is prepared by
- dissolving 1 mg of the precipitated mixture PrecMixt-1 in 1 mL of a solution for dissolving or diluting, which is obtained by mixing water and acetonitrile in a volume ratio of 1 to 1 and by adding 0.01 mL of 1.0 M (NH₄)₃PO₄ with a pH of 7.8 per 1 mL of the water-acetonitrile mixture, to obtain the analytical HPLC sample,
which has a water content of less than 20 wt.% based on the weight of the precipitated mixture PrecMixt-1,
to obtain a solution for a preparative liquid chromatography SoluChro-1;
(II-a) purifying the solution for a preparative liquid chromatography SoluChro-1 from step (I) by a first preparative liquid chromatography including collecting fractions and combining selected fractions,
(II-b) optionally purifying by a second preparative liquid chromatography including collecting fractions and combining selected fractions,
(II-c) optionally desalting of combined selected fractions from step (II-a) or optionally from (II-b);
(III) lyophilizing to obtain the purified liraglutide composition PuriComp-1.

14. The method for manufacturing a purified liraglutide composition PuriComp-1 according to claim 13, wherein the HPLC-UV chromatogram is obtained from a reverse phase high performance liquid chromatography, which comprises an injection of 0.5 to 3 µL of the analytical HPLC sample into a column with C18-modified silica, a column temperature of 50 °C, an UV detection at 220 nm, an eluent A obtained by adding 0.05 vol.% trifluoroacetic acid to a mixture of 98 vol.% deionized water and 2 vol.% acetonitrile, an eluent B obtained by adding 0.05 vol.% trifluoroacetic acid to acetonitrile and a gradient elution by a start with 85 vol.% eluent A and 15 vol.% eluent B and an increase of the amount of eluent B to 100 vol.%.

## Patentansprüche

1. Verfahren zur Herstellung einer gefällten Mischung PrecMixt-1, die Liraglutid oder ein Salz davon als Komponente (A) enthält, umfassend die Schritte
(i) Bereitstellung einer festphasenkonjugierten geschützten Liraglutid-Vorstufe
(ii) Behandeln der festphasenkonjugierten geschützten Liraglutid-Vorstufe mit einer Spaltungszusammensetzung CleaComp-1, die die Komponenten umfasst
(a) 75 bis 98,7 Volumenanteile Trifluoressigsäure,
(b) 1 bis 14 Volumenanteile Wasser,
(c) gegebenenfalls ein oder mehrere weitere von Wasser verschiedene Scavenger
wobei sich die Volumenanteile auf das Volumen der Spaltungszusammensetzung CleaComp1 beziehen, das 100 Volumenanteile beträgt, und die Summe der Komponenten (a), (b) und (c) kleiner oder genau 100 Volumenanteile ist, bei einer Temperatur Temp-(ii) zwischen 0 °C und 35 °C, um eine Spaltungssuspension CleaSusp-1 zu erhalten;
(iii) Entfernen fester Teile aus der Spaltungssuspension CleaSusp-1, um eine Spaltungslösung CleaSolu-1 zu erhalten;
(iv) Mischen der Spaltungslösung CleaSolu-1 mit einem Antilösungsmittel, um eine Fällungssuspension PrecSusp-1 zu erhalten;
(v) Rühren der Fällungssuspension PrecSusp-1 bei einer Temperatur Temp-(v) zwischen 0 °C und 35 °C für einen Zeitraum Time-(v), der mindestens 7 h beträgt und weiter andauert, bis eine analytische HPLC-Probe ein HPLC-UV-Chromatogramm mit einem Flächenanteil der Komponente (A) ergibt
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Liraglutid) (SEQ ID NO: 3) oder ein Salz davon,
und ein Flächenanteil der Komponente (B)
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-Liraglutid) (SEQ ID NO: 4) oder ein Salz davon, mit einem relativen Flächenanteilsverhältnis (B) zu (A) im Bereich von 0,080 bis 0,010, um eine Fällungssuspension PrecSusp-2 zu erhalten, wenn die analytische HPLC-Probe vorbereitet wird durch
- Entnahme einer Probe aus der gerührten Fällungssuspension,
- Filtern der Probe, um ein Probenpräzipitat zu erhalten,
- Trocknen des Probenpräzipitats unter Vakuum,
- Auflösen von 1 mg des getrockneten Probenpräzipitats in 1 ml einer Lösung zum Auflösen oder Verdünnen, die durch Mischen von Wasser und Acetonitril im Volumenverhältnis 1 zu 1 erhalten wird und durch Hinzufügen von 0,01 ml einer 1,0 M (NH₄)₃PO₄ mit einem pH-Wert von 7,8 pro 1 ml der Wasser-Acetonitril-Mischung, um die analytische HPLC-Probe zu erhalten;
(vi) Isolieren der Fällung aus der Fällungssuspension PrecSusp-2 um die gefällte Mischung PrecMixt-1 zu erhalten.

2. Verfahren gemäß Anspruch 1, wobei Schritt (ii) in einem Zeitraum Time-(ii) stattfindet, der zwischen 20 und 360 Minuten liegt.

3. Verfahren gemäß Anspruch 1 oder 2, wobei der Zeitraum Time-(v) mindestens 8 Stunden beträgt.

4. Verfahren gemäß Anspruch 3, wobei der Zeitraum Time-(v) zwischen 8 h und 72 h liegt.

5. Verfahren gemäß einem der vorangehenden Ansprüche, wobei die Temperatur Temp-(v) zwischen 4 °C und 32 °C liegt.

6. Verfahren gemäß einem der vorangehenden Ansprüche, wobei in Schritt (ii) die Spaltungszusammensetzung CleaComp-1 umfasst
(c) 0,1 bis 15 Volumenanteile eines von Wasser verschiedenen Scavengers, der ein Thiol, ein Silan, Phenol, ein Kresol, Anisol, Thioanisol, 3-Methylindol, N-Acetylindol, Tryptamin, Methyl-N-Acetyltryptophanat oder eine Mischung davon ist,
wobei sich die Volumenanteile auf das Volumen der Spaltungszusammensetzung CleaComp1 beziehen, das 100 Volumenanteile beträgt, und die Summe der Komponenten (a), (b) und (c) kleiner oder genau 100 Volumenanteile ist.

7. Verfahren gemäß einem der vorangehenden Ansprüche, wobei in Schritt (iv) das Antilösungsmittel ein Dialkylether oder eine Antilösungsmittelmischung AntiSolv-1 ist, die die Komponenten umfasst
(anti-a) 50 bis 99 Volumenanteile Dialkylether und
(anti-b) 1 bis 50 Volumenanteile eines aliphatischen C5-C12 Kohlenwasserstoffs oder Acetonitril,
wobei sich die Volumenanteile auf das Volumen der Antilösungsmittelmischung AntiSolv-1 beziehen, das 100 Volumenanteile beträgt, und die Summe der Komponenten (anti-a) und (anti-b) kleiner oder genau 100 Volumenanteile ist.

8. Verfahren gemäß einem der vorangehenden Ansprüche, wobei das HPLC-UV-Chromatogramm mit dem Flächenanteil der Komponente (A) und dem Flächenanteil der Komponente (B) ein relatives Flächenanteilsverhältnis (B) zu (A) in einem Bereich von 0,075 bis 0,015 aufweist.

9. Verfahren gemäß einem der vorangehenden Ansprüche, wobei das HPLC-UV-Chromatogramm aus einer RP (reverse phase)-Hochleistungsflüssigkeits-chromatographie erhalten wird, die eine Injektion von 0,5 bis 3 µL der analytischen HPLC-Probe in eine Säule mit C18-modifizierter Kieselsäure als stationärer Phase umfasst, eine Säulentemperatur von 50 °C, eine UV-Detektion bei 220 nm, einen Eluenten A, hergestellt durch Zugabe von 0,05 Vol.-% Trifluoressigsäure, basierend auf dem Gesamtvolumen einer Mischung aus 98 Vol.-% deionisiertem Wasser und 2 Vol.-% Acetonitril, einen Eluenten B, erhalten durch Zugabe von 0,05 Vol.-% Trifluoressigsäure zu Acetonitril und eine Gradientenelution der zwei Eluenten A und B durch einen Start mit 85 Vol.-% Eluent A und 15 Vol.-% Eluent B und eine Erhöhung der Menge an Eluent B auf 100 Vol.-%.

10. Verfahren gemäß einem der vorangehenden Ansprüche zur Herstellung einer gereinigten Zusammensetzung PuriComp-1, die Liraglutid oder ein Salz davon enthält, umfassend die Schritte
(vii) Auflösen der gefällten Mischung PrecMixt-1 um eine Lösung für eine präparative Flüssigkeitschromatographie SoluChro-1 zu erhalten;
(viii-a) Reinigen der Lösung für eine präparative Flüssigkeitschromatographie SoluChro-1 durch eine erste präparative Flüssigkeitschromatographie, einschließlich dem Sammeln von Fraktionen und dem Vereinigen ausgewählter Fraktionen,
(viii-b) gegebenenfalls Reinigen durch eine zweite präparative Flüssigkeitschromatographie einschließlich dem Sammeln von Fraktionen und dem Vereinigen ausgewählter Fraktionen,
(viii-c) gegebenenfalls Entsalzen der vereinigten ausgewählten Fraktionen aus Schritt (viii-a) oder gegebenenfalls Schritt (viii-b);
(ix) Gefriertrocknung, um die gereinigte Zusammensetzung PuriComp-1 zu erhalten.

11. Verfahren gemäß einem der vorangehenden Ansprüche, wobei die gefällte Mischung PrecMixt-1 einen Wassergehalt von weniger als 20 Gew.-% bezogen auf das Gewicht der gefällten Mischung PrecMixt-1, aufweist.

12. Verfahren gemäß einem der vorangehenden Ansprüche, wobei die gefällte Mischung PrecMixt-1 einen Anteil an Trifluoressigsäure zwischen 6 Gew.-% und 24 Gew.-% bezogen auf das Gewicht der gefällten Mischung PrecMixt-1 aufweist.

13. Verfahren zur Herstellung einer gereinigten Liraglutid-Zusammensetzung PuriComp-1, die Liraglutid oder ein Salz davon enthält, umfassend die Schritte
(I) Auflösen der gefällten Mischung PrecMixt-1, enthaltend die Komponenten
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Liraglutid) (SEQ ID NO: 3) oder ein Salz davon, und
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-Liraglutid) (SEQ ID NO: 4) oder ein Salz davon,
wobei eine analytische HPLC-Probe der gefällten Mischung PrecMixt-1 ein HPLC-UV-Chromatogramm ergibt mit einem Flächenanteil der Komponente (A) und einem Flächenanteil der Komponente (B) mit einem relativen Flächenanteilsverhältnis (B) zu (A) im Bereich von 0,080 bis 0,010, wenn die analytische HPLC-Probe vorbereitet wird durch
- Auflösen von 1 mg der gefällten Mischung PrecMixt-1 in 1 ml einer Lösung zum Auflösen oder Verdünnen, die durch Mischen von Wasser und Acentonitril im Volumenverhältnis 1 zu 1 erhalten wird und durch Hinzufügen von 0,01 ml einer 1,0 M (NH₄)₃PO₄ mit einem pH-Wert von 7,8 pro 1 ml der Wasser-Acetonitril-Mischung, um die analytische HPLC-Probe zu erhalten,
welche einen Wassergehalt von weniger als 20 Gew.-% bezogen auf das Gewicht der gefällten Mischung PrecMixt-1, aufweist,
um eine Lösung für eine präparative Flüssigkeitschromatographie SoluChro-1 zu erhalten;
(II-a) Reinigen der Lösung für eine preparative Flüssogchromatographie SoluChro-1 aus Schritt (I) durch eine erste präparative Flüssigkeitschromatographie, einschließlich dem Sammeln von Fraktionen und dem Vereinigen ausgewählter Fraktionen,
(II-b) gegebenenfalls Reinigen durch eine zweite preparative Flüssigkeitschromatographie einschließlich dem Sammeln von Fraktionen und dem Vereinigen ausgewählter Fraktionen,
(II-c) gegebenenfalls Entsalzen der vereinigten ausgewählten Fraktionen aus Schritt (II-a) oder gegebenenfalls von (II-b);
(III) Gefriertrocknung, um die gereinigte Liraglutid-Zusammensetzung PuriComp-1 zu erhalten.

14. Verfahren zur Herstellung einer gereinigten Liraglutid-Zusammensetzung PuriComp-1 nach Anspruch 13, wobei das HPLC-UV-Chromatogramm aus einer RP (reverse phase)-Hochleistungsflüssigkeits-chromatographie erhalten wird, die eine Injektion von 0,5 bis 3 µL der analytischen HPLC-Probe in eine Säule mit C18-modifizierter Kieselsäure umfasst, eine Säulentemperatur von 50 °C, eine UV-Detektion bei 220 nm, einen Eluenten A, hergestellt durch Zugabe von 0,05 Vol.-% Trifluoressigsäure, zu einer Mischung aus 98 Vol.-% deionisiertem Wasser und 2 Vol.-% Acetonitril, einen Eluenten B, erhalten durch Zugabe von 0,05 Vol.-% Trifluoressigsäure zu Acetonitril und eine Gradientenelution mit einem Start mit 85 Vol.-% Eluent A und 15 Vol.-% Eluent B eine Erhöhung der Menge an Eluent B auf 100 Vol.-%.

## Revendications

1. Procédé de fabrication d'un mélange précipité PrecMixt-1, qui contient du liraglutide ou un sel de celui-ci en tant que composant (A), comprenant les étapes de
(i) fourniture d'un précurseur de liraglutide protégé conjugué en phase solide ;
(ii) traitement du précurseur de liraglutide protégé conjugué en phase solide avec une composition de clivage CleaComp-1, qui comprend les composants suivants
(a) 75 à 98,7 parties en volume d'acide trifluoroacétique,
(b) 1 à 14 parties en volume d'eau,
(c) éventuellement un ou plusieurs piégeurs supplémentaires différents de l'eau, les parties en volume étant par rapport au volume de la composition de clivage CleaComp-1, qui est de 100 parties en volume, et la somme des composants (a), (b) et (c) est inférieure ou égale à 100 parties en volume, à une température Temp-(ii) comprise entre 0 °C et 35 °C pour obtenir une suspension de clivage CleaSusp-1 ;
(iii) retrait des parties solides de la suspension de clivage CleaSusp-1 pour obtenir une solution de clivage CleaSolu-1 ;
(iv) mélange de la solution de clivage CleaSolu-1 avec un antisolvant pour obtenir une suspension de précipitation PrecSusp-1 ;
(v) agitation de la suspension de précipitation PrecSusp-1 à une température Temp-(v) comprise entre 0 °C et 35 °C pendant une période de temps Time-(v), qui est d'au moins 7 h et dure jusqu'à ce qu'un échantillon HPLC analytique conduise à un chromatogramme HPLC-UV avec un pourcentage en aire du composant (A)
(A) His-Ala-Glu-Gly-Thr-Phe-Thr-Ser-Asp-Val-Ser-Ser-Tyr-Leu-Glu-Gly-Gln-Ala-Ala-Lys (palmitoyl-gamma-Glu)-Glu-Phe-Ile-Ala-Trp-Leu-Val-Arg-Gly-Arg-Gly (liraglutide) (SEQ ID NO : 3) ou un sel de celui-ci,
et un pourcentage en aire du composant (B)
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO : 4) ou un sel de celui-ci,
avec un rapport de pourcentage en aire relatif (B) à (A) dans la plage de 0,080 à 0,010, pour obtenir une suspension de précipitation PrecSusp-2, lorsque l'échantillon HPLC analytique est préparé par
- prélèvement d'un échantillon d'essai de la suspension de précipitation agitée,
- filtration de l'échantillon d'essai pour obtenir un précipité d'échantillon à tester,
- séchage di précipité de l'échantillon d'essai sous vide,
- dissolution de 1 mg du précipité de l'échantillon d'essai séché dans 1 ml d'une solution à dissoudre ou à diluer, obtenue par mélange d'eau et d'acétonitrile dans un rapport en volume de 1 à 1 et par ajout de 0,01 ml de (NH₄)₃PO₄ 1,0 M ayant un pH de 7,8 par 1 ml du mélange eau-acétonitrile, pour obtenir l'échantillon HPLC analytique ;
(vi) isolement de la précipitation à partir de la suspension de précipitation PrecSusp-2 pour obtenir le mélange précipité PrecMixt-1.

2. Procédé selon la revendication 1, dans lequel l'étape (ii) est effectuée dans une période de temps Time-(ii), qui est comprise entre 20 et 360 minutes.

3. Procédé selon la revendications 1 ou 2, dans lequel la période de temps Time-(v) est d'au moins 8 h.

4. Procédé selon la revendication 3, dans lequel la période de temps Time-(v) est comprise entre 8 h et 72 h.

5. Procédé selon l'une quelconque des revendications précédentes, dans lequel la température Temp-(v) est comprise entre 4 °C et 32 °C.

6. Procédé selon l'une quelconque des revendications précédentes, dans lequel, à l'étape (ii), la composition de clivage CleaComp-1 comprend
(c) 0,1 à 15 parties en volume d'un piégeur différent de l'eau, qui est un thiol, un silane, phénol, un crésol, anisole, thioanisole, 3-méthylindole, N-acétylindole, tryptamine, N-acétyl-tryptophanate de méthyle ou un mélange de ceux-ci,
dans lequel les parties en volume sont basées sur le volume de la composition de clivage CleaComp1, qui est de 100 parties en volume, et la somme des composants (a), (b) et (c) est inférieure ou égale à 100 parties en volume.

7. Procédé selon l'une quelconque des revendications précédentes, dans lequel à l'étape (iv), l'antisolvant est un éther dialkylique ou un mélange d'antisolvants AntiSolv-1 comprenant les composants
(anti-a)50 à 99 parties en volume d'éther dialkylique, et
(anti-b) 1 à 50 parties en volume d'un hydrocarbure en C₅-C₁₂ aliphatique ou d'acétonitrile,
dans lequel les parties en volume sont basées sur le volume du mélange d'antisolvants AntiSolv-1, qui est de 100 parties en volume, et la somme des composants (anti-a) et (anti-b) est inférieure ou égale à 100 parties en volume.

8. Procédé selon l'une quelconque des revendications précédentes, dans lequel le chromatogramme HPLC-UV avec le pourcentage en aire du composant (A) et le pourcentage en aire du composant (B) présente un rapport de pourcentage en aire relatif de (B) à (A) dans la plage de 0,075 à 0,015.

9. Procédé selon l'une quelconque des revendications précédentes, dans lequel le chromatogramme HPLC-UV est obtenu à partir d'une chromatographie liquide haute performance en phase inverse, qui comprend une injection de 0,5 à 3 µl de l'échantillon HPLC analytique dans une colonne avec de la silice modifiée C18 en tant que phase stationnaire, une température de colonne de 50 °C, une détection UV à 220 nm, un éluant A préparé par ajout de 0,05 % en volume d'acide trifluoroacétique sur la base du volume total d'un mélange de 98 % en volume d'eau désionisée et 2 % en volume d'acétonitrile, un éluant B obtenu par ajout de 0,05 % en volume d'acide trifluoroacétique à l'acétonitrile et élution à gradient des deux éluants A et B avec un départ à 85 % en volume d'éluant A et 15 % en volume d'éluant B et une augmentation de la quantité d'éluant B jusqu'à 100 % en volume.

10. Procédé selon l'une quelconque des revendications précédentes pour fabriquer une composition purifiée PuriComp-1, qui contient du liraglutide ou un sel de celui-ci, qui comprend les étapes
(vii) dissolution du mélange précipité PrecMixt-1 pour obtenir une solution pour une chromatographie liquide préparative SoluChro-1 ;
(viii-a) purification de la solution pour une chromatographie liquide préparative SoluChro-1 par une première chromatographie liquide préparative comprenant la collecte de fractions et la combinaison des fractions sélectionnées,
(viii-b) éventuellement purification par une deuxième chromatographie liquide préparative comprenant la collecte des fractions et la combinaison des fractions sélectionnées,
(viii-c) éventuellement dessalage des fractions sélectionnées combinées de l'étape
(viii-a) ou éventuellement de l'étape (viii-b) ;
(ix) lyophilisation pour obtenir la composition purifiée PuriComp-1.

11. Procédé selon l'une quelconque des revendications précédentes, dans lequel le mélange précipité PrecMixt-1 a une teneur en eau inférieure à 20 % en poids sur la base du poids du mélange précipité PrecMixt-1.

12. Procédé selon l'une quelconque des revendications précédentes, dans lequel le mélange précipité PrecMixt-1 a une teneur en acide trifluoroacétique comprise entre 6 % en poids et 24 % en poids sur la base du poids du mélange précipité PrecMixt-1.

13. Procédé de fabrication d'une composition de liraglutide purifiée PuriComp-1, qui contient du liraglutide ou un sel de celui-ci, qui comprend les étapes
(I) dissolution d'un mélange précipité PrecMixt-1, qui contient les composants
(A) His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (liraglutide) (SEQ ID NO : 3) ou un sel de celui, ci ; et
(B) CF₃CO-His-Ala-Glu-Gly-Thr-Phe-Thr-Ser-Asp-Val-Ser-Ser-Tyr-Leu-Glu-Gly-Gln-Ala-Ala-Lys(palmitoyl-gamma-Glu)-Glu-Phe-Ile-Ala-Trp-Leu-Val-Arg-Gly-Arg-Gly (Tfa-liraglutide) (SEQ ID NO : 4) ou un sel de celui-ci,
dans lequel un échantillon HPLC analytique du mélange précipité PrecMixt-1 conduit à un chromatogramme HPLC-UV avec un pourcentage en aire du composant (A) et un pourcentage en aire du composant (B) avec un rapport de pourcentage en aire relatif de (B) à (A), dans la plage de 0,080 à 0,010, l'échantillon HPLC analytique étant préparé par
- dissolution de 1 mg du mélange précipité PrecMixt-1 dans 1 ml d'une solution à dissoudre ou à diluer, qui est obtenue par mélange d'eau et d'acétonitrile dans un rapport en volume de 1 à 1 et par ajout de 0,01 ml de (NH₄)₃PO₄ 1,0 M ayant un pH de 7,8 pour 1 ml du mélange eau-acétonitrile, pour obtenir l'échantillon HPLC analytique, qui a une teneur en eau inférieure à 20 % en poids par rapport au poids du mélange précipité PrecMixt-1,
pour obtenir une solution pour une chromatographie liquide préparative SoluChro-1;
(II-a) purification de la solution pour une chromatographie liquide préparative SoluChro-1 provenant de l'étape (I) par une première chromatographie liquide préparative comprenant la collecte des fractions et la combinaison des fractions sélectionnées,
(II-b) éventuellement purification par une deuxième chromatographie liquide préparative comprenant la collecte de fractions et la combinaison des fractions sélectionnées,
(II-c) éventuellement dessalage des fractions sélectionnées combinées de l'étape (II-a) ou éventuellement (II-b) ;
(III) lyophilisation pour obtenir la composition de liraglutide purifiée PuriComp-1.

14. Procédé de fabrication d'une composition de liraglutide purifiée PuriComp-1 selon la revendication 13, dans lequel le chromatogramme HPLC-UV est obtenu à partir d'une chromatographie liquide haute performance en phase inverse, qui comprend une injection de 0,5 à 3 µl de l'échantillon HPLC analytique dans une colonne avec de la silice modifiée C18, une température de colonne de 50 °C, une détection UV à 220 nm, un éluant A obtenu par ajout de 0,05 % en volume d'acide trifluoroacétique sur la base du volume total d'un mélange de 98 % en volume d'eau désionisée et 2 % en volume d'acétonitrile, un éluant B obtenu par ajout de 0,05 % en volume d'acide trifluoroacétique à l'acétonitrile et une élution à gradient avec un départ à 85 % en volume d'éluant A et 15 % en volume d'éluant B et une augmentation de la quantité d'éluant B jusqu'à 100 % en volume.
